# EUROPEAN PATENT SPECIFICATION

(11) **EP 2 925 866 B1**
(45) Date of publication and mention of the grant of the patent: **25.07.2018**
(21) Application number: 13811114.1
(22) Date of filing: 29.11.2013
(51) Int. Cl.: C12N 15/113, A61K 31/7088

(54) **CIRCULAR RNA FOR INHIBITION OF MICRORNA**
ZIRKULÄRE RNA ZUR HEMMUNG VON MICRORNA
ARN CIRCULAIRE DESTINÉ À L'INHIBITION DE MICRO-ARN

(30) Priority: 30.11.2012 US 201261731788 P
(43) Date of publication of application: 07.10.2015
(73) Proprietor: Aarhus Universitet, 8000 Aarhus C (DK)
(72) Inventor: KJEMS, Jørgen, 8240 Risskov (DK); BIRKBALLE, Hansen, Thomas, 8230 Åbyhøj (DK)
(74) Representative: Plougmann Vingtoft a/s
(86) International application number: PCT/DK2013/050404
(87) International publication number: WO 2014/082644

(56) References cited:
- WO-A1-2010/029303
- WO-A1-2010/084371
- WO-A2-2007/112754
- WO-A2-2011/103394
- THOMAS B HANSEN ET AL: "miRNA-dependent gene silencing involving Ago2-mediated cleavage of a circular antisense RNA", THE EMBO JOURNAL, vol. 30, no. 21, 2 November 2011 (2011-11-02), pages 4414-4422, XP055103967, ISSN: 0261-4189, DOI: 10.1038/emboj.2011.359
- CAPEL B ET AL: "Circular transcripts of the testis-determining gene Sry in adult mouse testis", CELL, CELL PRESS, US, vol. 73, no. 5, 4 June 1993 (1993-06-04), pages 1019-1030, XP024245787, ISSN: 0092-8674, DOI: 10.1016/0092-8674(93)90279-Y [retrieved on 1993-06-04]
- EBERT MARGARET S ET AL: "MicroRNA sponges: competitive inhibitors of small RNAs in mammalian cells", NATURE METHODS, NATURE PUBLISHING GROUP, GB, vol. 4, no. 9, 1 September 2007 (2007-09-01), pages 721-726, XP002560284, ISSN: 1548-7091, DOI: 10.1038/NMETH1079 [retrieved on 2007-08-12]
- THOMAS B. HANSEN ET AL: "Natural RNA circles function as efficient microRNA sponges", NATURE, vol. 495, no. 7441, 27 February 2013 (2013-02-27), pages 384-388, XP055103964, ISSN: 0028-0836, DOI: 10.1038/nature11993
- SEBASTIAN MEMCZAK ET AL: "Circular RNAs are a large class of animal RNAs with regulatory potency", NATURE, vol. 495, no. 7441, 27 February 2013 (2013-02-27), pages 333-338, XP055103972, ISSN: 0028-0836, DOI: 10.1038/nature11928
- YUCHEN LIU ET AL: "Construction of circular miRNA sponges targeting miR-21 or miR-221 and demonstration of their excellent anticancer effects on malignant melanoma cells", THE INTERNATIONAL JOURNAL OF BIOCHEMISTRY & CELL BIOLOGY, vol. 45, no. 11, 11 September 2013 (2013-09-11), pages 2643-2650, XP055103970, ISSN: 1357-2725, DOI: 10.1016/j.biocel.2013.09.003

## Description

### Technical field of the invention

The present invention relates to inhibition of microRNAs (miRNA), other types of RNA and RNA-binding proteins. In particular the present invention relates to nucleic acid sequences that are circular and allow inhibition of the interactions between microRNAs and their RNA target. Also, the present invention relates to nucleic acid sequences that are circular and acts as an RNA blocker or protein decoy.

### Background of the invention

MicroRNAs (miRNA) are small important regulators of gene expression and are currently believed to regulate approximately 70% of human genes.

More than a thousand different miRNA have been characterized in the human genome and they all are assumed to function by a similar mechanism: The miRNAs base-pair with target messenger RNA (mRNA) and recruit nucleases that degrade the targeted RNA from the termini and/or inhibit translation.

In cancer and many other diseases, deregulation of gene-expression is observed and in many cases miRNAs have been shown to play an integral part or even the causative role in disease development.

Therefore, inhibiting miRNA activity is a promising approach not only in potential treatment of the disease but also in achieving a basic understanding of miRNA functions.

Hansen et al (EMBO J, 2011, 30, 4414-22) is a publication by the present inventors in which the function of miR-671 and a circular antisense transcript of CDR1 is examined.

The circular transcript is shown to be cleaved by miR-671, an unusual mechanism for a human miRNA, but no clear function or mechanism is identified.

WO 2010/084371 is a patent application that discloses circular interfering RNA molecules (ciRNA) which comprise sense and antisense strands connected by a loop i.e. they are double stranded.

Hansen et al. (miRNA-dependent gene silencing involving Ago2-mediated cleavage of a circular antisense RNA", THE EMBO JOURNAL, vol. 30, no. 21, 2 November 2011 (2011-11-02), pages 4414-4422), discloses that miRNAs can also regulate gene expression by targeting non-coding antisense transcripts in human cells. Specifically, it is shown that miR-671 directs cleavage of a circular antisense transcript of the Cerebellar Degeneration-Related protein 1 (CDR1) locus in an Ago2-slicer-dependent manner.

Capel et al. (Circular transcripts of the testis-determining gene Sry in adult mouse testis", CELL, vol. 73, no. 5, 4 June 1993 (1993-06-04), pages 1019-1030) discloses a circular RNA comprising 16 target sites for mi R-138. Said RNA comprises a splice acceptor and a splice donor site. Capel et al. further discloses a DNA vector comprising a promoter and 13 kb of the Sry locus and its transfection into COS and SN18 cells and RNA transcribed from said DNA.

WO 2010/029303 A1 discloses a mirtron or a gene capable of expressing a mirtron for use in modifying the expression of a target gene in a mammalian cell to prevent or treat a disease, the sequence of the mirtron comprising: (i) a 5' splice site; (ii) a 3' splice site; (iii) a branch-point recognition sequence; (iv) a 3' polypyrimidine tract greater than 15 nucleotides in length; and (v) an antisense sequence that is at least partially complementary to a sequence in the target gene.

WO 2007/112754 A2 discloses linear single stranded oligonucleotides comprising LNA's at the ends, which is complementary to a human microRNA sequence for use as a medicament.

Ebert et al. (MicroRNA sponges: competitive inhibitors of small RNAs in mammalian cells", NATURE METHODS, vol. 4, no. 9, 1 September 2007 (2007-09-01), pages 721-726) discloses microRNA inhibitors that can be expressed in cells, as RNAs produced from transgenes. Termed 'microRNA sponges', these competitive inhibitors are transcripts expressed from strong promoters, containing multiple, tandem binding sites to a microRNA of interest.

WO 2010/084371 A1 discloses a circular interfering RNA (ciRNA) molecule comprising the sense and the antisense strands of an interfering RNA molecule targeting a gene of interest, wherein said sense and antisense strands are closed at one end by a loop structure and at the other end by a splice junction sequence generated by splicing activity of permuted intron-exon sequences, and wherein the circular RNA molecule inhibits the expression of the gene of interest in cells expressing said gene of interest.

WO 2011/103394 A2 discloses a method of gene inhibition by circular, circular-like, or bidirectional interfering nucleotide (iNA) duplexes. The method can silence one or more gene(s) through RNAi mechanism.

To date, miRNA inhibition is usually performed with linear synthetic and chemically modified so-called anti-miRs.

These anti-miRs, when introduced into cells in high concentration, attach itself to the miRNA and inactivate it.

Disadvantages of these anti-miRs are the high concentration which leads to a higher risk of off-target effects, the problem of delivery of the anti-miRs in a sufficiently high concentration inside the cells and furthermore the linear nature of these anti-miRs allow degradation by exonucleases.

Inhibitors of the specific interactions between microRNAs and their mRNA target, which are produced as a natural RNA by transcription inside the cells, but resistant to degradation by exonucleases, are desired.

### Summary of the invention

An aspect of the invention relates to a single stranded circular RNA polynucleotide which is at least partly complementary to a human or animal microRNA sequence, for use as a medicament; wherein the polynucleotide comprises a region of contiguous nucleobase sequence which is 100% complementary to the human or animal microRNA seed region. Thus, part of the disclosure relates to a RNA polynucleotide which is circular, which is capable of inhibiting the specific interaction between microRNAs and their mRNA target while being resistant to degradation by exonucleases.

Another aspect of the invention relates to a pharmaceutical composition comprising
- a single stranded circular RNA polynucleotide, which is at least partly complementary to a human or animal microRNA sequence, wherein the polynucleotide comprises a region of contiguous nucleobase sequence which is 100% complementary to the human or animal microRNA seed region; and
- at least one of a pharmaceutically acceptable diluent, carrier and adjuvant;
for use as a medicament.

Yet an aspect relates to in vitro use of a single stranded circular RNA polynucleotide as defined according to the invention for blocking and inhibition of RNA.

In here is also disclosed a circular RNA which is at least partly complementary to a human microRNA sequence.

In here is also disclosed a RNA polynucleotide comprising a splice acceptor site (SA) upstream of one or more copies of nucleic acid sequence that is partially complementary to a human microRNA sequence, and a splice donor site (SD) downstream of the nucleic acid sequence that is partially complementary to a human microRNA sequence.

Yet another part of the disclosure relates to provide a polynucleotide including but not limited to a deoxyribonucleic acid (DNA) vector comprising a promoter and sequence encoding the RNA capable of circularization and which contains one or more nucleic acid sequences that are a guide sequence complementary to the seed sequence of a human microRNA sequence. Circularization of the transcript can be achieved by, but not limited to, providing a splice acceptor (SA) site upstream of a splice donor (SD) site in addition to an inverted repeat. Yet another aspect of the present invention is a DNA containing an inverted repeat partially or fully outside the RNA encoding segment to direct the circularization. Yet another aspect of the present invention is to express the RNA from a plasmid, virus or from a stably integrated DNA segment in the genome of the cell. Yet another aspect of the present invention is to obtain the circularization by using engineered RNA self-splicing or by enzymatic or chemical ligation in vitro prior to introduction to cells. Yet another aspect of the present invention is to use other RNA ligase activities in the cell to form circles including tRNA ligase. Yet another aspect of the present invention is to use stable intron lariats as RNA circles.

Yet another part the disclosure relates to a splice acceptor site (SA) upstream of a nucleic acid sequence that is a guide sequence corresponding to the seed sequence of a human microRNA sequence, and a splice donor site (SD) downstream of the nucleic acid sequence that is a guide sequence complementary to the seed sequence of a human microRNA sequence.

Further parts of the present disclosure relates to a cell comprising these polynucleotides and pharmaceutical compositions comprising the circular polynucleotide RNA of the present invention and a pharmaceutically acceptable diluent, carrier, or adjuvant.

Yet a part of the disclosure relates to a method for inactivating the function of microRNA or reducing the effective amount of a microRNA in a cell or an organism or a method for de-repression of a microRNA targeted mRNA in a cell or an organism, comprising administering a pharmaceutical composition of the present invention, to the cell or the organism.

Another part of the disclosure relates to the use of a polynucleotide circular RNA as of the present invention for the manufacture of a medicament for the treatment of a disease or medical disorder associated with the presence or over-expression of the microRNA.

A further part of the disclosure relates to a method for the treatment of a disease or medical disorder associated with the presence or over-expression of the microRNA, comprising the step of administering a pharmaceutical composition of the present invention to a person in need thereof.

### Brief description of the figures

Figure 1
   CiRS-7: a, b, Schematic representation of the genomic locus encoding the human (a) and mouse (b) CiRS-7 and predicted miR-7 and miR-671 target sites (greyscaled according to target site strength, see legend) are shown. Vertebrate conservation scores are depicted and the horizontal black bar represents the genomic location of CiRS-7. In a, zoom-in of a CiRS-7 subregion is shown below. In b, Ago2 HITS-CLIP reads mapped onto CiRS-7 locus are shown. c, CiRS-7 expression vectors; CiRS-7-fs consists of the CiRS-7-exon without flanking sequences and splice sites. CiRS-7-ir includes splice sites and the endogenous flanking genomic sequence (1000 bp upstream and 200 bp downstream). In CiRS-7, a part of the upstream sequence has been inverted and inserted downstream, as illustrated by the directional bar. d, Northern blot with 5 ug of RNA from HEK293 cells transfected with EV (pcDNA3), CiRS-7-ir (pcDNA3-CiR7-ir), CiRS-7 (pcDNA3-CiRS-7) or total RNA from human brain. The blot was probed against CiRS-7 and 18S (loading control). Migration of circular CiRS-7 and vector-based non-circular product (*) is marked. e, Northern blot with 10 ug (agarose northern, upper two panels) or 20 ug (PAGE northern, lower two panels) using RNA from HEK293 cells co-transfected with EV, CiRS-7-fs or CiRS-7 together with miR-7 or miR-769 expression vectors (pJEBB-7 or pJEBB-769, respectively). The blots were hybridized with probes as indicated next to each panel. 18S and miR-15b serves as loading controls.
Figure 2
   Interaction between CiRS-7 and miR-7: a, Immunoprecipitation (IP) of myc-tagged Ago2 from HEK293 cells transfected with either myc-Ago2 and pJEBB-7 or myc-Ago2 and pJEBB-769. Empty vector (EV) serves as myc-Ago2 negative control. CiRS-7 and GAPDH levels are quantified by RT-qPCR and the relative IP/Input ratios are plotted. b, 3' end biotinylated miRNA duplexes were transfected into HEK293 cells. After streptavidin capture, the input and bound fractions were evaluated by RT-qPCR (analysed as in a). c, RNA FISH on HEK293 cells co-transfected with pcDNA3-CiRS-7 and the pCK-7. CiRS-7 and miR-7 probes were labelled with Cy5 and Cy3, respectively. d, IF-FISH on CiRS-7-transfected HEK293 cells with or without miR-7 (pCK-7) co-transfection immunoblotted with hDcplA antibody and hybridized with Cy5-labelled CiRS-7 probe. e, f, In situ hybridization of CiRS-7 showing intense neuronal expression throughout neocortex (NCx), hippocampal subregions CA1 and CA3, and dentate gyrus (DG). Arrows in f point at CiRS-7 expressing interneurons situated at the transition between stratum radiatum (r) and the molecular layer (m) of CA1. g, h, In situ hybridization showing miR-7 expression in neurons and glial cells. Arrows in h point at miR-7 expressing interneurons. miR-7 expressing glial cells are numerous in the corpus callosum (cc) where they are organized in rows between the myelinated fibre bundles. Small miR-7 expressing glial cells are also widely distributed throughout the hippocampal neuropil layers. g, granule cell layer; h, dentate hilus; o, stratum oriens CA1; r, stratum radiatum CA1. Bars: 200 µm (e, g) and 100 µm (f, h).
Figure 3

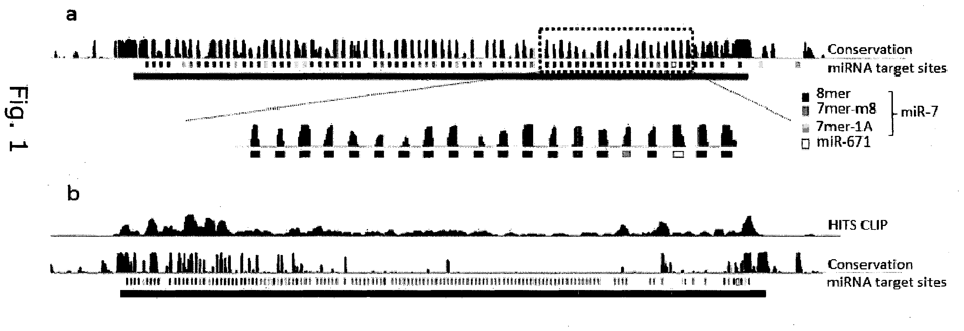

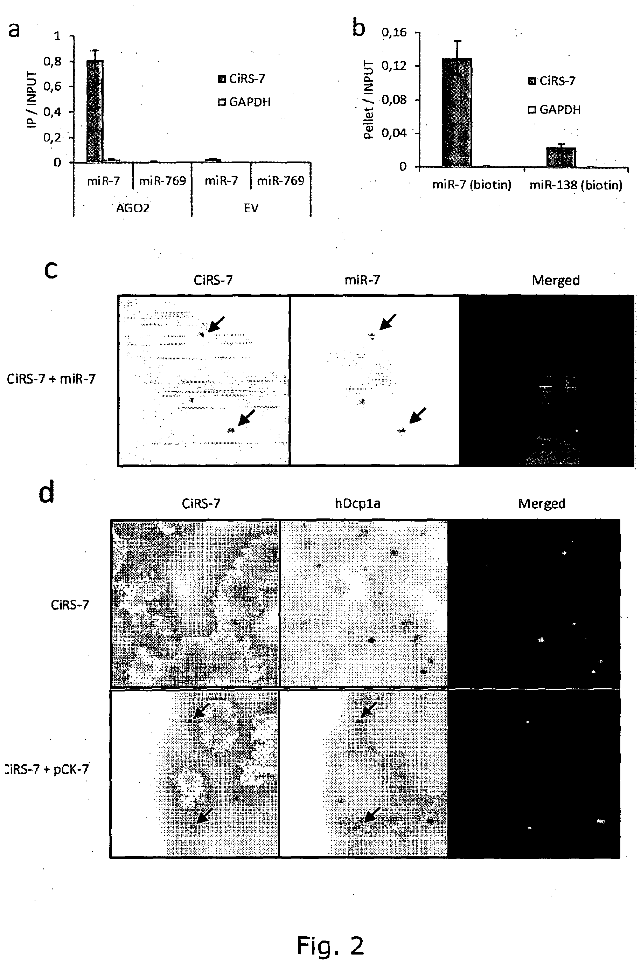

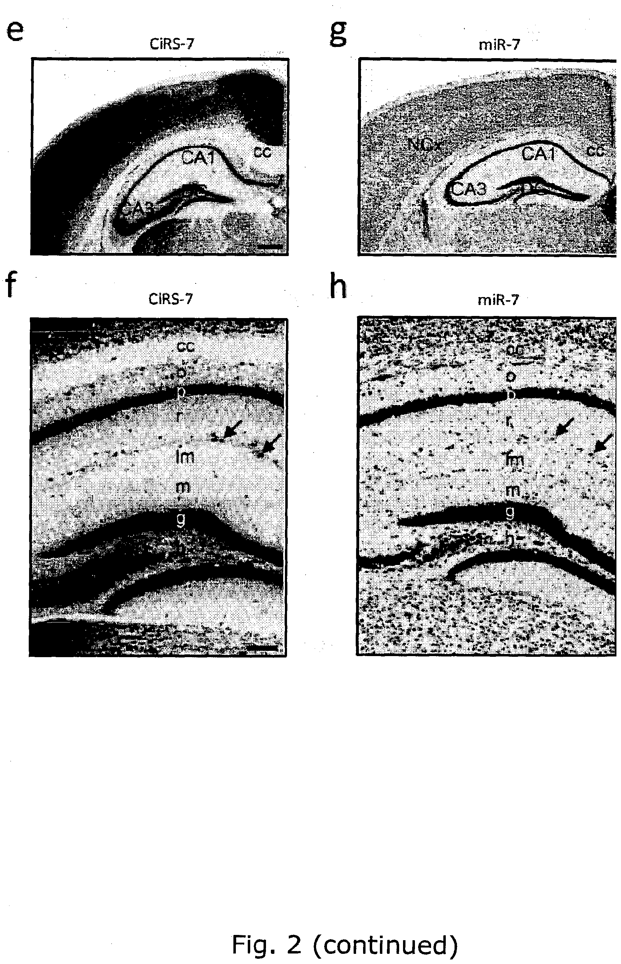

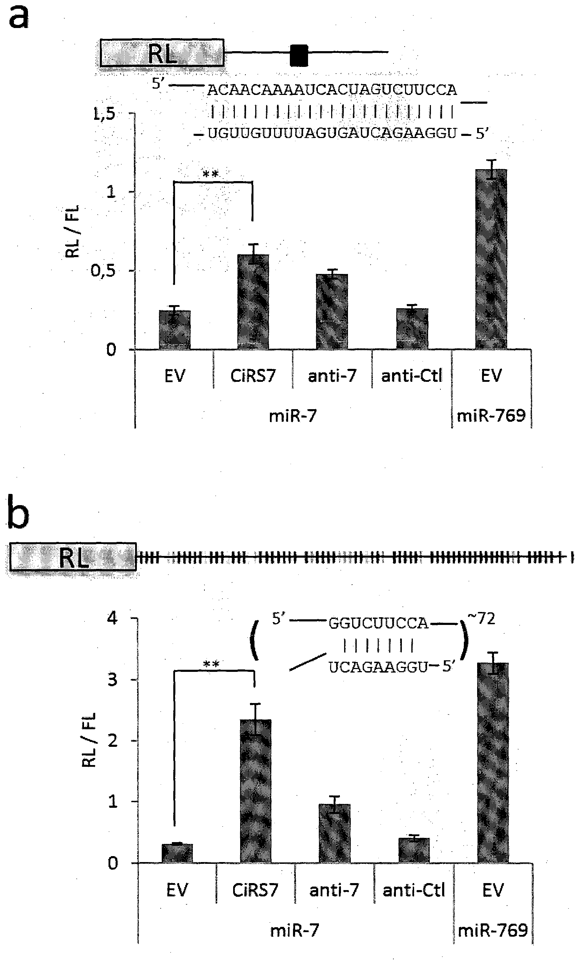

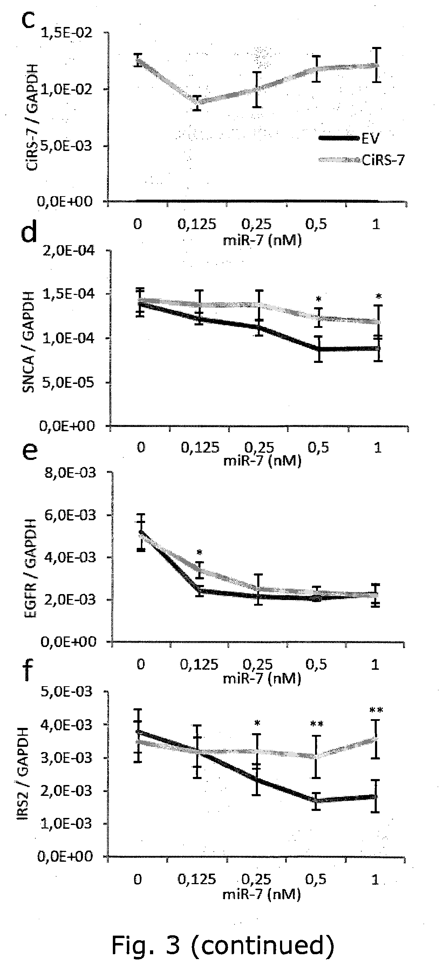

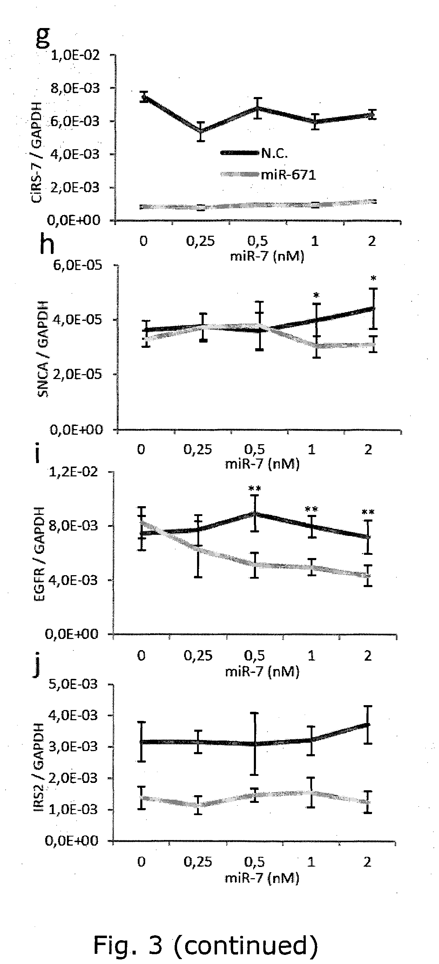

   CiRS-7 sponges miR-7 activity: a, b, Luciferase reporter assays using reporter constructs with either a perfect target site (a) or the entire CiRS-7 sequence (b) inserted into the 3'UTR of renilla luciferase. Forty eight hours after transfection with luciferase expression vectors (psiCheck) and the indicated constructs (in case of anti-miR, a final concentration of 50nM was used), luminescence was measured. The relative levels of renilla luminescence, normalized to firefly, are plotted. c, e, f, g, RT-qPCR quantification of CiRS-7 (c), SNCA (d), EGFR (e) and IRS2 (f) levels in HeLa-CiRS-7 or HeLa-EV cells transfected with gradient concentration of miR-7 as indicated. g, h, I, j, RT-qPCR quantification of CiRS-7 (g), SNCA (h), EGFR (i) and IRS2 (j) levels in HeLa-CiRS-7 cells transfected with miR-671 or N.C. (negative control) 24 hrs prior to transfections with miR-7 gradient. (*, p<0.05; **, p<0.01)
Figure 4
   Circular SRY interacts with miR-138: a, Schematic representation of the SRY locus with splice sites (SA and SD) facilitating the SRY circularization and an open read frame depicted as the black bar. Perpendicular lines represent putative target sites for miR-138 (color code as in Fig 1a). b, Luciferase reporter assays with the entire SRY sequence inserted into the 3'UTR of renilla luciferase. Forty eight hours after transfection with luciferase expression vectors (psiCheck-SRY or psiCheck-ctrl) and pJEBB-138 or pJEBB-769, luminescence was measured. The relative levels of renilla luminescence normalized to firefly are plotted. c, Immunoprecipitation (IP) of myc-tagged Ago2 from HEK293 cells co-transfected with SRY expression vector, myc-Ago2 or empty vector (EV) and pJEBB-138 or pJEBB-769 as denoted. SRY and GAPDH levels are quantified by RT-qPCR and the relative IP/Input ratios are plotted. d, As described in Fig 2b, 3' biotinylated miR-7 or miR-138 was transfected into HEK293 cells along with SRY expression vector. SRY and GAPDH levels were analysed in the input and bound fractions were evaluated by RT-qPCR.
Figure 5, Supplementary Table S1
   Ranked hotspot analysis of HITS-CLIP dataset. Hotspot analysis conducted on HITS-CLIP from mouse brain15. Each hotspot represents a cluster of reads with less than 50bp distance to the closest neighbouring read.
Figure 6, Supplementary Table S2: Primers and Probes.
Figure 7, Supplementary Figure S1
   Streptavidin-aptamer capture of Ago2 and miR-7. a, b, T7-transcripts covering the entire CiRS-7 sequence with or without a streptavidin aptamer (tRSA) sequence in the 5' end (as schematically depicted above), were incubated with cell lysate pre-transfected with either pJEBB-7 or pJEBB-769. After streptavidin capture, the input and bound fraction were analysed by western blot using anti-myc antibody or anti-HuR (control) (a) or by miRNA taqman® qPCR (normalized to INPUT) (b). c, Northern blot with RNA from T7 transcription prior and after biotin-capture probed with CiRS-7.

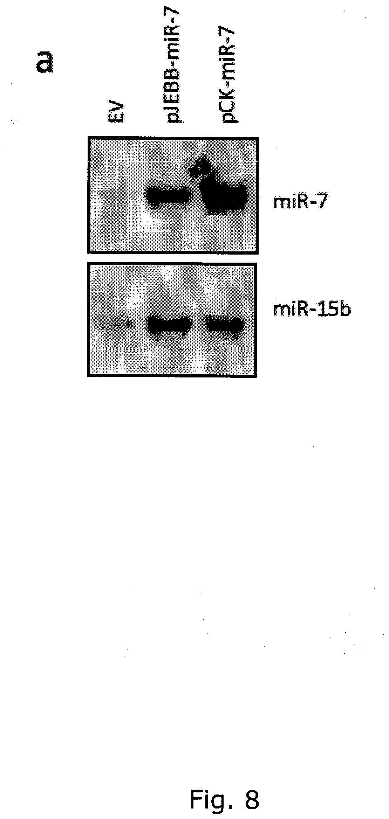

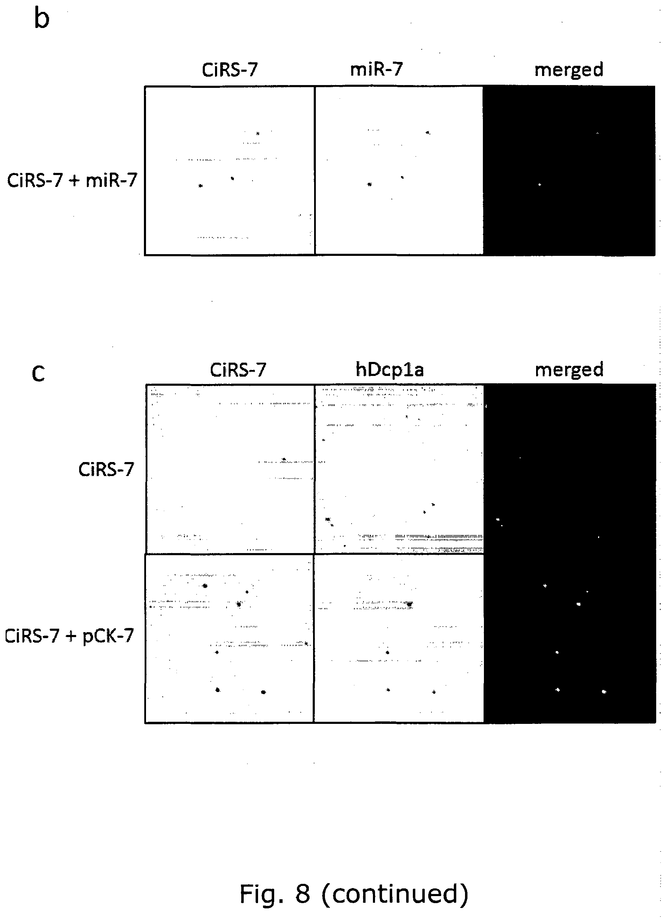

Figure 8, Supplementary Figure S2
   RNA-FISH and IF-FISH on HeLa cells. a, Northern blot with RNA from HEK293 cells transfected with empty vector (EV), pJEBB-7 or pCK-7 validating miRNA biogenesis from plasmid-based expression. The blot was probed against miR-7 and miR-15b (loading control). b, c, RNA-FISH and IF-FISH on HeLa cells transfected with constructs as indicated (as in Fig 2c-d).

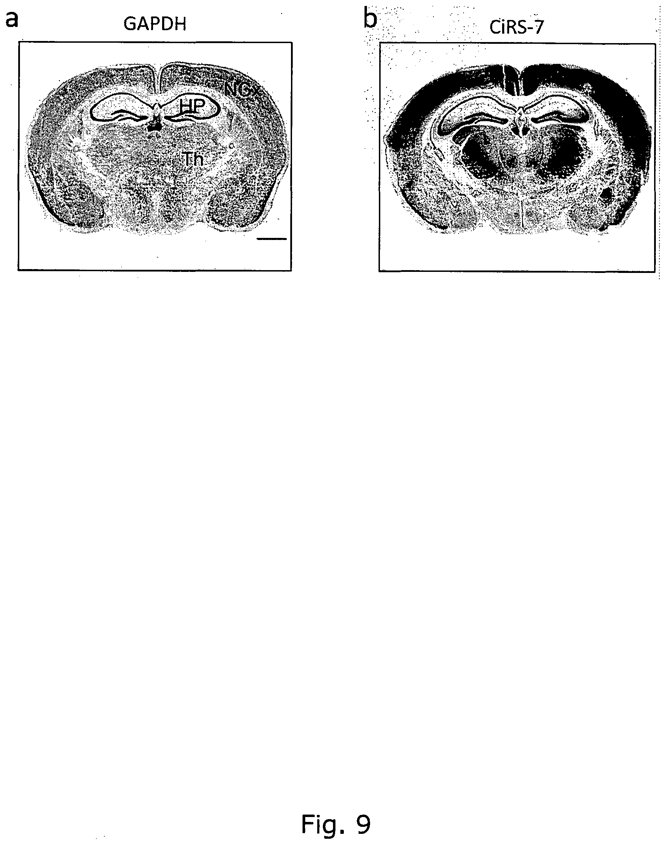

Figure 9, Supplementary Figure S3
   Sub-brain expression of CiRS-7 in mouse. a, b, Mouse brains sections hybridized with alkaline phosphatase-coupled GAPDH mRNA (a) or CiRS-7 (b) probe. HP, hippocampus; NCx, neocortex; Th, thalamus. Bar: 1 mm.

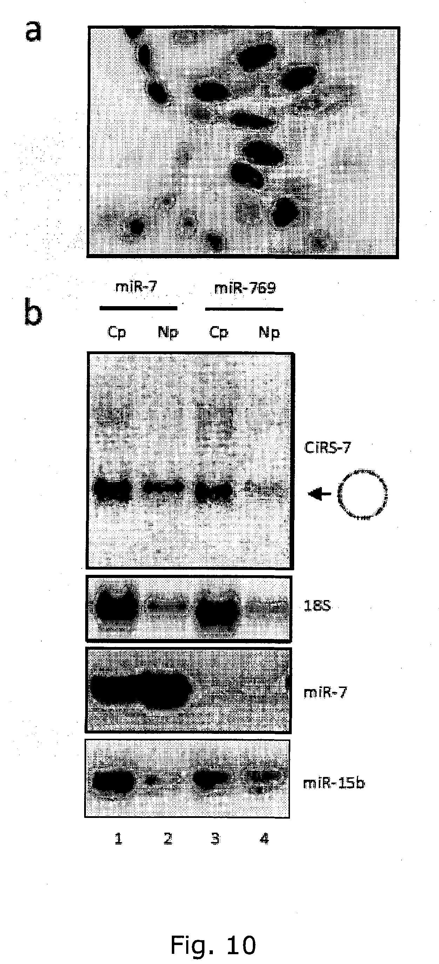

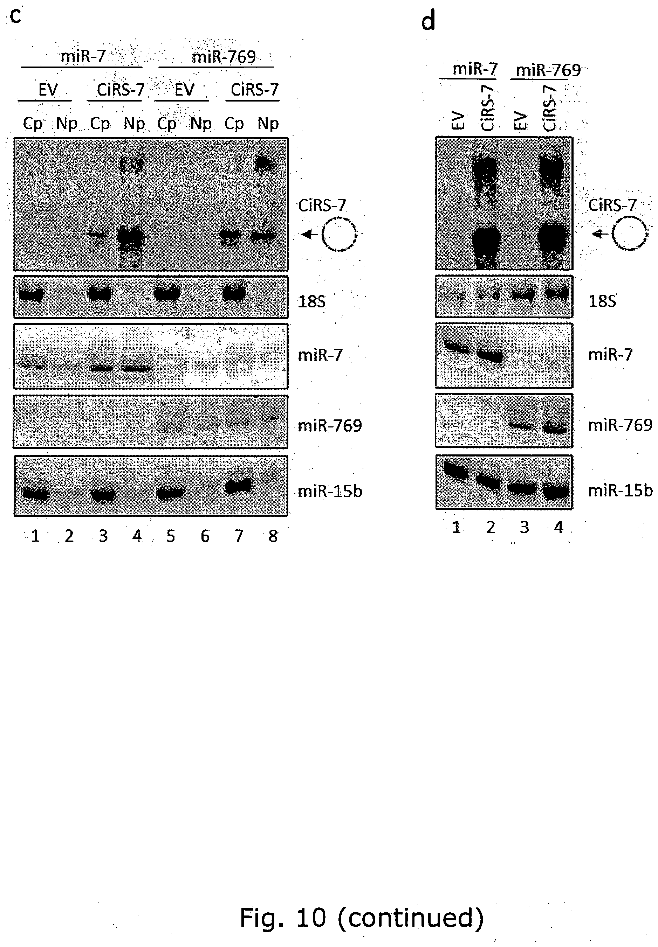

Figure 10, Supplementary Figure S4
   Subcellular localization of CiRS-7 and miR-7: a, Mouse brain section hybridized with alkaline phosphatase-coupled miR-7. b, Stable CiRS-7 expressing HEK293 cells transfected with miR-7 or miR-769 (pJEBB-7 or pJEBB-769, respectively). Forty eight hours after transfection, nuclear (Np) and cytoplasmic (Cp) RNA fractions were analysed by northern blotting. c, HEK293 cells were co-transfected with CiRS-7 or empty vector (EV) together with miR-7 or miR-769 and analysed as in (a). d, Total RNA from HEK293 cells were cotransfected with constructs as indicated and analysed by northern blotting.

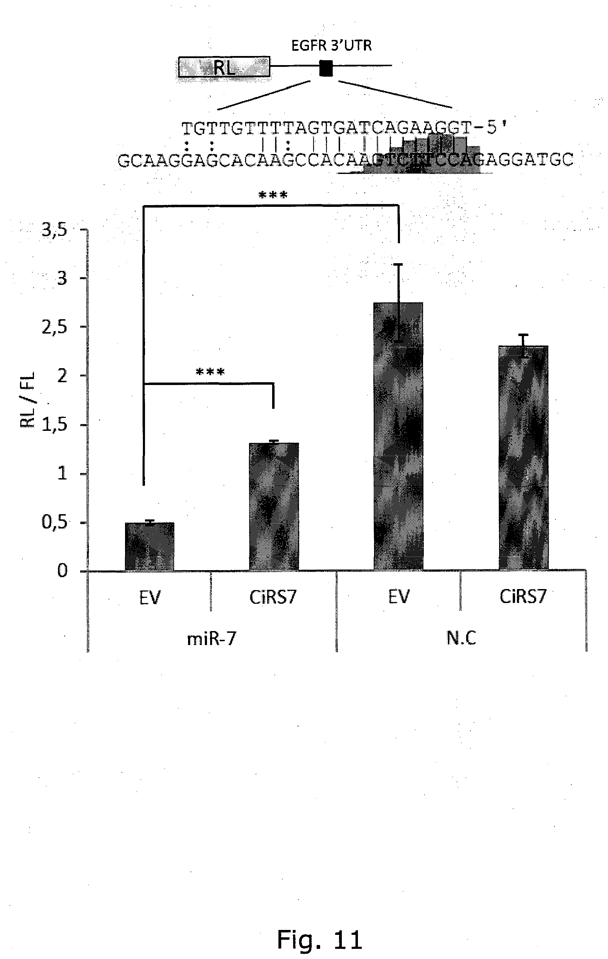

Figure 11, Supplementary Figure S5
   Luciferase reporter assay. The 3'UTR of miR-7 target gene, EGFR, was inserted into psiCheck vector (schematic representation of target site depicted above). HEK293 cells were co-transfected with either 10 nM miR-7 or 10 nM N.C. (negative control) and pcDNA3-CiRS-7 or pcDNA3-EV. Forty eight hours after transfection, relative luminescence was measured and plotted.

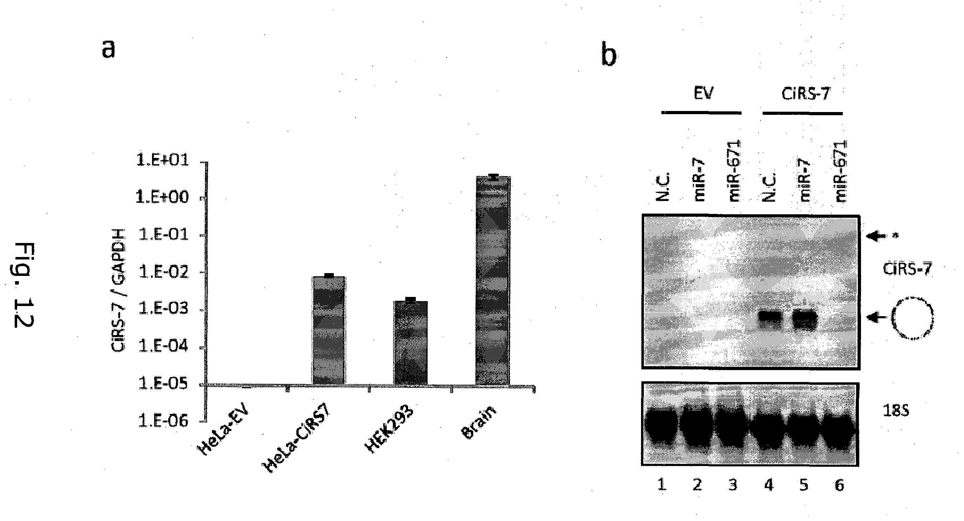

Figure 12, Supplementary Figure S6
   CiRS-7 expression in stable HeLa cells. a, Northern blot using 10 ug RNA from HeLa-EV or Hela-CiRS-7 cells transfected with 10 nM N.C., miR-7 or miR-671. Blot was probed with CiRS-7 and 18S (loading control). The asterix (*) denotes a high-molecular, non-circular RNA species produced from the CiRS-7 construct. b, qRT-PCR on RNA from HeLa-EV, HeLa-CiRS-7, HEK293 cells, and total brain quantifying CiRS-7 levels relative to GAPDH mRNA.

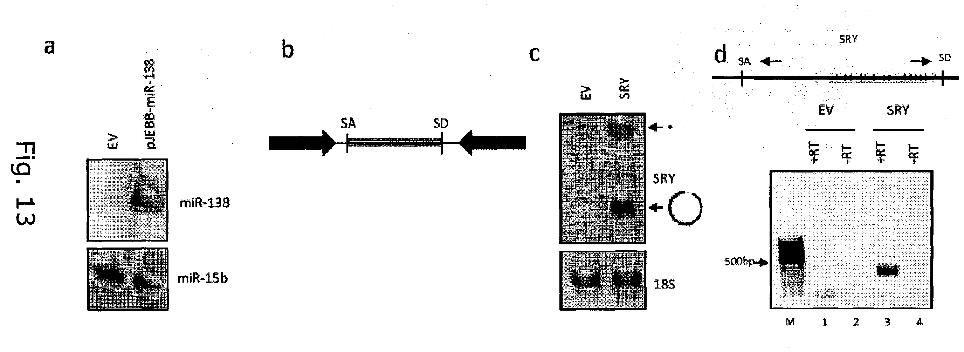

Figure 13, Supplementary Figure S7
   miR-138 and SRY expression. a, Northern blot using 30 ug RNA from HEK293 cells transfected with either empty vector (EV, pJEBB) or pJEBB-138. Blot was probed against miR-138 (upper panel) and miR-15b (loading control, lower panel). b, Schematic representation of SRY expression vector (pcDNA3-SRY) in which the tet-responsive CMV promoter from the pcDNA5/FRT/TO vector has been inserted in an inverted orientation downstream the SRY locus. c, Northern blot using 10 ug RNA from HEK293 cells transfected with either empty vector (EV, pcDNA3) or the SRY expression construct (pcDNA3-SRY). Blot was probed with SRY and 18S (upper and lower panels, respectively). The asterix (*) denotes a high-molecular, non-circular RNA species produced from the SRY construct. d, RT-PCR on RNA from HEK293 cells transfected with either EV or SRY using primers depicted as arrows above.

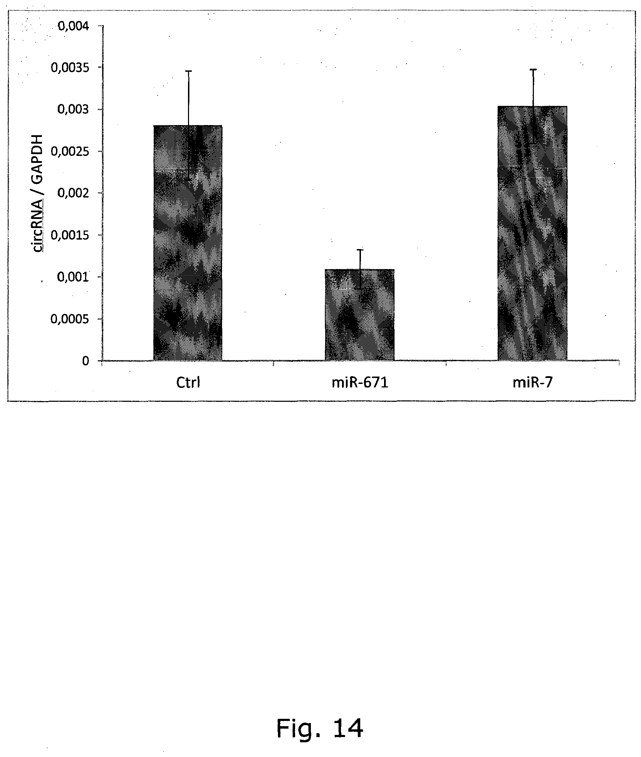

Figure 14 shows quantification of circular RNA (CiRS-7; with ∼70 sponge sites for miR-7 and a target site (cleavage site) for miR-671) levels in HEK293 cells after transfection with 10 nM non-related control microRNA (Ctrl), miR-7 or miR-671 mimics (Applied Biosystems) as indicated using Lipofectamine 2000 (Invitrogen). CiRS-7 levels are plotted relative to *GAPDH.*

The present invention will now be described in more detail in the following.

### Detailed description of the invention

In the present disclosure the inventors present the idea of using circular RNA as efficient and stable miRNA inhibitors (also known as anti-miRs).

A microRNA (abbreviated miRNA) is a short ribonucleic acid (RNA) molecule found in eukaryotic cells.

A microRNA molecule has an average of 22 nucleotides.

miRNAs are post-transcriptional regulators that bind to partly complementary sequences on target messenger RNA transcripts (mRNAs), usually resulting in translational repression or target degradation and gene silencing.

miRNAs have also been reported to act as transcriptional silencers potentially through epigenetic effects.

The human genome encode over 1000 miRNAs, which target more than 70% of mammalian genes and are abundant in many human cell types.

The above mentioned approach has several advantages:
The circular nature of the RNA inhibitor will enable it to bind the miRNA but, remains resistant to miRNA destabilization mediated by for example but not limited to exonucleases. As a consequence the miRNA becomes unavailable for binding to and initiate degradation of their targeted RNAs such as mRNAs. Such target RNAs will thus be relieved of any repression caused by the said miRNA.

The sequences in the circular RNA can readily be exchanged to target any expressed miRNA of choice.

The circular RNA is stabilized against normal RNA degradation in the cells which usually occur from the termini of a transcript. Therefore, this approach does not depend on potentially toxic and artificial building blocks normally used to increase RNA stability in target cells.

Such RNAs with the capacity to inhibit interactions between a microRNA and its mRNA target are in the present context also called circular microRNA sponges and the effect is called "sponging".

The inventors have devised a simple method, an insertion of inverted repeats flanking the sequence encoding the circular RNA, to produce large amounts circular RNA intracellularly from a conventional expression vector.

Circular RNAs are produced exploiting the natural machinery for RNA biogenesis.

They can be expressed from a DNA vector, other RNA molecules, transient or stable viral transduction, or from transgenes incorporated in the genome. Thus it is possible to use inducible or tissue-specific promoters for expression of circular RNA molecules.

It is possible to include multiple target sites against one or more miRNA within the circular RNA inhibitor and thereby tether and inactivate several different miRNAs at once using only one construct.

It is possible to control the presence of the circular RNA by targeting it with a miRNA or small interfering RNA perfectly complementary to the circular RNA. For example, a natural miRNA-671 exists that can cleave and quickly remove the circular RNA directed to microRNA-7.

This enables a tight control of circular RNA expression and an efficient antidote if a treatment based on circular RNAs is desired to be terminated.

### RNA polynucleotide characteristics

An aspect of the invention relates to a single stranded circular RNA polynucleotide which is at least partly complementary to a human or animal microRNA sequence, for use as a medicament; wherein the polynucleotide comprises a region of contiguous nucleobase sequence which is 100% complementary to the human or animal microRNA seed region.

In here is also disclosed a polynucleotide RNA that comprises a sequence which is at least partly complementary to a human microRNA sequence, wherein said RNA polynucleotide is circular.

In animals miRNA complementarity typically encompasses the 5' bases 2-7 of the microRNA, the microRNA seed region, and one miRNA can therefore target many different sites on the same mRNA or on many different mRNAs.

MicroRNAs are therefore promiscuous.

The sequence which is at least partly complementary or 100% complementary to a human microRNA sequence is also called the anti-miR sequence or blockmir sequence.

Another embodiment of the present invention relates to the RNA polynucleotide of the present invention where at least part of the polynucleotide RNA is complementary to the mature human microRNA sequence.

Another embodiment of the present invention relates to a polynucleotide comprising an antisense sequence to the seed sequence of a microRNA, wherein the polynucleotide is circular.

The polynucleotide of the present invention can be purified or isolated from a sample, a cell culture or similar.

Thus, in a further embodiment of the present invention is the RNA polynucleotide of the present invention an isolated and/or purified polynucleotide.

In yet another embodiment of the present invention the RNA polynucleotide is capable of recruiting the RNAi machinery.

In another embodiment of the present invention the RNA polynucleotide is not capable of recruiting the RNAi machinery.

Herein the RNAi machinery refers to the cellular components necessary for the activity of siRNAs and microRNAs or for the RNAi pathway.

A major player of the RNAi machinery is the RNA induced silencing complex (the RISC complex).

Herein, nucleobase, base, nucleic acid, refer to the base moiety of a nucleotide unit. The nucleotide unit may be of DNA, RNA, INA, LNA, 2'F and 2'MeO modified RNA or any other nucleic acid or any nucleic acid capable of specific base pairing. The nucleotide unit may also be part of PNA (peptide nucleic acid).

In some embodiments, the nucleotide unit may be a universal nucleotide unit.

When referring the length of a sequence, reference may be made to the number of nucleotide units or to the number of bases.

When referring to a complementary sequence, G pairs to C, A pairs to T and U and vice versa. In a preferred embodiment, G also pairs to U and vice versa to form a so-called wobble base pair.

A wobble base pair is a non-Watson-Crick base pairing between two nucleotides in RNA molecules.

The four main wobble base pairs are guanine-uracil, inosine-uracil, inosine-adenine, and inosine-cytosine (G-U, I-U, I-A and I-C).

Thus, in another preferred embodiment, the base inosine (I) may comprise a part of a microRNA or any other polynucleotide of the invention and might optionally base pair to A, C and U. In still another preferred embodiment, universal bases may be used.

### Universal bases can typically base pair to G, C, A, U and T.

Often universal bases do not form hydrogen bonds with the opposing base on the other strand.

In still another preferred embodiment, a complementary sequence refers to a contiguous sequence exclusively of Watson-Crick base pairs.

The polynucleotide of the invention may also recruit RNase H to mediate cleavage of the target RNA.

A lariat circle is formed during RNA spicing by the 2'OH of a specific adenosine in the intron attacking the 5' splice site, thereby forming the said lariat structure

The disclosure also provides methods for providing microRNA targets of target RNAs, methods for validating microRNA target regions of target RNAs and methods of modulating the activity of target RNAs using polynucleotides of the invention.

In here is also disclosed a circular polynucleotide RNA comprising an antisense sequence that comprises a guide sequence corresponding partially to the sequence of a microRNA, with the proviso that the polynucleotide is not a microRNA.

Such a polynucleotide is of interest because it can be used to block the target region of a target RNA, said target region being involved in microRNA regulation of the target RNA, thereby relieving the target RNA from the repression of specific miRNAs.

The circular polynucleotide of the invention can also inhibit or block any other RNA in the cells. Therefore, in another preferred embodiment, the circular polynucleotide of the invention can be used to inhibit ribosomal RNAs, tRNAs, snRNAs, snoRNAs, siRNA, long non-coding RNA, piRNA and specific mRNA, referred to as RNA blocker. In this preferred embodiment, the circular polynucleotide of the invention can also block miRNA target sites relieving the target from miRNA-mediated repression.

The inhibitory effect of the circular polynucleotide of the invention is not limited to RNA. Providing one or more binding sites for proteins can lead to a sequestering and inactivation of protein function referred to as protein decoy. Another preferred embodiment is to insert RNA sequences that bind cellular proteins with the aim to inactivate them. Yet another aspect and an embodiment is to use the circular polynucleotide of the invention as a scaffold to sequester proteins to enhance function, e.g. by dimerization or multimerization of the same protein or by bringing multiple proteins together to enhance function.

In a preferred embodiment, the circular polynucleotide of the invention comprise a sequence selected from the group consisting of sequences that are capable of base pairing to the complementary sequence of a sequence selected from the group consisting of position 1-22, position 1-21, position 1-20, position 1-19, position 1-18, position 1-17, position 1-16, position 1-15, position 1-14, position 1-13, position 1-12, position 1-11, position 1-10, position 1-9, position 1-8, position 1-7, position 1-6, position 2-22, position 2-21, position 2-20, position 2-19, position 2-18, position 2-17, position 2-16, position 2-15, position 2-14, position 2-13, position 2-12, position 2-11, position 2-10, position 2-9, position 2-8, position 2-7, position 3-22, position 3-21, position 3-20, position 3-19, position 3-18, position 3-17, position 3-16, position 3-15, position 3-14, position 3-13, position 3-12, position 3-11, position 3-10, position 3-9, position 3-8, position 4-22, position 4-21, position 4-20, position 4-19, position 4-18, position 4-17, position 4-16, position 4-15, position 4-14, position 4-13, position 4-12, position 4-11, position 4-10, position 4-9, position 5-22, position 5-21, position 5-20, position 5-19, position 5-18, position 5-17, position 5-16, position 5-15, position 5-14, position 5-13, position 5-12, position 5-11, position 5-10, position 6-22, position 6-21, position 6-20, position 6-19, position 6-18, position 6-17, position 6-16, position 6-15, position 6-14, position 6-13, position 6-12, position 6-11, position 7-22, position 7-21, position 7-20, position 7-19, position 7-18, position 7-17, position 7-16, position 7-15, position 7-14, position 7-13, position 7-12, position 8-22, position 8-21, position 8-20, position 8-19, position 8-18, position 8-17, position 8-16, position 8-15, position 8-14, position 8-13, position 9-22, position 9-21, position 9-20, position 9-19, position 9-18, position 9-17, position 9-16, position 9-15, position 9-14, position 10-22, position 10-21, position 10-20, position 10-19, position 10-18, position 10-17, position 10-16, position 10-15, position 11-22, position 11-21, position 11-20, position 11-19, position 11-18, position 11-17, position 11-16, position 12-22, position 12-21, position 12-20, position 12-19, position 12-18, position 12-17, position 13-22, position 13-21, position 13-20, position 13-19, position 13-18, position 14-22, position 14-21, position 14-20, position 14-19, position 15-22, position 15-21, position 15-20, position 16-22, position 16-21, position 17-22"of a human microRNA, preferably defined by any of SEQ ID NOs: 51-86.

In a preferred embodiment, the circular polynucleotide of the invention comprise a sequence selected from the group consisting of sequences that are capable of base pairing to the complementary sequence of a sequence selected from the group consisting of position 1-22, position 1-21, position 1-20, position 1-19, position 1-18, position 1-17, position 1-16, position 1-15, position 1-14, position 1-13, position 1-12, position 1-11, position 1-10, position 1-9, position 1-8, position 1-7, position 1-6, position 2-22, position 2-21, position 2-20, position 2-19, position 2-18, position 2-17, position 2-16, position 2-15, position 2-14, position 2-13, position 2-12, position 2-11, position 2-10, position 2-9, position 2-8, position 2-7, position 3-22, position 3-21, position 3-20, position 3-19, position 3-18, position 3-17, position 3-16, position 3-15, position 3-14, position 3-13, position 3-12, position 3-11, position 3-10, position 3-9, position 3-8, position 4-22, position 4-21, position 4-20, position 4-19, position 4-18, position 4-17, position 4-16, position 4-15, position 4-14, position 4-13, position 4-12, position 4-11, position 4-10, position 4-9, position 5-22, position 5-21, position 5-20, position 5-19, position 5-18, position 5-17, position 5-16, position 5-15, position 5-14, position 5-13, position 5-12, position 5-11, position 5-10, position 6-22, position 6-21, position 6-20, position 6-19, position 6-18, position 6-17, position 6-16, position 6-15, position 6-14, position 6-13, position 6-12, position 6-11, position 7-22, position 7-21, position 7-20, position 7-19, position 7-18, position 7-17, position 7-16, position 7-15, position 7-14, position 7-13, position 7-12, position 8-22, position 8-21, position 8-20, position 8-19, position 8-18, position 8-17, position 8-16, position 8-15, position 8-14, position 8-13, position 9-22, position 9-21, position 9-20, position 9-19, position 9-18, position 9-17, position 9-16, position 9-15, position 9-14, position 10-22, position 10-21, position 10-20, position 10-19, position 10-18, position 10-17, position 10-16, position 10-15, position 11-22, position 11-21, position 11-20, position 11-19, position 11-18, position 11-17, position 11-16, position 12-22, position 12-21, position 12-20, position 12-19, position 12-18, position 12-17, position 13-22, position 13-21, position 13-20, position 13-19, position 13-18, position 14-22, position 14-21, position 14-20, position 14-19, position 15-22, position 15-21, position 15-20, position 16-22, position 16-21, position 17-22"of a human microRNA, preferably defined by any of SEQ ID NOs: 51-86 and are not capable of forming a consecutive base pair with the neighbouring nucleotide of either side of the aforementioned positions.

The phrase "are capable of base pairing to" is related to the term complementary sequence. I.e. a first sequence is capable of base pairing to a second sequence, which is complementary to the first sequence.

In another preferred embodiment, the circular polynucleotide of the invention consists of an antisense sequence selected from the group consisting of sequences that are capable of base pairing to the sequence or the complementary sequence of a sequence selected from the group consisting selected from the group consisting of position 1-22, position 1-21, position 1-20, position 1-19, position 1-18, position 1-17, position 1-16, position 1-15, position 1-14, position 1-13, position 1-12, position 1-11, position 1-10, position 1-9, position 1-8, position 1-7, position 1-6, position 2-22, position 2-21, position 2-20, position 2-19, position 2-18, position 2-17, position 2-16, position 2-15, position 2-14, position 2-13, position 2-12, position 2-11, position 2-10, position 2-9, position 2-8, position 2-7, position 3-22, position 3-21, position 3-20, position 3-19, position 3-18, position 3-17, position 3-16, position 3-15, position 3-14, position 3-13, position 3-12, position 3-11, position 3-10, position 3-9, position 3-8, position 4-22, position 4-21, position 4-20, position 4-19, position 4-18, position 4-17, position 4-16, position 4-15, position 4-14, position 4-13, position 4-12, position 4-11, position 4-10, position 4-9, position 5-22, position 5-21, position 5-20, position 5-19, position 5-18, position 5-17, position 5-16, position 5-15, position 5-14, position 5-13, position 5-12, position 5-11, position 5-10, position 6-22, position 6-21, position 6-20, position 6-19, position 6-18, position 6-17, position 6-16, position 6-15, position 6-14, position 6-13, position 6-12, position 6-11, position 7-22, position 7-21, position 7-20, position 7-19, position 7-18, position 7-17, position 7-16, position 7-15, position 7-14, position 7-13, position 7-12, position 8-22, position 8-21, position 8-20, position 8-19, position 8-18, position 8-17, position 8-16, position 8-15, position 8-14, position 8-13, position 9-22, position 9-21, position 9-20, position 9-19, position 9-18, position 9-17, position 9-16, position 9-15, position 9-14, position 10-22, position 10-21, position 10-20, position 10-19, position 10-18, position 10-17, position 10-16, position 10-15, position 11-22, position 11-21, position 11-20, position 11-19, position 11-18, position 11-17, position 11-16, position 12-22, position 12-21, position 12-20, position 12-19, position 12-18, position 12-17, position 13-22, position 13-21, position 13-20, position 13-19, position 13-18, position 14-22, position 14-21, position 14-20, position 14-19, position 15-22, position 15-21, position 15-20, position 16-22, position 16-21, position 17-22"of a human microRNA, preferably defined by any of SEQ ID NOs: 51-86.

Preferably, the polynucleotide of the invention comprises an antisense or sense sequence that comprises a contiguous stretch of nucleotides, complementary or identical to the micro RNA target sequence of a target RNA selected from the group consisting of: less than 60 bases, less than 50 bases, less than 40 bases, less than 39 bases, less than 38 bases, less than 37 bases, less than 36 bases, less than 35, less than 34 bases, less than 33 bases, less than 32 bases, less than 31 bases, bases, less than 30 bases, less than 29 bases, less than 28 bases, less than 27 bases, less than 26 bases, less than 25 bases, less than 24 bases, less than 23 bases, less than 22 bases, less than 21 bases, less than 20 bases, less than 19 bases, less than 18 bases, less than 17 bases, less than 16 bases, less than 15 bases, less than 14 bases, less than 13 bases, less than 12 bases, less than 11 bases, less than 10 bases, less than 9 bases, less than 8 bases, less than 7 bases, more than 60 bases, more than 50 bases, more than 40 bases, more than 39 bases, more than 38 bases, more than 37 more, more than 36 bases, more than 35, more than 34 bases, more than 33 bases, more than 32 bases, more than 31, more than 30 bases, more than 29 bases, more than 28 bases, more than 27 bases, more than 26 bases, more than 25 bases, more than 24 bases, more than 23 bases, more than 22 bases, more than 21 bases, more than 20 bases, more than 19 bases, more than 18 bases, more than 17 bases, more than 16 bases, more than 15 bases, more than 14 bases, more than 13 bases, more than 12 bases, more than 11 bases, more than 10 bases, more than 9 bases, more than 8 bases, more than 7 bases, more than 6 bases and more than 5 bases.

A contiguous stretch of bases is intended to mean a non-interrupted sequence of bases that all fit into a duplex formed between the polynucleotide of the invention and the target RNA. I.e. there are preferably no bulges in the duplex and it is preferred that the sequences are complementary (see the definition of complementary sequences above).

Most preferred is perfect Watson-Crick duplex between the polynucleotide of the invention and target region of the target RNA.

The terms contiguous and continuous are used interchangeably herein.

In another embodiment, the polynucleotides of the invention comprise an antisense or sense sequence that comprises a contiguous stretch of bases complementary to the microRNA target sequence of a target RNA, said contiguous stretch of bases being selected from the group consisting of between 10 and 14 bases, between 12 and 16 bases, between 14 and 18 bases, between 16 and 20, between 10 and 25 bases, between 12 and 24 bases, between 14 and 22 bases, between 15 and 22 bases and between 15 and 20 bases.

More preferred is a contiguous stretch of bases between 7 and 25 bases.

Most preferred is several contiguous stretches of bases between 5 and 20 bases.

Also preferred is an adenosine (A) residue in the polynucleotide opposing the 5' nucleotide in the microRNA.

The preferred microRNA targeted by the present polynucleotides are: miR-7-5p (SEQ ID NO 51), miR-7-1-3p (SEQ ID NO 52), miR-7-2-3p (SEQ ID NO 53), miR-21-5p (SEQ ID NO 54), miR-21-3p (SEQ ID NO 55), miR-96-5p (SEQ ID NO 56), miR-96-3p (SEQ ID NO 57), miR-185-5p (SEQ ID NO 58), miR-185-3p (SEQ ID NO 59), miR-324-5p (SEQ ID NO 60), miR-324a-3p (SEQ ID NO 61), miR-133b (SEQ ID NO 62), miR-205-5p (SEQ ID NO 63), miR-205-3p (SEQ ID NO 64), miR-200a-5p (SEQ ID NO 65), miR-200a-3p (SEQ ID NO 66), miR-200b-5p (SEQ ID NO 67), miR-200b-3p (SEQ ID NO 68), miR-200c-5p (SEQ ID NO 69), miR-200c-3p (SEQ ID NO 70), miR-141-5p (SEQ ID NO 71), miR-141-3p (SEQ ID NO 72), miR-429 (SEQ ID NO 73), miR-132-5p (SEQ ID NO 74), miR-132-3p (SEQ ID NO 75), miR-134 (SEQ ID NO 76), miR-124-5p (SEQ ID NO 77), miR-124-3p (SEQ ID NO 78), miR-155-5p (SEQ ID NO 79), miR-155-3p (SEQ ID NO 80), miR-221-5p (SEQ ID NO 81), miR-221-3p (SEQ ID NO 82), miR-222-5p (SEQ ID NO 83), miR-222-3p (SEQ ID NO 84), miR-122-5p (SEQ ID NO 85), miR-122-3p (SEQ ID NO 86).

### Sequence identity

The polynucleotide of the present invention can also be defined by the sequence identity with a human microRNA or the complementary sequence of a human microRNA.

Two sequences that are complementary at less than 100% of the sequence are defined as being at least partly complementary.

As commonly defined "identity" is here defined as sequence identity between genes or proteins at the nucleotide, base or amino acid level, respectively. Specifically a DNA and an RNA sequence are considered identical if the transcript of the DNA sequence can be transcribed to the identical RNA sequence.

Thus, in the present context "sequence identity" is a measure of identity between proteins at the amino acid level and a measure of identity between nucleic acids at nucleotide level.

The protein sequence identity may be determined by comparing the amino acid sequence in a given position in each sequence when the sequences are aligned. Similarly, the nucleic acid sequence identity may be determined by comparing the nucleotide sequence in a given position in each sequence when the sequences are aligned.

To determine the percent identity of two amino acid sequences or of two nucleic acids, the sequences are aligned for optimal comparison purposes (e.g., gaps may be introduced in the sequence of a first amino acid or nucleic acid sequence for optimal alignment with a second amino or nucleic acid sequence).

The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity = # of identical positions/total # of positions (e.g., overlapping positions) x 100).

In one embodiment the two sequences are the same length.

In another embodiment the two sequences are of different length and gaps are seen as different positions.

One may manually align the sequences and count the number of identical amino acids. Alternatively, alignment of two sequences for the determination of percent identity may be accomplished using a mathematical algorithm. Such an algorithm is incorporated into the NBLAST and XBLAST programs of (Altschul et al. 1990). BLAST nucleotide searches may be performed with the NBLAST program, score = 100, wordlength = 12, to obtain nucleotide sequences homologous to a nucleic acid molecules of the invention. BLAST protein searches may be performed with the XBLAST program, score = 50, wordlength = 3 to obtain amino acid sequences homologous to a protein molecule of the invention.

To obtain gapped alignments for comparison purposes, Gapped BLAST may be utilised.

Alternatively, PSI-Blast may be used to perform an iterated search which detects distant relationships between molecules. When utilising the NBLAST, XBLAST, and Gapped BLAST programs, the default parameters of the respective programs may be used. See http://www.ncbi.nlm.nih.gov. Alternatively, sequence identity may be calculated after the sequences have been aligned e.g. by the BLAST program in the EMBL database (www.ncbi.nlm.gov/cgi-bin/BLAST). Generally, the default settings with respect to e.g. "scoring matrix" and "gap penalty" may be used for alignment. In the context of the present invention, the BLASTN and PSI BLAST default settings may be advantageous.

The percent identity between two sequences may be determined using techniques similar to those described above, with or without allowing gaps. In calculating percent identity, only exact matches are counted.

An embodiment of the present invention thus relates to sequences of the present invention that has some degree of sequence variation.

In one embodiment of the present invention has the sequence identity of the anti-miR or blockmir sequence comprised within the polynucleotide of the present invention 100 % sequence identity with the anti-miR or the microRNA sequence, such as at least 99%, such as at least 95%, such as at least 90%, such as at least 85%, such as at least 80%, such as at least 75%, such as at least 70%, such as at least 65%, such as at least 60%, such as at least 55%, such as at least 50%, such as at least 45%, such as at least 40%, such as at least 35%, such as at least 30%, such as at least 25% sequence identity.

In another embodiment of the present invention are 7 out of 7 nucleotides at the 5' end overlapping, such as at least 6 out of 7, such as at least 5 out of 7, such as at least 4 out of 7, such as at least 3 out of 7, such as at least 2 out of 7 nucleotides at the 5' end.

In one embodiment of the present invention refers this complementarity to the match between the seed or anti-seed sequence of the microRNA and the sequence comprised within the polynucleotide which is at least party complementary.

It should be noted that while the sequences in the present application are both RNA and DNA sequences, the present invention contemplates the corresponding RNA sequence, and DNA and RNA complementary sequences as well.

Thus, in cases where a DNA sequence is mentioned refers such DNA sequence also to the RNA equivalent i.e. with Ts exchanged with Us as well as their complimentary sequences.

### Cassettes, vectors and expression of RNA of the invention

An expression vector, otherwise known as an expression construct, is generally a plasmid or a virus or virus derived vector (such as but not limited to an adenoviral, retroviral, baculoviral or lentiviral vectors) that is used to introduce a specific gene (in the present context the DNA molecule of the present invention) into a target cell.

Once the expression vector is inside the cell, it can express an RNA which subsequently may be translated into a protein. The circular RNA product will usually not be translated due to the lack of 5'-cap and poly-A tail. However, introduction of internal ribosome binding sites will potentially enable protein production from circular RNA products

However, in the present case the expression vector may encode the RNA of the present invention which does not encode a protein because there is no poly-A tail.

Thus, in here is also disclosed an expression vector which encodes a RNA that cannot be translated into a protein.

The plasmid is frequently engineered to contain regulatory sequences that act as enhancer and promoter regions and lead to efficient transcription of the gene carried on the expression vector.

The goal of a well-designed expression vector is the production of sufficient amounts of stable circular RNA to exert an effect as microRNA inhibitor, target RNA blocker or as protein decoy.

Expression vectors are basic tools for biotechnology and the production of proteins such as insulin that are important for medical treatments of specific diseases like diabetes.

Thus, a part of the disclosure relates to an expression vector comprising a DNA molecule as described above.

A cassette is the expression vector that is prepared for insertion or the DNA molecule of the present invention.

Thus, invention part of the disclosure relates to a cassette that is capable of expressing the RNA molecule of the present invention.

The cassette or expression vector may be several sizes.
Thus, in one embodiment of the present invention is the size of the cassette or expression vector less than 5 kb, such as less than 3 kb, such as less than 2 kb, such as less than 1 kb.

In another part of the disclosure is the size 200-1500 bases, such as 300-1200 bases, such as 400-1000 bases, such as 500-750 bases.

Another part of the disclosure provides a cassette vector for expressing the nucleic acid sequences of the present invention, comprising, inserted therein, the nucleic acid sequence according to the invention and having an active promoter upstream thereof.

A part of the disclosure relates to a DNA polynucleotide comprising a promoter, a nucleic acid sequence that is complementary to a human microRNA sequence, a splice acceptor site (SA) upstream of the nucleic acid sequence that is complementary to a human microRNA sequence, and a splice donor site (SD) downstream of the nucleic acid sequence that is complementary to a human microRNA sequence.

Another part of the disclosure relates to a DNA polynucleotide comprising a promoter a nucleic acid sequence that is a guide sequence corresponding to the seed sequence of a human microRNA sequence, a splice acceptor site (SA) upstream of the nucleic acid sequence that is a guide sequence corresponding to the seed sequence of a human microRNA sequence, and a splice donor site (SD) downstream of the nucleic acid sequence that is a guide sequence corresponding to the seed sequence of a human microRNA sequence.

The DNA may comprise two or more of the nucleic acids of the present invention.

Thus, a further part of the disclosure relates to a DNA polynucleotide comprising a promoter, two or more nucleic acid sequences that is selected from the group consisting of a nucleic acid sequence that is complementary to a human microRNA sequence and a nucleic acid sequence that is a guide sequence corresponding to the seed sequence of a human microRNA sequence, a splice acceptor site (SA) upstream of the two or more nucleic acid sequences that is selected from the group consisting of a nucleic acid sequence that is complementary to a human microRNA sequence and a nucleic acid sequence that is a guide sequence corresponding to the seed sequence of a human microRNA sequence, and a splice donor site (SD) downstream of the two or more nucleic acid sequences that is selected from the group consisting of a nucleic acid sequence that is complementary to a human microRNA sequence and a nucleic acid sequence that is a guide sequence corresponding to the seed sequence of a human microRNA sequence.

Yet another part of the disclosure relates to a DNA polynucleotide comprising a promoter, a nucleic acid sequence that acts as inhibitor of RNA or protein, a splice acceptor site (SA) upstream of the nucleic acid sequence that is a sequence acting as an RNA blocker or protein decoy, and a splice donor site (SD) downstream of the nucleic acid sequence that is a sequence acting as inhibitor of RNA or protein.

Yet another part of the disclosure relates to a DNA polynucleotide comprising a promoter, a nucleic acid sequence that acts as inhibitor of RNA or protein, a splice donor (SD) upstream of the nucleic acid sequence that is a sequence acting as an RNA blocker or protein decoy, and a splice acceptor site (SA) downstream of the nucleic acid sequence that is a sequence acting as inhibitor of RNA or protein.

The DNAs, vector or cassettes may comprise one or more introns.

In one part of the disclosure the DNAs, vector or cassettes comprises at least one intron.

An inverted repeat (or IR) is a sequence of nucleotides that is the reversed complement of another sequence further downstream, for example, 5'-GACTGC....GCAGTC---3'.

When no nucleotides intervene between the sequence and its downstream complement, it is called a palindrome.

Inverted repeats define the boundaries in transposons and retroviruses.

Inverted repeats also indicate regions capable of self-complementary base pairing (regions within a single sequence which can base pair with each other).

Thus, in one part of the disclosure the DNA polynucleotide of the present invention further comprises inverted repeats.

Another part of the disclosure relates to an RNA which transcribed from the DNA of the present invention.

Another part of the disclosure relates to inverted repeats positioned in the DNA in part or fully outside the RNA coding segment.

A further embodiment of the present invention relates to an RNA of the present invention which is capable of splicing.

In another part of the disclosure the DNA polynucleotide does not comprise a microRNA or does not comprise a sequence corresponding to the complete sequence of a microRNA.

The RNA or DNA polynucleotides disclosed in here are capable of expressing short RNA sequences of choice as circular RNAs.

In one embodiment of the present invention is the size of the circular RNAs are 25-200 nucleotides, such as 40-100 nucleotides, such as 50-70 nucleotides.

As mentioned above are these RNA sequences in one embodiment of the present invention microRNAs or anti-miRs.

In another embodiment of the invention are these RNA sequences the HIV Rev Response Element (RRE) or other viral RNAs.

In one embodiment of the present invention the circular RNA comprises (streptavidin) aptamer sequences.

In one embodiment of the present invention the circular RNA comprises motifs for RNA binding proteins.

In one embodiment of the present invention the RNA produced from DNAs, vector or cassettes comprises motifs facilitating RNA-RNA interaction.

### Splicing

Several methods of RNA splicing occur in nature; the type of splicing depends on the structure of the spliced sequence and the catalysts required for splicing to occur.

A splice donor site (SD, the 5' end of the sequence) and an acceptor site (SA, 3' end of the sequence) are usually required for splicing.

The splice donor site (SD) includes an almost invariant sequence GU at the 5' end of the polynucleotide, within a larger, less highly conserved region.

The splice acceptor site (SA) at the 3' end of the polynucleotide terminates the intronic polynucleotide with an almost invariant AG sequence.

Thus, in one embodiment of the present invention the RNA polynucleotide of the present invention comprises a splice acceptor site (SA) and a splice donor site (SD).

Another part of the disclosure relates to a single or double stranded DNA molecule of the disclosure that comprises a splice acceptor site (SA) and a splice donor site (SD).

Upstream (5'-ward) from the AG there is usually a region high in pyrimidines (C and U), or polypyrimidine tract.

Upstream from the polypyrimidine tract is the branch point, which usually includes an adenine nucleotide.

In further embodiment of the present invention the RNA polynucleotide comprises a region high in pyrimidines (C and U), or polypyrimidine tract.

The splicing of the two ends of the polynucleotide may also be done by self-splicing which occurs through the function of a ribozyme that performs the functions of the spliceosome by RNA alone.

Thus, in part of the disclosure the polynucleotide of the present invention comprises a ribozyme function that allows splicing of the two ends.

In another part of the disclosure the splicing is done by ribonucleases that cleave the RNA and ligases join the ends together.

In another part of the disclosure the splicing is done by an inverted group I intron.

In another embodiment of the present invention the polynucleotide of the present invention comprises a cis-acting regulatory site i.e. silencers and/or enhancers.

Splicing enhancers are sites to which splicing activator proteins bind, increasing the probability that a nearby site will be used as a splice junction.

These also may occur in the intron (intronic splicing enhancers, ISE) or exon (exonic splicing enhancers, ESE).

Most of the activator proteins that bind to ISEs and ESEs are members of the SR protein family. Such proteins contain RNA recognition motifs and arginine and serine-rich (RS) domains.

Splicing silencers are sites to which splicing repressor proteins bind, reducing the probability that a nearby site will be used as a splice junction.

These can be located in the intron itself (intronic splicing silencers, ISS) or in a neighboring exon (exonic splicing silencers, ESS). They vary in sequence, as well as in the types of proteins that bind to them.

The majority of splicing repressors are heterogeneous nuclear ribonucleoproteins (hnRNPs) such as hnRNPA1 and polypyrimidine tract binding protein (PTB).

Spliceosomal splicing and self-splicing involves a two-step biochemical process.

Both steps involve transesterification reactions that occur between RNA nucleotides.

Spliceosomal and self-splicing transesterification reactions occur via two sequential transesterification reactions.

First, the 2'OH of a specific branch-point nucleotide within the intron that is defined during spliceosome assembly performs a nucleophilic attack on the first nucleotide of the intron at the 5' splice site forming the lariat intermediate.

Second, the 3'OH of the released 5' exon then performs a nucleophilic attack at the last nucleotide of the intron at the 3' splice site thus joining the exons and releasing the intron lariat.

In one part of the disclosure the splicing of the two ends of the polynucleotide is done *in vitro.*

In another part of the disclosure the splicing of the two ends of the polynucleotide is done *in vivo* e.g. where the polynucleotide is expressed and subsequently made circular through the function of the host splice machinery.

In another part of the disclosure the splicing of the two ends of the polynucleotide is regulated by introducing splice-enhancer or splice-repressor sequences.

### Promoters

A promoter is a region of DNA that initiates transcription of a particular gene.

Promoters are usually located near the genes they transcribe, on the same strand and upstream (towards the 3' region of the anti-sense strand).

For the transcription to take place the RNA polymerase must attach to the DNA near a gene.

Promoters contain specific DNA sequences and response elements that provide a secure initial binding site for RNA polymerase and for transcription factors that recruit RNA polymerase.

These transcription factors have specific activator or repressor sequences of corresponding nucleotides that attach to specific promoters and regulate gene expressions.

The core promoter is the minimal portion of the promoter required to properly initiate transcription which includes a Transcription Start Site (TSS) and elements directly upstream.

These elements are a binding site for a RNA polymerase and general transcription factor binding sites, e.g. a TATA box.

Thus, in part of the disclosure the promoter is a core promoter.

A proximal promoter is the proximal sequence upstream of the gene that tends to contain primary regulatory elements.

These elements are usually approximately 250 bp upstream of the start site and they include specific transcription factor binding sites.

A distal promoter is the distal sequence upstream of the gene that may contain additional regulatory elements, often with a weaker influence than the proximal promoter.

These element are usually anything further upstream (but not an enhancer or other regulatory region whose influence is positional/orientation independent) and specific transcription factor binding sites.

Thus, in a further part of the disclosure the promoter comprises a proximal and/or a distal promoter.

In another part of the disclosure the promoter is a cell or tissue-specific promoter that allows for expression in a desired type of cell or tissue.

### Artificial nucleic acid sequences and modifications

The nucleic acid sequences may be produced synthetically or transcribed in vitro from an expression vector.

Such nucleic acid sequences may comprise, by direct incorporation or subsequent modification, alternative nucleobase units or modifications.

Thus, one embodiment of the present invention relates to the RNA polynucleotide of the present invention, wherein at least one of the nucleobase units of the polynucleotide is a Locked Nucleic Acid (LNA) nucleobase unit, a INA unit, a TINA unit, a 2'-OMe RNA unit and/or a 2'-fluoro DNA unit.

### Cells

The nucleic acids of the present invention may be comprised within a cell.

In one embodiment, the cell, or in another embodiment, cell systems of this invention comprise primary cultures or other.

"Primary cultures" refers, in one embodiment, to a culture of cells that is directly derived from cells or tissues from an individual, as well as cells derived by passage from these cells, or immortalized cells.

In one embodiment, "cell line" refers to a population of cells capable of continuous or prolonged growth and division *in vitro.*

The term "cell lines" also includes immortalized cells.

Often, cell lines are clonal populations derived from a single progenitor cell. Such cell lines are also termed "cell clones".

It is further known in the art that spontaneous or induced changes can occur in karyotype during storage or transfer of such clonal populations.

Therefore, cells derived from the cell clones referred to may not be precisely identical to the ancestral cells or cultures.

According to the present invention, such cell clones may be capable of supporting replication of a vector, virus, viral particle, etc., of this invention, without a significant decrease in their growth properties, and are to be considered as part of this invention.

Thus one embodiment of the present invention relates to a cell comprising the nucleic acid according to the present invention, the composition of the present invention.

It is to be understood that the nucleic acid of the present invention relates to the RNA polynucleotide of the invention.

### Pharmaceutical compositions

One embodiment of the present invention relates to a composition comprising a nucleic acid molecule according to the invention suspended in a suitable amount of a pharmaceutical acceptable diluent or excipient.

In another embodiment, this invention provides for compositions comprising an isolated nucleic acid ofthis invention..

In one embodiment, the term "composition" refers to any such composition suitable for administration to a subject, and such compositions may comprise a pharmaceutically acceptable carrier or diluent, for any of the indications or modes of administration as described. The active materials in the compositions of this invention can be administered by any appropriate route, for example, orally, parenterally, intravenously, intradermally, subcutaneously, or topically, in liquid or solid form.

Such composition is usually known as a pharmaceutical composition.

An aspect of the invention relates to a pharmaceutical composition comprising
- a single stranded circular RNA polynucleotide, which is at least partly complementary to a human or animal microRNA sequence, wherein the polynucleotide comprises a region of contiguous nucleobase sequence which is 100% complementary to the human or animal microRNA seed region; and
- at least one of a pharmaceutically acceptable diluent, carrier and adjuvant; for use as a medicament.

Thus, one embodiment of the present invention relates to a pharmaceutical composition comprising a polynucleotide of the present invention, a pharmaceutically acceptable diluent, carrier, or adjuvant, wherein the circular polynucleotide is at least partly complementary to a human microRNA sequence or comprises a guide sequence corresponding to the seed sequence of a human microRNA sequence.

It is to be understood that any applicable drug delivery system may be used with the compositions and/or agents/vectors/cells/nucleic acids of this invention, for administration to a subject, and is to be considered as part of this invention.

It is also to be understood that any of the methods of this invention, whereby a nucleic acid, vector or cell of this invention is used, may also employ a composition comprising the same as herein described, and is to be considered as part of this invention.

"Pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopoeia or other generally recognized pharmacopoeia for use in animals, and more particularly in humans.

The term "excipient" refers to a diluent, adjuvant, carrier, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

The term "adjuvant" refers to a compound or mixture that enhances the immune response to an antigen. An adjuvant can serve as a tissue depot that slowly releases the antigen and also as a lymphoid system activator that non-specifically enhances the immune response. Often, a primary challenge with an antigen alone, in the absence of an adjuvant, will fail to elicit a humoral or cellular immune response.

Adjuvants include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, keyhole limpet hemocyanins, dinitrophenol, and potentially useful human adjuvants such as BCG (bacille Calmette-Guerin) and Corynebacterium parvmm.

Preferably, the adjuvant is pharmaceutically acceptable.

Thus one embodiment of the present invention relates to a composition comprising a nucleic acid molecule according to the present invention suspended in a suitable amount of a pharmaceutical acceptable diluent or excipient.

### Methods of inhibiting microRNAs and treatment

The polynucleotides of the present invention can be used to regulate the function of microRNAs and thereby also the expression of specific genes.

Thus, one part of the disclosure relates to a method for reducing the effective amount of a microRNA or microRNA target in a cell or an organism, comprising administering a pharmaceutical composition of the present invention, to the cell or the organism.

One part of the disclosure relates to a method of the present invention, wherein the reduction of the effective amount of the microRNA or micro RNA target occurs via in vivo antagonism.

A further part of the disclosure relates to a method for de-repression of a target RNA of a microRNA in a cell or an organism, comprising administering a pharmaceutical composition of the present invention, to the cell or the organism.

Another aspect and an embodiment of the present invention relates to the use of a polynucleotide circular RNA of the present invention for the manufacture of a medicament for the treatment of a disease or medical disorder associated with the presence or over-expression of the microRNA.

Another part of the disclosure relates to a method for the treatment of a disease or medical disorder associated with the presence or over-expression of the microRNA, comprising the step of administering a pharmaceutical composition of the present invention to a person in need of treatment.

Another aspect and an embodiment of the present invention relates to the use of a polynucleotide circular RNA of the present invention for the manufacture of a medicament for the treatment of a disease or medical disorder associated with the presence or over-expression of the other RNA species or RNA-binding proteins.

In one embodiment of the present invention is the disease selected from the group consisting of cancer, a viral infection, obesity, diabetes, and a neurological disease.
In another embodiment of the present invention is the disease selected form the group consisting of Parkinson's disease, lymphoma, leukemia, CLL, colon cancer, breast cancer, heart disease, schizophrenia, HCV, herpesvirus and HIV.
It should be noted that embodiments and features described in the context of one of the aspects of the present invention also apply to the other aspects and embodiments of the invention.

In yet another instance, the disclosure can be used to cure or ameliorate diseases such as but not limited to those listed below miR2Disease, a manually curated database, aims at providing a comprehensive resource of miRNA deregulation in various human diseases. Each entry in the miR2Disease contains detailed information on a miRNA-disease relationship, including miRNA ID, disease name, a brief description of the miRNA-disease relationship, miRNA expression pattern in the disease state, detection method for miRNA expression, experimentally verified miRNA target gene(s), and literature reference. DOID: Disease Ontology ID. http://www.mir2disease.org/

| | |
|---|---|
| Abdominal Aortic Aneurysm | DOID:7693 |
| acute lymphoblastic leukemia (ALL) | DOID:9952 |
| acute myeloid leukemia (AML) | DOID:9119 |
| acute myocardial infarction | DOID:9408 |
| acute promyelocytic leukemia (APL) | DOID:9119 |
| adenoma | DOID:657 |
| adrenocortical carcinoma | DOID:3948 |
| alcoholic liver disease (ALD) | DOID:9454 |
| Alzheimer's disease | DOID:10652 |
| anaplastic thyroid carcinoma (ATC) | DOID:7012 |
| anxiety disorder | DOID:2030 |
| asthma | DOID:2841 |
| Astrocytoma | DOID:3069 |
| Atopic Dermatitis | DOID:3310 |
| autism spectrum disorder (ASD) | DOID:1208 |
| B-cell chronic lymphocytic leukemia | DOID:1040 |
| B-cell lymphoma | DOID:353 |
| Becker muscular dystrophy (BMD) | DOID: 14691 |
| bladder cancer | DOID:4007 |
| Brain Neoplasm | DOID:1319 |
| breast cancer | DOID:3459 |
| Burkitt lymphoma | DOID:8584 |
| cardiac hypertrophy | DOID: 11984 |
| cardiomyopathy | DOID:3978 |
| Cardiovascular Disease | DOID:2348 |
| Cerebellar neurodegeneration | DOID:2786 |
| cervical cancer | DOID:2893 |
| cholangiocarcinoma | DOID:4947 |
| cholesteatoma | DOID:869 |
| choriocarcinoma | DOID:3594 |
| chronic lymphocytic leukemia (CLL) | DOID:1040 |
| chronic myeloid leukemia (CML) | DOID:8552 |
| chronic pancreatitis | DOID:13315 |
| Colon Carcinoma | DOID:1520 |
| colorectal cancer | DOID:1985 |
| congenital heart disease | DOID:114 |
| coronary artery disease | DOID:3363 |
| Cowden Syndrome | DOID:3471 |
| dermatomyositis (DM) | DOID:10223 |
| Diabetic Nephropathy | DOID:2370 |
| diarrhea predominant irritable bowel syndrome (IBS-D) | DOID:9778 |
| diffuse large B-cell lymphoma (DLBCL) | DOID:353 |
| Dilated Cardiomyopathy | DOID: 12930 |
| Down syndrome (DS) | DOID:14250 |
| Duchenne muscular dystrophy (DMD) | DOID: 14691 |
| endometrial cancer | DOID:2871 |
| Endometrial EndometrioidAdenocarcinoma | DOID:5827 |
| Endometriosis | DOID:289 |
| epithelial ovarian cancer (EOC) | DOID:4001 |
| esophageal cancer | DOID:1107 |
| Esophagus squamous cell carcinoma | DOID:3748 |
| essential thrombocythemia (ET) | DOID:2224 |
| facioscapulohumeral muscular dystrophy (FSHD) | DOID:11727 |
| follicular lymphoma (FL) | DOID:8524 |
| follicular thyroid carcinoma (FTC) | DOID:3962 |
| frontotemporal dementia | DOID:10216 |
| gastric cancer (stomach cancer) | DOID:5517 |
| glioblastoma | DOID:3068 |
| glioblastoma multiforme (GBM) | DOID:3073 |
| glioma | DOID:2627 |
| glomerular disease | DOID:2976 |
| Glomerulosclerosis | DOID:3945 |
| hamartoma | DOID:3462 |
| HBV-related cirrhosis | DOID:5082 |
| HCV infection | DOID:7324 |
| head and neck cancer | DOID:11934 |
| head and neck squamous cell carcinoma (HNSCC) | DOID:5520 |
| hearing loss | DOID:10004 |
| Heart Disease | DOID:114 |
| heart failure | DOID:6000 |
| Hepatitis B | DOID:2043 |
| Hepatitis C | DOID:1883 |
| hepatocellular carcinoma (HCC) | DOID:684 |
| Hilar Cholangiocarcinoma | DOID:4927 |
| Hodgkin's lymphoma | DOID:8567 |
| homozygous sickle cell disease (HbSS) | DOID:10923 |
| Huntington's disease (HD) | DOID:12858 |
| Hypertension | DOID:10763 |
| Hypopharyngeal cancer | DOID:8532 |
| Inclusion body myositis (IBM) | DOID:3429 |
| Insulinoma | DOID:3903 |
| Intrahepatic cholangiocarcinoma (ICC) | DOID:4928 |
| kidney cancer | DOID:4451 |
| Kidney Disease | DOID:557 |
| laryngeal carcinoma | DOID:6624 |
| Late Insomnia (sleep disease DOID:535) | DOID:535 |
| Leiomyoma of lung | DOID:5136 |
| Leukemia | DOID:1240 |
| limb-girdle muscular dystrophies types 2A (LGMD2A) | DOID:11724 |
| lipoma | DOID:9291 |
| Lung Adenocarcinoma | DOID:3910 |
| lung cancer | DOID:3905 |
| lymphoproliferative disease | DOID:619 |
| malignant lymphoma | DOID:353 |
| malignant melanoma | DOID:1909 |
| Malignant mesothelioma (MM) | DOID:1790 |
| mantle cell lymphoma (MCL) | DOID:353 |
| medulloblastoma | DOID:3858 |
| melanoma | DOID:1909 |
| meningioma | DOID:3565 |
| metabolic disease | DOID:14667 |
| miyoshi myopathy (MM) | DOID:423 |
| multiple myeloma (MM) | DOID:9538 |
| multiple sclerosis | DOID:2377 |
| MYC-rearranged lymphoma | DOID:353 |
| Myelodysplastic Syndrome | DOID:4972 |
| myeloproliferative disorder | DOID:2356 |
| myocardial infarction | DOID:5844 |
| myocardial injury | DOID:12931 |
| myoma | DOID:2691 |
| nasopharyngeal carcinoma (NPC) | DOID:9261 |
| nemaline myopathy (NM) | DOID:3191 |
| Nephritis | DOID:10952 |
| neuroblastoma (NB) | DOID:769 |
| neutrophilia is_obsolete | |
| Niemann-Pick type C (NPC) disease | DOID:14770 |
| non-alcoholic fatty liver disease (NAFLD) | DOID:46 |
| non-small cell lung cancer (NSCLC) | DOID:3908 |
| Obesity | DOID:9970 |
| Oesophageal adenocarcinoma | DOID:4914 |
| Oral Carcinoma | DOID:8894 |
| osteosarcoma | DOID:3347 |
| ovarian cancer (OC) | DOID:2144 |
| pancreatic cancer | DOID:4905 |
| pancreatic ductal adenocarcinoma (PDAC) | DOID:3498 |
| Pancreatic Neoplasia | DOID:3588 |
| Panic Disease | DOID:594 |
| papillary thyroid carcinoma (PTC) | DOID:3969 |
| Parkinson's disease | DOID:14330 |
| PFV-1 infection | DOID:934 |
| Pharyngeal Disease | DOID:939 |
| pituitary adenoma | DOID:3829 |
| Polycystic Kidney Disease | DOID:5898 |
| Polycystic liver disease | DOID: 1173 |
| polycythemia vera (PV) | is_obsolete |
| polymyositis (PM) | DOID:10222 |
| primary biliary cirrhosis (PBC) | DOID:12236 |
| primary myelofibrosis | DOID:5737 |
| prion disease | DOID:649 |
| prostate cancer | DOID:10286 |
| Psoriasic Arthritis | DOID:9008 |
| psoriasis | DOID:9088 |
| pulmonary hypertension | DOID:6432 |
| recurrent ovarian cancer | DOID:6708 |
| Renal Cell Carcinoma | DOID:4450 |
| renal clear cell carcinoma | DOID:4467 |
| retinitis pigmentosa (RP) | DOID:10584 |
| retinoblastoma | DOID:768 |
| rhabdomyosarcoma | DOID:3247 |
| Rheumatic Heart Disease | DOID:9814 and Atrial Fibrillation DOID:9814 |
| Rheumatoid Arthritis | DOID:7148 |
| sarcoma | DOID:1115 |
| schizophrenia | DOID:5419 |
| sepsis | DOID:2292 |
| serous ovarian cancer | DOID:5745 |
| Sezary syndrome | DOID:8541 |
| skin disease | DOID:37 |
| Small Cell Lung Cancer | DOID:3907 |
| Spinocerebellar ataxia | DOID:1441 |
| squamous carcinoma | DOID:1749 |
| T-cell leukemia | DOID:715 |
| teratocarcinoma | DOID:3305 |
| testicular germ cell tumor | DOID:5556 |
| thalassemia | DOID:10241 |
| thyroid cancer | DOID:3963 |
| tongue squamous cell carcinoma | DOID:10944 |
| tourette's syndrome | DOID:11119 |
| type 2 diabetes | DOID:9351 |
| ulcerative colitis (UC) | DOID:8577 |
| uterine leiomyoma (ULM) | DOID:13223 |
| uveal melanoma | DOID:1909 |
| vascular disease | DOID:178 |
| vesicular stomatitis | DOID:10881 |
| Waldenstrom Macroglobulinemia(WM) | DOID:9080 |

Other diseases are metabolic diseases in general, cancer diseases and regulation of insulin and blood sugar.

### Especially interesting disease target comprise

Inhibition of onco-miRs, such as miR-15, miR16, miR-17, miR-18, miR-19, miR-21, miR-155 or miR-191, as a potential cancer treatment.

Inhibition of miR-9 and miR-128 known to be upregulated in Alzheimers disease, as a potential treatment.

Inhibition of miR-29 involved in insulin resistance as a potential treatment of type 2 diabetes.

Inhibition of miR-208a known to induce arrhythmia as a potential treatment of cardiac hypertrophy.

Inhibition of virally encoded miRNAs, such hcmv-miR-UL112, to potentially treat viral infection.

Treatment of hepatits C by inhibition of miR-122 known to be essential for Hepatitis C infection.

### Other Circular RNA as RNA/motif blocker are:

Blocking specific target sites for onco-miRs, such as miR-17:E2F1, miR-19:PTEN, miR-21:PDCD4, or miR-155:SOCS1, as a potential cancer treatment.

Blocking the Open-Reading-Frame (ORF) of oncogenes, such as Myc or Ras. This approach also applies to the potential treatment of other diseases with gain-of-function gene expression.

### Circular RNA as protein decoys:

Sequestering and inhibition of RNA-binding proteins involved in cancer progression, such as Sam68 or SF2/ASF, as a potential cancer treatment.

Sequestering and inhibition of RNA-binding proteins involved in neurological degeneration, such as Nova or Hu, as a potential treatment.

Sequestering and inhibition of virally encoded RNA-binding proteins, such as Rev, as a potential anti-viral treatment.

### Additional aspects and embodiments

Additional aspects and embodiments of the invention are provided below. Part of the disclosure concerns a circular RNA polynucleotide which is at least partly complementary to a human or animal microRNA sequence.

The expression "circular RNA polynucleotide" refers to RNA polynucleotide consisting of a circular structure as well as a structure comprising a circular structure, such as a circular lariat.

According to an embodiment, the invention concerns circular RNA polynucleotide having at least one mismatch to said microRNA. A preferred target sequence in the circular RNA has at least one mismatch to the microRNA that makes it resistant to endonucleolytic cleavage. Accordingly, preferably said target sequence is not fully complementary to a non-native or wild-type sequence, but comprises a number of mutations selected among 1, 2, 3, 4, 5, 6, 7, 8, 9, or 10 mutations, insertions and/or deletions.
According to an embodiment, the invention concerns circular RNA polynucleotide, wherein said circular RNA polynucleotide is resistant to endonucleolytic cleavage caused by said microRNA. A preferred sequence is resistant to endonucleolytic cleavage by the microRNA it regulate as interpreted from a simple assay where both the circular RNA and microRNA is expressed in the same cell. The assay, demonstrated below microRNA-7, can be conducted by a skilled scientist for any other part of circular RNA and microRNA. As an example of a micro-RNA, that does not contain any mismatches in the central part and therefore is able to cleave the circular RNA, microRNA-671 included.
A circular RNA polynucleotide according to the invention preferably allows inhibition of interactions between microRNA and their RNA target. A circular RNA polynucleotide according to the invention is preferably resistant to degradation by exonucleases or endonucleases.

According to an embodiment, the invention concerns circular RNA polynucleotide, wherein said circular RNA polynucleotide contains more than one binding site for microRNA. The microRNA may be any microRNA.

According to an embodiment, the invention concerns circular RNA polynucleotide, wherein said circular RNA polynucleotide contains at least two, such as three, at least four, such as five, at least six, such as seven, at least eight, such as nine binding sites for microRNA.

According to an embodiment, the invention concerns circular RNA polynucleotide, wherein said circular RNA polynucleotide contains at least ten, such as fifteen, at least twenty, such as twenty five, at least thirty, such as thirty five binding sites for microRNA.

According to an embodiment, the invention concerns circular RNA polynucleotide, for use as a medicament.

According to an embodiment, the invention concerns circular RNA polynucleotide, for use in vivo or in vitro in laboratory work.

An aspect of the invention relates to in vitro use of a single stranded circular RNA polynucleotide according to the invention for blocking and inhibition of RNA.

According to an embodiment, the invention concerns circular RNA polynucleotide, for use in vivo or in vitro in laboratory work such as but not limited to inhibition of miRNAs, such as but not limited to miR-15, miR-16, miR-17, in cell cultures or organisms to study the relevance/loss-of function phenotype of the specific miRNA. Such uses comprise, but are not limited to:
Inhibition of miRNA target sites, such as but not limited to the miR-17 target site in E2F1 mRNA or the miR-19 target site in PTEN mRNA, in cell cultures or organisms to study the relevance of the specific target site.
Inhibition of protein binding sites, such as but not limited to the HuR binding site in the TNF-a mRNA or the Pumilio (Pum1 and Pum2) binding site in the E2F3 mRNA, in cell cultures or organisms to study the relevance of the specific protein-mRNA interaction.
Inhibition of protein binding, protein activity or protein function, such as but not limited to the uPAR binding of uPA or the activity of HCV NS5B RNA replicase, in cell cultures or organisms to study the effect of protein loss-of-function.
Inhibition of translation of mRNAs, such as but not limited to RAS and MYC, in cell cultures or organisms to study the effect of the specific translation and to deplete the specific protein
Inhibition of the secondary structure of RNAs, such as but not limited to tRNA, snoRNA, pre-miRNA, in cell cultures or organisms to study the relevance of the secondary structure.
Inhibition of DNA motifs, such as but not limited to TATA-boxes, GC-islands and transcription factor binding sites, in cell cultures or organisms to study the effect of the specific DNA motifs.
Coupling of circular RNA with fatty acid moieties to increate transfection efficiency and to tether fatty acid binding proteins in cell cultures and organisms.
Coupling of circular RNA with carbohydrate moieties, oligo- and polysaccharides, such as but not limited to galactose, glycogen, or poly-L-Lysine to tether carbohydrate binding proteins in cell cultures and organisms.
Coupling of circular RNA with protein/peptide, moieties, such as NPY (NeuroPeptide Y), Erythropoietin, or Follistatin to tether protein-binding proteins in cell cultures and organisms.
Coupling of circular RNA with fluorescent dyes, such as but not limited to cy3 and cy5, to track the presence of circular RNA in cell cultures and organisms. Coupling of circular RNA with other chemical structures, such as but not limited to NADH, aspirin, or aspartame to potentially study the interactome of the coupled chemical structure.

The circular RNA can contain natural and/or unnatural nucleotides, such as but not limited to -2'OMe, -2'F, or LNA, to modify the chemical properties of the circular RNA.

### Regulation of circular RNA expression:

Circular RNA can be introduced into cells, tissues or whole animals using either in vitro synthesized circular RNA or circular RNA expressing cassettes, such as but not limited to plasmids or retroviral vectors using delivery methods such as but not limited to transfection or electroporation.
Control of circular RNA expression in cells, tissue and whole animal by using inducible promoters, such as but not limited to Tet-on or LTR-Tat, in cells, tissue and whole animal.
Expression of RNA circles from tissue specific promoters to allow tissue specific inhibition of RNA and protein in cells, tissue and whole animal.
Control of circular RNA expression using recombination-based systems, such as but not limited to Cre-Lox or Flp-In in cells, tissue and whole animal.
Control of circular RNA expression using splicing regulators, such as but not limited to hnRNP-A1, PTB, or spliceostatin A.
Control of circular RNA expression using RNA binding proteins, such as HuR or RNase H.
Control of circular RNA expression using RNA depletion methods, such as but not limited to siRNAs, miRNAs and Gapmers.
Control of circular RNA expression using antisense technology, such as but not limited to antisense Morpholino.
According to an embodiment, the invention concerns circular RNA polynucleotide, which comprises a splice acceptor site (SA) and a splice donor site (SD). The invention concerns circular RNA polynucleotide, which is at least partly complementary to a human or animal microRNA sequence, for use as a medicament, wherein the polynucleotide comprises a region of contiguous nucleobase sequence which is 100% complementary to the human or animal microRNA seed region. Also described is a circular RNA polynucleotide, wherein the polynucleotide comprises a region of contiguous nucleobase sequence which is partially complementary to the human or animal microRNA seed region. The invention concerns circular RNA polynucleotide comprising an antisense sequence that comprises a guide sequence corresponding to the seed sequence of microRNA.
Part of the disclosure concerns DNA comprising:
a) a promoter,
b) a nucleic acid sequence that is completely or partially complementary to a human or animal microRNA sequence,
c) a splice acceptor site (SA) upstream or downstream of said nucleic acid sequence of b), and
d) a splice donor site (SD) downstream or upstream of said nucleic acid sequence of b).
Part of the disclosure concerns DNA comprising:
a) a promoter,
b) a nucleic acid sequence that is complementary to the seed sequence of a human or animal microRNA sequence,
c) a splice acceptor site (SA) upstream or downstream of said nucleic acid sequence of b), and
d) a splice donor site (SD) downstream or upstream of said nucleic acid sequence of b).
Part of the disclosure concerns DNA comprising:
a) a promoter,
b) two or more nucleic acid sequences that is selected from the group consisting of a nucleic acid sequence that is completely or partially complementary to a human or animal microRNA sequence and a nucleic acid sequence that is complementary to the seed sequence of a human or animal microRNA sequence,
c) a splice acceptor site (SA) upstream or downstream of said two or more nucleic acid sequences of b), and
d) a splice donor site (SD) downstream or upstream of said two or more nucleic acid sequences of b).
Part of the disclosure concerns DNA, which further comprises inverted repeats.

Part of the disclosure concerns DNA, wherein said nucleic acid sequence(s) is not microRNA or does not comprise a sequence corresponding to the complete sequence of microRNA.

Part of the disclosure concerns RNA transcribed from the DNA according to the invention.
According to an embodiment, the invention concerns RNA polynucleotide, wherein at least one of the nucleobase units of the polynucleotide is selected among a Locked Nucleic Acid (LNA) unit, a Unlocked Nucleic Acid (UNA) unit, a TINA unit, a 2'-OMe RNA unit and a 2'-fluoro RNA unit.
According to an aspect and an embodiment, the present invention concerns a cell comprising the circular RNA polynucleotide according to the invention.
According to an aspect and an embodiment, the present invention concerns a pharmaceutical composition comprising a circular polynucleotide according to the invention, and at least one of a pharmaceutically acceptable diluent, carrier and adjuvant, wherein the circular polynucleotide is at least partly complementary to a human or animal microRNA sequence or comprises a guide sequence complementary to the seed sequence of a human or animal microRNA sequence. The disclosure also concerns a method for inhibiting, inactivating or reducing the effective amount of microRNA in a cell or an organism, comprising administering to the cell or the organism an entity selected among the group consisting of a circular RNA and a pharmaceutical composition according to the invention.

According to one instance, the disclosure concerns the method wherein the reduction of the effective amount of the microRNA occurs via *in vivo* antagonism.
The disclosure also concerns a method for de-repression of a target mRNA of a microRNA in a cell or an organism, comprising administering to the cell or the organism an entity selected among the group consisting of a circular RNA polynucleotide, DNA, RNA, a cell and a pharmaceutical composition according to the invention.

The disclosure also concerns a use of a circular RNA polynucleotide according to the invention for the manufacture of a medicament for the treatment, amelioration or prevention of a disease or medical disorder associated with the presence or over-expression of microRNA.

A circular RNA polynucleotide according to the invention for the treatment, amelioration or prevention of a disease or medical disorder associated with the presence or over-expression of microRNA.

The disclosure also concerns a method for the treatment, amelioration or prevention of a disease or medical disorder associated with the presence or over-expression of microRNA, comprising the step of administering an entity selected among the group consisting of a circular RNA polynucleotide, and a pharmaceutical composition according to the invention, to a person or animal in need of treatment.

The disclosure also concerns the use, circular RNA polynucleotide, or method according to the invention, wherein the disease or medical disorder is selected from the group consisting of Parkinson's disease, cancer, a viral infection, Hepatitis C, diabetes, epilepsy, a neurological disease, insulin deficiency and blood sugar, and the following diseases and medical disorders: Abdominal Aortic Aneurysm, acute lymphoblastic leukemia (ALL), acute myeloid leukemia (AML), acute myocardial infarction, acute promyelocytic leukemia (APL), adenoma, adrenocortical carcinoma, alcoholic liver disease (ALD), Alzheimer's disease, anaplastic thyroid carcinoma (ATC), anxiety disorder, asthma, Astrocytoma, Atopic Dermatitis, autism spectrum disorder (ASD), B-cell chronic lymphocytic leukemia, B-cell lymphoma, Becker muscular dystrophy (BMD), bladder cancer, Brain Neoplasm, breast cancer, Burkitt lymphoma, cardiac hypertrophy, cardiomyopathy, Cardiovascular Disease, Cerebellar neurodegeneration, cervical cancer, cholangiocarcinoma, cholesteatoma, choriocarcinoma, chronic lymphocytic leukemia (CLL), chronic myeloid leukemia (CML), chronic pancreatitis, Colon Carcinoma, colorectal cancer, congenital heart disease, coronary artery disease, Cowden Syndrome, dermatomyositis (DM), Diabetic Nephropathy, diarrhea predominant irritable bowel syndrome (IBS-D), diffuse large B-cell lymphoma (DLBCL), Dilated Cardiomyopathy, Down syndrome (DS), Duchenne muscular dystrophy (DMD), endometrial cancer, Endometrial Endometrioid Adenocarcinoma, Endometriosis, epithelial ovarian cancer (EOC), esophageal cancer, Esophagus squamous cell carcinoma, essential thrombocythemia (ET), facioscapulohumeral muscular dystrophy (FSHD), follicular lymphoma (FL), follicular thyroid carcinoma (FTC), frontotemporal dementia, gastric cancer (stomach cancer), glioblastoma, glioblastoma multiforme (GBM), glioma, glomerular disease, Glomerulosclerosis, hamartoma, HBV-related cirrhosis, HCV infection, head and neck cancer, head and neck squamous cell carcinoma (HNSCC), hearing loss, Heart Disease, heart failure, Hepatitis B, Hepatitis C, hepatocellular carcinoma (HCC), Hilar Cholangiocarcinoma, Hodgkin's lymphoma, homozygous sickle cell disease (HbSS), Huntington's disease (HD), Hypertension, Hypopharyngeal cancer, Inclusion body myositis (IBM), Insulinoma, Intrahepatic cholangiocarcinoma (ICC), kidney cancer, Kidney Disease, laryngeal carcinoma, Late Insomnia (sleep disease), Leiomyoma of lung, Leukemia, limb-girdle muscular dystrophies types 2A (LGMD2A), lipoma, Lung Adenocarcinoma, lung cancer, lymphoproliferative disease, malignant lymphoma, malignant melanoma, Malignant mesothelioma (MM), mantle cell lymphoma (MCL), medulloblastoma, melanoma, meningioma, metabolic disease, miyoshi myopathy (MM), multiple myeloma (MM), multiple sclerosis, MYC-rearranged lymphoma, Myelodysplastic Syndrome, myeloproliferative disorder, myocardial infarction, myocardial injury, myoma, nasopharyngeal carcinoma (NPC), nemaline myopathy (NM), Nephritis, neuroblastoma (NB), neutrophilia, Niemann-Pick type C (NPC) disease, non-alcoholic fatty liver disease (NAFLD), non-small cell lung cancer (NSCLC), Obesity, Oesophageal adenocarcinoma, Oral Carcinoma, osteosarcoma, ovarian cancer (OC), pancreatic cancer, pancreatic ductal adenocarcinoma (PDAC), Pancreatic Neoplasia, Panic Disease, papillary thyroid carcinoma (PTC), Parkinson's disease, PFV-1 infection, Pharyngeal Disease, pituitary adenoma, Polycystic Kidney Disease, Polycystic liver disease, polycythemia vera (PV), polymyositis (PM), primary biliary cirrhosis (PBC), primary myelofibrosis, prion disease, prostate cancer, Psoriasic Arthritis, psoriasis, pulmonary hypertension, recurrent ovarian cancer, Renal Cell Carcinoma, renal clear cell carcinoma, retinitis pigmentosa (RP), retinoblastoma, rhabdomyosarcoma, Rheumatic Heart Disease and Atrial Fibrillation, Rheumatoid Arthritis, sarcoma, schizophrenia, sepsis, serous ovarian cancer, Sezary syndrome, skin disease, Small Cell Lung Cancer, Spinocerebellar ataxia, squamous carcinoma, T-cell leukemia, teratocarcinoma, testicular germ cell tumor, thalassemia, thyroid cancer, tongue squamous cell carcinoma, tourette's syndrome, type 2 diabetes, ulcerative colitis (UC), uterine leiomyoma (ULM), uveal melanoma, vascular disease,vesicular stomatitis, and Waldenstrom Macroglobulinemia (WM).
The disclosure also concerns use of a polynucleotide according to the invention for recruitment and inhibition of RNA-binding proteins. This is also referred to as protein decoy.

According to an aspect and an embodiment, the present invention concerns the in vitro use of a polynucleotide according to the invention for blocking and inhibition of RNA. According to an embodiment, the invention concerns the in vitro use, wherein said RNA is selected among miRNA target sites, open-reading-frames and non-coding RNA.
The disclosure also concerns use of a polynucleotide according to the invention for assembly of proteins into new functional entities.

Part of the disclosure concerns a method for expression of a polynucleotide according to the invention in a target cell using a viral vector.
Part of the disclosure concerns a method for expression of a polynucleotide according to the invention, wherein said viral vector is selected among an adenoviral, retroviral, baculoviral sindbis derived vector, polio virus derived vector and lentiviral vector.
Part of the disclosure concerns a method for expression of a polynucleotide according to the invention in a target cell using DNA.
Part of the disclosure concerns a method for expression of a polynucleotide according to the invention, wherein said DNA is selected among plasmids or linear DNA vectors.

According to one instance, the disclosure concerns a method for delivery of a polynucleotide according to the invention by a method selected among chemical-based transfection, non-chemical methods, particle-based methods, nucleofection-based methods, heat-shock-based methods, homologous DNA recombination, CRISPR system, flip-in system, and Cre-lox system.
The delivery may *inter alia* be performed by chemical-based transfection, such as but not limited to selected among the calcium phosphate method, liposome-based methods, polymers-based transfection or dendrimers-based transfection; Non-chemical methods, such as but not limited to electroporation, optical transfection, protoplast fusion, or hydrodynamic delivery; Particle-based methods, such as but not limited to magnetofection, impalefection or particle bombardment; Other methods, such as but not limited to nucleofection-based methods or heat-shock-based methods; Stable integration of circular RNA expression vector into host chromosome by, but not limited to, homologous DNA recombination, CRISPR system, flip-in system, and Cre-lox system.
Part of the disclosure concerns a use of circular RNA as a microRNA (miRNA) inhibitor.
The invention will now be described in further details in the following non-limiting examples.

### Examples

### Example 1 - Natural RNA circles function as efficient miRNA sponges

microRNAs (miRNAs) are important regulators of gene expression acting by direct base pairing to 3'UTR target sites in mRNAs. Recently, miRNA activity has been shown to be affected by the presence of miRNA sponge transcripts, the so-called competing endogenous RNA (ceRNA) in humans and target mimicry expression in plants.

The present inventors have previously identified a highly expressed circular RNA (cRNA) in human and mouse brain. Here the present inventors show that the cRNA acts as a miR-7 sponge and the present inventors term this circular transcript CiRS-7 (Circular RNA Sponge for miR-7). CiRS-7 harbours more than 70 selectively conserved putative miRNA target sites and it is highly and widely associated with Ago-proteins in a miR-7 dependent manner.

While the circular RNA is completely resistant towards miRNA-mediated target destabilization, it strongly suppresses miR-7 activity resulting in elevated levels of miR-7 targets.

In the mouse brain, we observe overlapping neuronal expression patterns of CiRS-7 and miR-7 in the neocortex and hippocampus, and in thalamus and substantia nigra suggesting a high degree of endogenous interaction.

The present inventors further show that the circular testis specific RNA, SRY, serves as a miR-138 sponge, suggesting that miRNA sponge effects achieved by circular RNA formation is a general phenomenon.

Thus, this serves as the first functional study of a naturally expressed circular RNA.

### Introduction

miRNAs belong to an established class of small non-coding RNA species primarily involved in the post-transcriptional regulation of gene expression. The mature miRNA is incorporated into the RNA induced silencing complex, RISC, and targets specific mRNAs harbouring seed-match target sites in the 3' UTR.

Currently, the prevalent model suggests that RISC recruits via GW182 a deadenylase complex that in turn facilitates polyA-tail removal thereby destabilizing the targeted mRNA.

While several studies have focused on the regulation of miRNAs, predominantly on the identification of factors involved in the biogenesis of subsets of miRNAs, interesting 'post-biogenesis' inhibition of miRNA activity has recently been observed.

Conceptual studies overexpressing miRNA target site concatamers, so-called miRNA sponges, were initially performed and showed to result in loss of miRNA function accompanied with elevated levels of endogenous targets.

Subsequently, an endogenously expressed linear non-coding RNA (ncRNA) was identified as a miRNA sponge, which is able to sequester miRNA-399 using a near perfect, non-cleavable target site.

This phenomenon was coined miRNA mimicry. Very recently, linear competing endogenous RNA (ceRNA) was identified and characterized in mammalian systems emphasizing the existence of pseudogenes and ncRNA expressed to tether and inhibit specific miRNAs.

Collectively, these data suggest that miRNA sponges are widespread regulators of miRNA activity in higher eukaryotes. However, commonly, miRNA sponges or miRNA competing transcripts so far identified all share non-dramatic expression levels and a limited number of miRNA target sites, and it has been questioned whether their expression levels are sufficient for inflicting effective inhibition.

The present inventors recently identified a circular RNA (cRNA) with high endogenous expression levels in human and mouse brain and demonstrated that the cRNA can become endonucleolytically cleaved by miR-671 in an Ago2 dependent manner, but the function was unknown.

### Results and discussion

Here the present inventors demonstrate that the cRNA functions as a post-transcriptional regulator of a miRNA implicated in several human disease conditions. Searching for miRNA target sites within the circular RNA, the present inventors identified 73 conventional seed-targets for miR-7.

Plotting the target site position and nucleotide conservation, we observed that almost all identified target sites were selectively and highly conserved in mammals (Fig. 1a).

In addition, analysis of online available Ago2 HITS-CLIP conducted in mouse brain revealed a high degree of Ago2-occupancy on the mouse variant of the circular RNA with sequence reads dispersed throughout the cRNA with highest reads density covering the region with highest 8-mer target sites abundance (Fig. 1b).

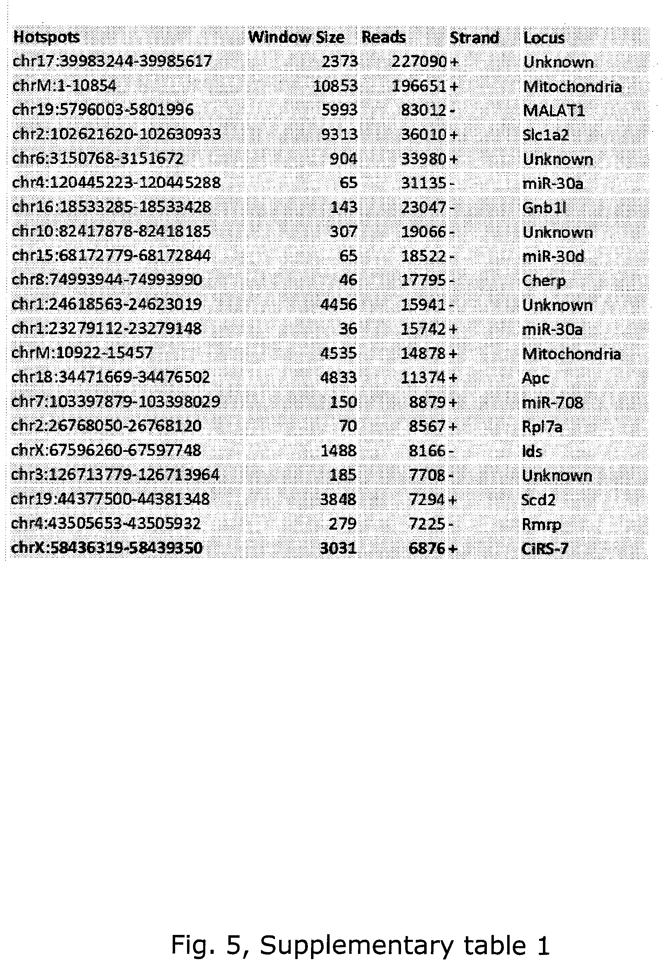

A hotspot analysis revealed that the cRNA was one of the most abundant loci in the dataset (Figure 5, supplementary Table S1). This conforms to our previous observation showing a high expression of cRNA in brain and other studies profiling brain-specific expression of miR-7.

To study the impact of cRNA expression on miR-7 activity, the present inventors established a vector-based system expressing the circular RNA. The biogenesis of circular RNA is currently unknown, however previous studies have shown that exons flanked by inverted repeats induce circular RNA formation in vitro and is suggested to be essential for circular SRY RNA production.

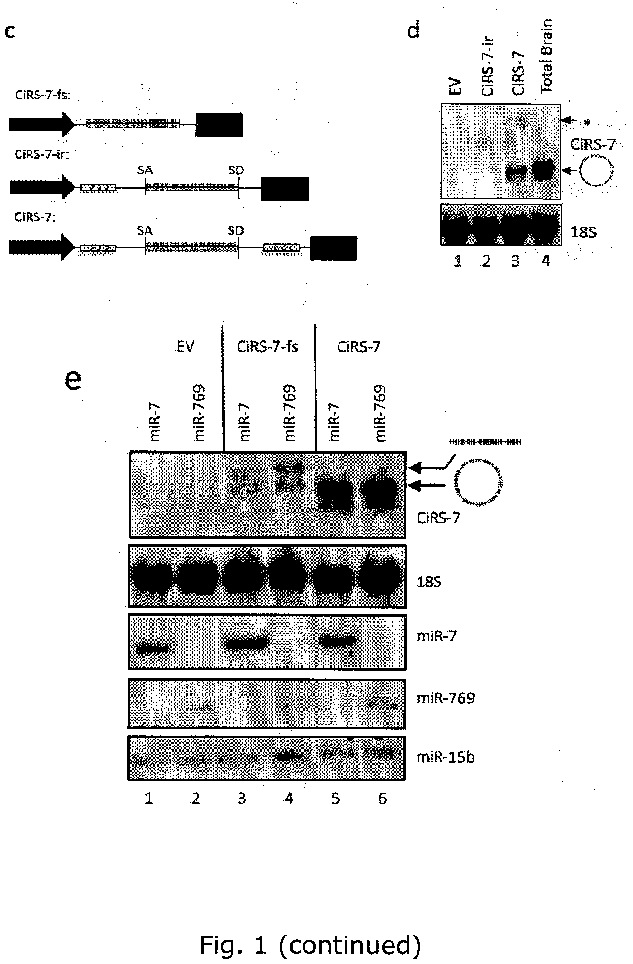

Thus, the present inventors inserted the CiRS-7 exon along with the endogenous flanking sequence (approximately 1000 nt upstream and 200 nt downstream) into pcDNA3 (pcDNA3-CiRS-7-ir, Fig. 1c).

Subsequently, the present inventors copied part of the upstream flanking sequence and inserted it in an inverted orientation downstream (pcDNA3-CiRS-7, Fig. 1c).

Transiently expressing of pcDNA3-CiRS-7, but not the construct lacking the downstream inverted sequence (pcDNA3-CiRS-7-is), resulted in production of cRNA species indistinguishable from the endogenously expressed cRNA by northern analysis (Fig. 1d) and RT-PCR sequencing (data not shown).

In addition, the present inventors constructed a linear and polyadenylated miR-7 sponge containing all miR-7 sponge sites but excluding the splice sites and flanking sequences (CiRS-7-fs, Fig. 1c).

By co-expressing the circular RNA- or the linear RNA-producing vectors with miR-7 or miR-769 expression vectors, the present inventors observed unaffected levels of CiRS-7, whereas the linear counterpart displayed miR-7 sensitivity presumably due to miRNA mediated deadenylation and subsequent degradation (Fig. 1e).

This suggests that CiRS-7 is resistant towards the conventional miRNA destabilization of mRNA and therefore not prone to miR-7 dependent regulation.

To determine whether a CiRS-7 serves as a target for miR-7, the present inventors conducted Ago2-IP in HEK293 cells transiently expressing miR-7 or miR-769 (control) together with myc-tagged Ago2. By qRT-PCR, this showed a high enrichment of endogenous CiRS-7 specifically in miR-7 transfected cells, suggesting that miR-7 indeed is facilitating Ago-association with the CiRS-7 (Fig. 2a).

Furthermore, the present inventors devised a T7 transcript containing all putative miR-7 target sites in conjunction with or without a reported streptavidin-aptamer motif 20.

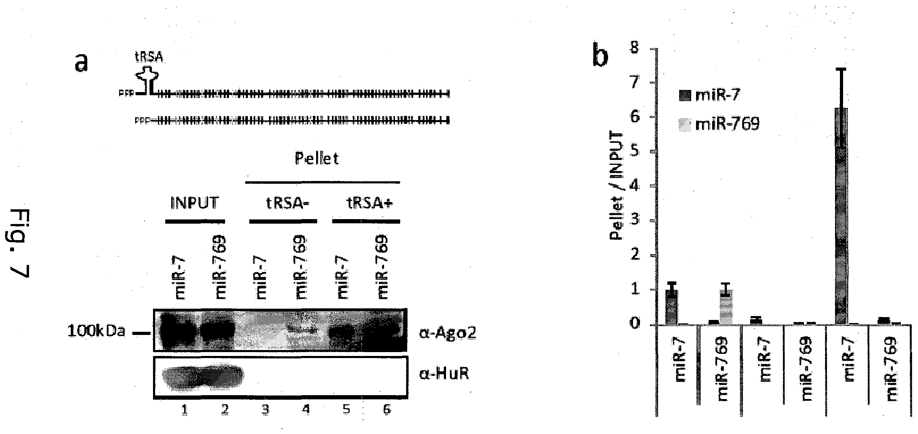

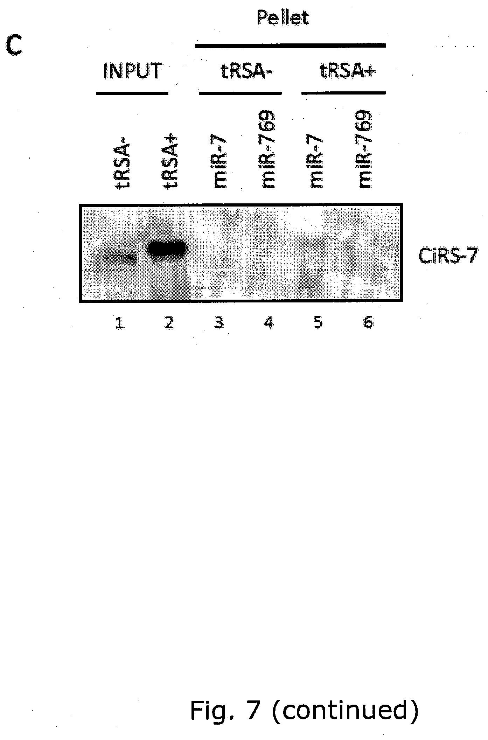

Incubating the T7 transcripts with lysate from HEK293 cells expressing either miR-7 or miR-769 as control, Ago2 and miR-7 were extracted efficiently only from miR-7 containing cell lysate and in an aptamer specific fashion (Figure 7, supplementary Fig. 1a-c).

Additionally, using a biotin-coupled miR-7 mimic, the present inventors observed a significant enrichment of CiRS-7 in the miR-7 captured fraction compared to when using biotinylated miR-138 (Fig. 2b).

Thus, the present inventors conclude that the widespread Ago-occupancy is caused by miR-7-directed association of Ago proteins to the prevalent and conserved miR-7 target sites in the cRNA.

To evaluate the subcellular localization of miR-7 and CiRS-7, the present inventors performed RNA-FISH on HEK293 cells (Fig. 2c-d) and HeLa cells (Figure 8, supplementary Fig. 2b-c) co-transfected with CiRS-7 and miR-7.

This showed a high degree of co-localization between CiRS-7 and miR-7 within distinct cellular bodies. Immunoflourescent staining with the P-body specific marker, hDcp1a21, revealed that CiRS-7 co-localized with P-bodies, however only when co-expressed with miR-7 suggesting that miR-7 compartmentalises CiRS-7 to the bodies (Fig. 2d and Figure 8, supplementary Fig 2c).

To investigate whether miR-7 and CiRS-7 are coexpressed at the global scale in the brain, we visualised the expression in the adult mouse brain using an alkaline phosphatase coupled miR-7 and CiRS-7 probe, respectively. The two RNAs showed a distinct neuronal expression (Fig. 2e-h) comparable to GAPDH mRNA (Figure 9, supplementary Fig. 3a), comprising both cortical pyramidal neurons and interneurons (Fig. 2e-h).

The expression of CiRS-7 in the neuropil areas of the hippocampal CA1 and CA3, and in the molecular layer of the dentate gyrus (Fig. 2e-f), likely reflects that CiRS-7 is translocated into the dendritic tree.

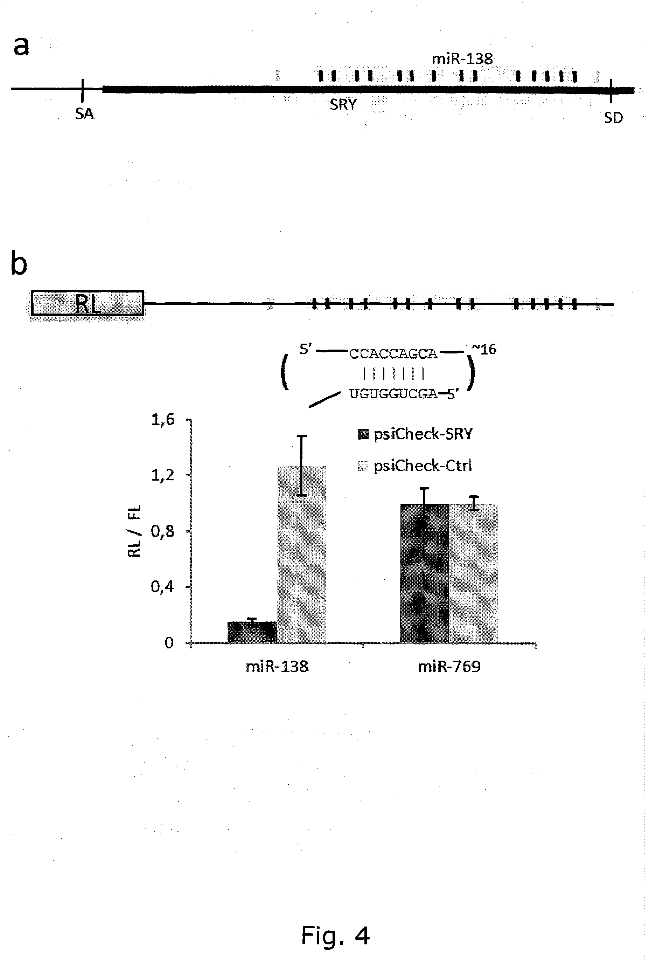

In comparison, miR-7 is expressed in the nucleus and neuronal cell body (Fig. 2g-h, Supplementary Fig 4a), and notably, also in glial cells in the corpus callosum (Fig. 2f).

Both RNAs are also expressed in thalamus (Figure 9, supplementary Fig. 3b, CiRS-7 only) and substantia nigra (data not shown). The very high expression of CiRS-7 coinciding with miR-7 expression strongly suggests that miR-7 is under control of CiRS-7 in rodent brain.

The nuclear localisation of miR-7 was confirmed by subcellular fractionation of cells (Figure 10, supplementary Fig. 4b) which showed a concomitant increase in nuclear prevalence of CiRS-7 when miR-7 was co-expressed (Figure 10, supplementary Fig. 4b-c).

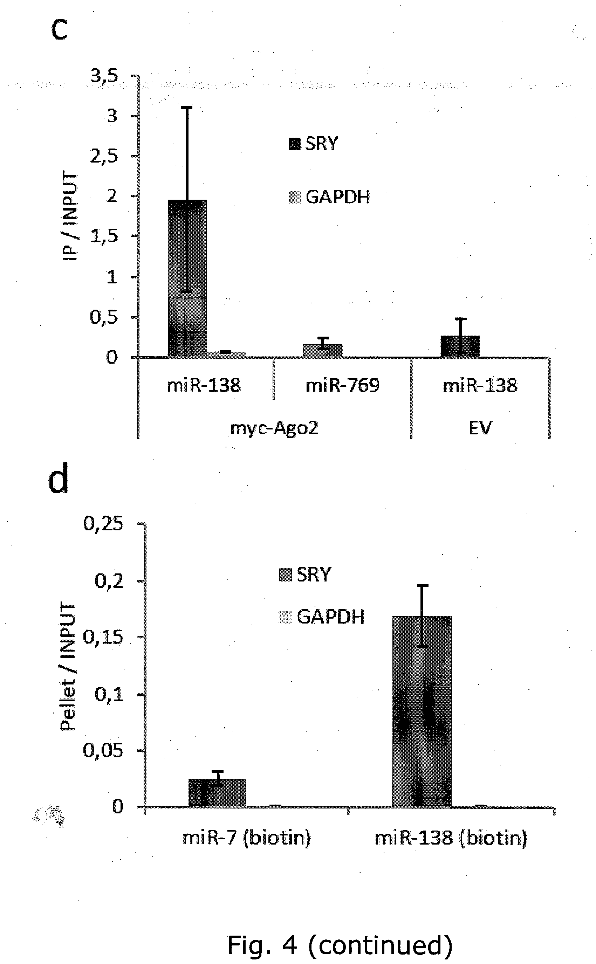

Moreover, the presence of CiRS-7 resulted in elevated levels of miR-7 (Figure 10, supplementary Fig 4c-d), suggesting, collectively, that CiRS-7 and miR-7 have a clear impact on each other in terms of sublocalization within the cell.

To test the effect of CiRS-7 expression on miR-7 activity, the present inventors constructed two luciferase reporter constructs each monitoring miR-7 activity by inserting a perfect miR-7 target (Fig. 3a) or the entire CiRS-7 sequence (Fig. 3b) into the 3'UTR of renilla luciferase (RL).

Co-transfecting the luciferase reporter with miR-7 and CiRS-7 expression vectors showed a substantial reduction of knockdown potential when the CiRS-7 was co-introduced with miR-7 (Fig. 3a and b).

In fact, the expression of CiRS-7 proved more efficient than a conventional anti-miR approach, suggesting that CiRS-7 is indeed a highly efficient miRNA sponge.

Similarly, using the 3'UTR of EGFR, which is a known target for miR-7, in a luciferase reporter assay, CiRS-7 reduced the knockdown efficiency exerted by miR-7 effectively (Figure 11, supplementary Fig. 5).

Next, the present inventors evaluated the effect of CiRS-7 expression upon miR-7-dependent regulation of endogenous targets.

To this end, a single copy of the CiRS-7 expression vector was stably inserted into Flp-In HeLa cells that otherwise have no or very little endogenous expression of CiRS-7.

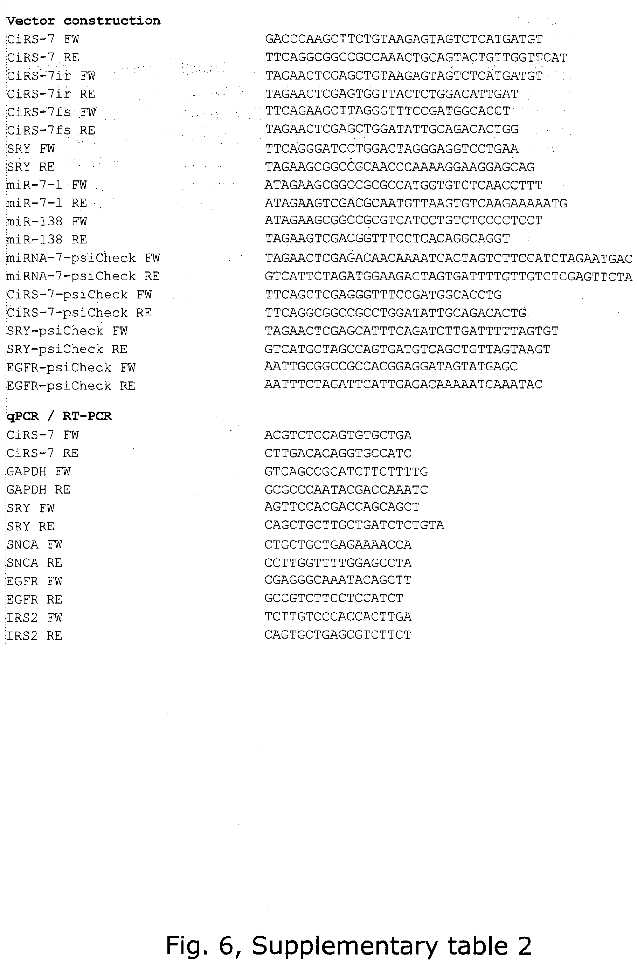

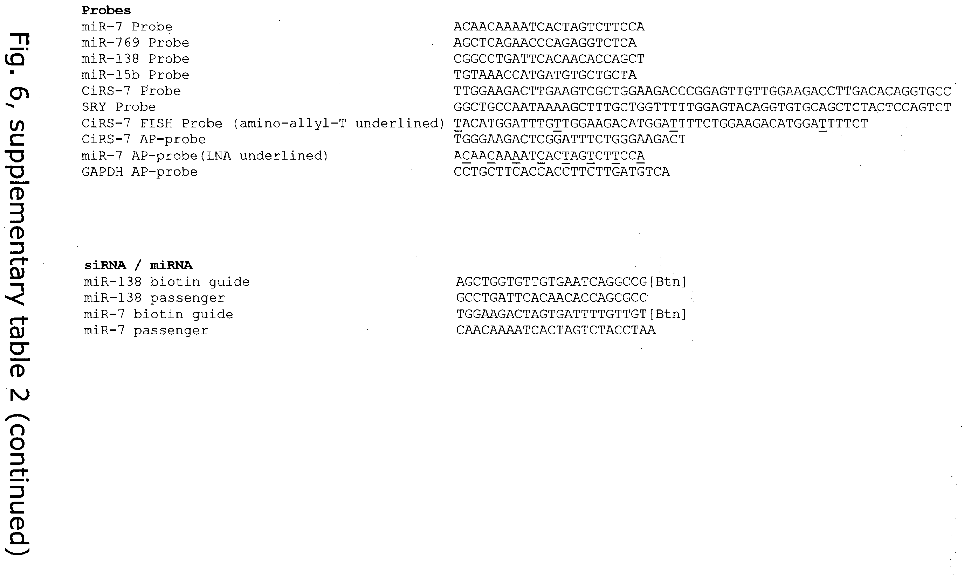

Analysing the expression levels of the constructed HeLa cells by northern and qRT-PCR revealed CiRS-7 levels comparable to the endogenous levels observed in HEK293, however approximately 100-fold less than total brain levels (Figure 12, supplementary Fig. 6a).

As in HEK293 cells, the stably expressed CiRS-7 in HeLa cells was sensitive towards miR-671 as previously published14 but remained resistant towards miR-7 (Fig. 3c and 3g; Figure 12, supplementary Fig. 6b).

Transfecting miR-7 in a dose-gradient manner revealed that established miR-7 targets (SNCA22, EGFR23 and IRS224) responded more efficiently in the empty cell line without CiRS-7 expression (HeLa-EV) compared to CiRS-7-expressing cells (HeLa-CiRS-7) (Fig. 3d-f).

In addition, transfecting miR-671 into HeLa-CiRS-7 prior to introducing dose-gradient miR-7 levels, the evaluated target genes regained miR-7 sensitivity compared to control transfections (Fig. 3h-j).

This suggests that miR-671 likely functions as an indirect regulator of miR-7 activity by targeting and reducing CiRS-7 levels.

The existence of a circular RNA acting as a potent miRNA sponge immediately questions whether circular miRNA sponges are a more general phenomenon.

Indeed, by predicting miRNA target sites within the other well-known, testis-specific circular SRY RNA, the present inventors identified 16 putative target sites for miR-138 (Fig. 4a).

To validate SRY as a miR-138 target transcript, the present inventors inserted the SRY sequence into the 3'UTR of renilla and tested the knockdown potential of miR-138, expressed from a vector (Figure 13, supplementary Fig. 7a).

Indeed, upon co-expression miR-138 with psiCheck-SRY the present inventors observed a dramatic and significant reduction of renilla luminescence compared to control experiments (Fig. 4b).

Next, the present inventors devised a vector expressing circular SRY RNA (Figure 13, supplementary Fig. 7b) to study a direct interaction between miR-138 and SRY. Proper biogenesis of circular SRY was verified by northern blot (Figure 13, supplementary Fig. 7c) and RT-PCR (Figure 13, supplementary Fig. 7d).

Immunoprecipitation of myc-tagged Ago2 from miR-138 transfected cells resulted in enrichment of SRY RNA compared to miR-769 transfected cells (Fig. 4c). Additionally, using biotin-labelled miR-138, SRY RNA was specifically captured (Fig. 4d), suggesting that SRY indeed is able to interact with miR-138 and - like CiRS-7 - acts as a miRNA sponge.

Thus the present inventors propose that the circular SRY be denoted CiRS-138.

Our observation that very abundant endogenous circular RNA molecules, which are inherently resistant to exo-nucleolytic RNA-decay, can serve as efficient miRNA sponges, adds to the growing cellular repertoire of regulatory functions in gene expression.

Conclusively, considering the widespread involvement of miR-7 in cancer as a key regulator of various cancer pathways and also the suggested implication of miR-7 in Parkinson Disease by direct targeting of a-synuclein protein expression, CiRS-7 likely serves as a crucial factor significantly engaged in the functioning of neurons as well as a responsible candidate in neurological disorders and brain tumour development.

Moreover, identifying circular SRY as a CiRS-138 potentially sheds light on the early but undisclosed mechanisms governing male determination.

### Materials and Methods

### Constructs and Transfections

HEK293 Flp-In T-Rex cells (Invitrogen) were maintained under standard culture conditions. Transfections were conducted using either calcium phosphate using standard procedures or Lipofectamine 2000 (Invitrogen) according to supplier's protocol. The construction of pJEBB-769 has previously been published.

pJEBB-7 and pJEBB-138 were constructed similarly; PCR amplification of miR-7-1 locus using miR-7 FW / miR-7 RE or miR-138 locus (in mouse) using miR-138 FW / miR-138 RE and inserting the fragments into a NotI and SalI digested eGFP expression cassette (pJEBB).

pCK-7 was generated by digesting pJEBB-7 with NotI and XbaI and inserting the resulting fragment into NotI and XbaI digested pCK.

pcDNA3-CiRS-7-ir was constructed by PCR amplifying the cRNA locus including 1000 bp upstream and 200 bp downstream the non-linear splice sites using CiRS-7 FW / CiRS-7 RE.

The PCR fragment was inserted into HindIII and NotI digested pcDNA3 (Invitrogen).

An approximately 800 bp DNA stretch upstream the SA was amplified using CiRS-7ir FW / CiRS-7ir RE and inserted in the reverse orientation downstream in an XhoI digested pcDNA3-CiRS-7-ir, thus generating the pcDNA3-CiRS-7.

For the generation of pcDNA3-CiRS-fs, PCR amplification of CiRS-7 exon using CiRS-7ir FW / CiRS-7ir RE was inserted into pcDNA3 using HindIII and XhoI.

To establish HeLa cells stably expressing CiRS-7, CiRS-7 was copy-pasted into pcDNA5 using HindIII and ApaI and transfected into Flp-InTM HeLa cells (Invitrogen) along with pOG44. 48 hrs after transfections, the cell media was supplemented with hygromycin B according to manufacturer's protocol.

pcDNA3-SRY was constructed by PCR amplifying the SRY locus from male mouse DNA using SRY FW / SRY RE and inserting the amplicon into pcDNA3 using BamHI and NotI. Subsequently, SalI digested CMV promoter from pcDNA5 was inserted into the XhoI-site in an inverse orientation.

Luciferase reporter vectors were constructed by inserting annealed primers (miR-7-psiCheck FW / miR-7-psiCheck RE) or PCR amplicons produced by CiRS-7-psiCheck FW / CiRS-7-psiCheck FW, SRY-psiCheck FW / SRY-psiCheck RE or EGFR-psiCheck FW / EGFR-psiCheck RE into psiCheck-2 vector.

Dose-gradient transfection of miR-7 mimic (Applied Biosystems) was conducted using Lipofectamine 2000 (Invitrogen). In each case, a total concentration of 10 nM was used with varying ratios between miR-7 and N.C (negative control) as indicated.

### Northern blot

For miRNA, 20-30 µg whole cell RNA was loaded on a 12% denaturing PAGE gels. RNA was transferred to Amersham hybondTM-N+ membranes (GE Healthcare). The membranes were hybridized with 32P-labelled DNA sis (listed in Table S2) in Church buffer (0.5M NaPO4, 7% SDS, 1mM EDTA, 1% BSA, pH 7.5) at 37oC and washed in 2xSSC (300 mM NaCl, 30 mM Na-citrate, pH 7.0) with 0.1% SDS at room temperature. The membranes were exposed on phosphorimager screens and analyzed using Bio-Rad Quantity One® software (Bio Rad).

For cRNA, agarose northern blot was performed with 5-10 µg RNA separated in 1.2% agarose. Subsequent hybridization and wash was carried out at 50oC, otherwise conducted as described above.

### Ago-IP

Myc-Tagged Ago2 was co-transfected with either pJEBB-7 or pJEBB-769 in p10 dishes. Forty eight hours post-transfection, Ago-IP was performed as described previously26. Briefly, cells were lysed in 150 mM KCI, 25 mM Tris-HCI pH 7.4, 5 mM EDTA, 0.5% Triton X-100, 5 mM DTT supplemented with Ribolock

(Fermentas) and proteinase inhibitor cocktail (Roche). The lysate was mixed with myc-antibody (Abcam, ab9106) coupled sepharose beads (GE Healthcare) and left under rotation for 4 hrs at 4oC. Beads were subsequently washed 5 times in lysis buffer and the RNA was extracted using TRIzol reagent (Invitrogen).

### Streptavidin aptamer

T7 transcription was performed on PCR products containing the entire cRNA sequence with or without the inclusion of a streptavidin aptamer sequence using T7 MEGAscript (Ambion). The streptavidin-based capturing of RNA with associated proteins was conducted as previously published20.

Briefly, the T7 transcript was renatured and mixed with streptavidin beads (Dynabeads MyOneTM Streptavidin C1, Invitrogen) for 1 hr at 4oC and washed twice times in lysis buffer (10 mM HEPES pH 7.0, 200 mM NaCl, 1% Triton X-100, 10 mM MgCl2 and 1 mM DTT).

Cells transfected 48 hrs prior to harvest with either pJEBB-7 or pJEBB-769 were lysed and added to the RNA coupled beads using half a confluent p10 dish per streptavidin capture. After rotating for 4 hrs at 4oC, the beads were washed 5 times and RNA and protein were extracted and analysed by northern blot with probes listed in Table S2 and by western blot using anti-Ago2 (Abcam, ab57113) and anti-HuR (Santa Cruz, SC-5261) as a load control.

### Biotin-coupled miRNA capture

Biotin-labelled miRNAs were transfected into HEK293 cells at a final concentration of 20 nM. Forty eight hours post transfection, cells were harvested and treated as described above.

### IF and RNA-FISH

Cells were seeded on glass coverslips in 12-well plates and transfected with CiRS-7 and pCK-7 using calcium phosphate (HEK293 cells) or Lipofectamine 2000 (HeLa cells). 48 hrs post-transfection, cells were washed in PBS and fixed in 4% paraformaldehyde for 15 min and permeabilized overnight in 70% ethanol. Cells were then washed twice in PBSM, and rehydrated for 10 min in 50% formamide and 2xSSC.

In case of IF, cells were blocked with 3% BSA in PBSM for 1 hr followed by incubation with primary antibody (hDcp1A27) in 3%BSA/PBSM supplemented with RNaseOUT (Fermentas) at 37oC. After washing three times in PBSM, cells were incubated with secondary antibody (Alexa Flour 488, A21206, Invitrogen). Then, cells were washed 3 times before proceeding with RNA FISH.

In case of FISH, cells were incubated at 37°C in a solution containing 50% formamide, 2xSSC, 0.25 mg/mL Escherichia coli tRNA, 0.25 mg/mL salmon sperm DNA (Invitrogen), 2.5 mg/mL BSA (Roche), and 0.5 ng/mL fluorescently labelled CiRS-7 (Figure 6, table S2) and miR-7 (Exiqon, 38485-02) probes. After 3 hrs, cells were washed twice for 20 min at 37°C in 50% formamide and 2xSSC, followed by four 5-min washes in PBS (the penultimate wash containing DAPI) and an additional brief wash in nuclease-free water. Cells were mounted in ProLong Gold (Invitrogen) and left overnight at room temperature.

### Mice for the in vivo study.

This study was performed in accordance with guidelines approved by the Danish Animal Ethical Committee (J. no. 2011/561-1950), using 8-9 week old C57BL/6 male mice (n=5) (Taconic, Denmark). The mice were euthanized by cervical dislocation, the brains frozen in gaseous CO2 and processed into 30 µm coronal cryostat sections.

### In situ hybridization

In situ hybridization was performed as previously described using a custom designed alkaline phosphatase-labelled DNA and LNA probes (Figure 6, table S2) recognizing GAPDH, CiRS-7 and miR7, respectively (DNA Technology A/S, Denmark). The specificity of the signal was tested by hybridizing parallel sections 1) with 100-fold excess of unlabeled probe, 2) pre-treatment with RNase A (Pharmacia Biotech), or 3) buffer alone. All controls were devoid of signal.

### Subcellular fractionation

Subcellular fractionation was performed as previously described. Briefly, cells grown in p10-dishes were gently lysed in 200 ul lysis buffer (10 mM Tris, pH 8.0, 140 mM NaCl, 1.5 mM MgCI2, 0.5% Nonidet P-40), incubated on ice, and centrifuged for 3 min at 1000xg. The supernatant was kept as the cytoplasmic fraction. The nuclear pellet was washed three times in 1 mL lysis buffer. RNA was subsequently purified using TRIzol (Invitrogen).

### Luciferase reporter assay

HEK293 cells seeded in 12-well plates were transfected with 0.5 µg pcDNA3/pcDNA3-CiRS-7, 0.2 µg pJEBB-7/pJEBB-769, and 0.1 µg psiCheck vector, and harvested after 48 hrs using the Dual-Luciferase Reporter Assay kit (Promega). In case of anti-miR experiments, anti-miR™ miRNA Inhibitor (MH10047, Applied Biosystems) was used at a final concentration of 50 nM. Luminescence was measured on a BMG FLUOstar luminometer (BMG labtech, Offenburg Germany).

### RT-PCR and qPCR

Total RNA was extracted from cells in culture using TRIzol reagent (Invitrogen) according to standard procedures. cDNA for qRT-PCR expression analyses was synthesized from total RNA by MLV-RT (Invitrogen) according to the supplied protocol using random hexamer primer. qPCR was performed with Platinum SYBRGreen qPCR Supermix UDG (Invitrogen) on either a MxPro3000 cycler (Stratagene) or on a LightCycler 480 (Roche) according to standard procedures using primers listed in Figure 6, Table S2. RNA levels were normalized to GAPDH.

In case of miRNA taqman qPCR, miR-7 (000386) and miR-769 (001998) assays (Applied Biosystems) were used adhering to manufacturer's protocol.

### Assay I

A preferred target sequence in the circular RNA has at least one mismatch to the microRNA that make it resistant to endonucleolytic cleavage. A preferred sequence is resistant to endonucleolytic cleavage by the microRNA it regulate as interpreted from a simple assay where both the cicular RNA and microRNA is expressed in the same cell (See Figure 12b). The assay, here demonstrated for microRNA-7, can be conducted by a skilled scientist for any other part of circular RNA and microRNA. As an example of a micro-RNA that do not contain any mismatches in the central part, and therefore is able to cleave the circular RNA, microRNA-671 included.

The assay is a Northern blot using RNA from HeLa cells with empty vector expression cassette as a control for probe specificity (HeLa-EV, Fig 12b) or Hela cells expressing circular RNA with 77 target sites for microRNA-7 (HeLa-ciRS-7, Fig 12b). The blot was probed with antisense oligo complementary to the circular RNA (upper panel) and 18S (lower panel, loading control). The asterix (*) denotes the circular RNA.

The Hela cells were transfected with 10 nM non-related control microRNA (N.C.), microRNA-7 or miR-671 mimics (Applied Biosystems) as indicated using Lipofectamine 2000 (Invitrogen). RNA was harvested 48 hours post transfection using TRIzol reagent (Invitrogen). The northern blot was performed with 10 ug RNA separated in 1.2% agarose. RNA was transferred to Amersham hybond-N+ membrane (GE Healthcare). The membrane was hybridized with [32P]-labelled DNA oligonucleotides (ciRS-7 probe:
TTGGAAGACTTGAAGTCGCTGGAAGACCCGGAGTTGTTGGAAGACCTTGACACAGGTGC C, 18S probe:
TTACCGCGGCTGCTGGCACCAGACTTGCCCTCCAATGGATCCTCGTTAAAGGATTTAAAG TGGACTCATTCCAATTACAG) in Church buffer (0.5M NaPO4, 7% SDS, 1mM EDTA, 1% BSA, pH7.5) at 55 °C and washed three times in 2xSSC (300mM NaCl, 30mM Na-citrate, pH7.0) with 0.1% SDS at 50 °C. The membrane was exposed on phosphorimager screen and analysed using Quantity One or Image Lab software (Bio Rad).

### Assay II

A preferred sponge sequence in the circular RNA has at least one mismatch to the microRNA that makes it resistant to Ago2-mediated endonucleolytic cleavage. A preferred sponge sequence is resistant to endonucleolytic cleavage by the microRNA, thus instead of being cleaved by the microRNA, it sequesters and inhibits the microRNA in cells where both the circular RNA and microRNA is expressed (See Figure 14). The assay, here demonstrated for microRNA-7, can be conducted by a skilled scientist for any other pair of circular RNA and microRNA. The circular RNA in this assay contains 74 sponge sites for microRNA-7 and 1 cleavage site for microRNA-671. Therefore, the observed effect is decreased and unchanged levels of circular RNA when introduced to microRNA-671 and microRNA-7, respectively (Figure 14).

Total RNA was extracted from cells in culture using TRIzol reagent (Invitrogen) according to standard procedures. Complementary DNA for qRT-PCR expression analyses was synthesized from 1 ug total RNA by MLV-RT (Invitrogen) according to the supplied protocol using random hexamer primer. Quantitative PCR was performed with Platinum SYBR Green qPCR Supermix UDG (Invitrogen) on either MxPro3000 cycler (Stratagene) or LightCycler 480 (Roche), using primers specific for ciRS-7 (ACGTCTCCAGTGTGCTGA / CTTGACACAGGTGCCATC) or *GAPDH* (GTCAGCCGCATCTTCTTTTG / GCGCCCAATACGACCAAATC).

## Claims

1. A single stranded circular RNA polynucleotide which is at least partly complementary to a human or animal microRNA sequence, for use as a medicament;
wherein the polynucleotide comprises a region of contiguous nucleobase sequence which is 100% complementary to the human or animal microRNA seed region.

2. The single stranded circular RNA polynucleotide for use according to claim 1, which comprises a splice acceptor site (SA) and a splice donor site (SD).

3. The single stranded circular RNA polynucleotide for use according to any of the preceding claims, wherein at least part of the polynucleotide is complementary to the mature human or animal microRNA sequence.

4. The single stranded circular RNA polynucleotide for use according to any of the preceding claims, wherein at least one of the nucleobase units of the polynucleotide is selected among a Locked Nucleic Acid (LNA) unit, a Unlocked Nucleic Acid (UNA) unit, a TINA unit, a 2'-OMe RNA unit and a 2'-fluoro RNA unit.

5. The single stranded circular RNA polynucleotide for use according to any of the preceding claims, wherein the microRNA is selected from the group consisting of miR-7-5p (SEQ ID NO 51), miR-7-1-3p (SEQ ID NO 52), miR-7-2-3p (SEQ ID NO 53), miR-21-5p (SEQ ID NO 54), miR-21-3p (SEQ ID NO 55), miR-96-5p (SEQ ID NO 56), miR-96-3p (SEQ ID NO 57), miR-185-5p (SEQ ID NO 58), miR-185-3p (SEQ ID NO 59), miR-324-5p (SEQ ID NO 60), miR-324a-3p (SEQ ID NO 61), miR-133b (SEQ ID NO 62), miR-205-5p (SEQ ID NO 63), miR-205-3p (SEQ ID NO 64), miR-200a-5p (SEQ ID NO 65), miR-200a-3p (SEQ ID NO 66), miR-200b-5p (SEQ ID NO 67), miR-200b-3p (SEQ ID NO 68), miR-200c-5p (SEQ ID NO 69), miR-200c-3p (SEQ ID NO 70), miR-141-5p (SEQ ID NO 71), miR-141-3p (SEQ ID NO 72), miR-429 (SEQ ID NO 73), miR-132-5p (SEQ ID NO 74), miR-132-3p (SEQ ID NO 75), miR-134 (SEQ ID NO 76), miR-124-5p (SEQ ID NO 77), miR-124-3p (SEQ ID NO 78), miR-155-5p (SEQ ID NO 79), miR-155-3p (SEQ ID NO 80), miR-221-5p (SEQ ID NO 81), miR-221-3p (SEQ ID NO 82), miR-222-5p (SEQ ID NO 83), miR-222-3p (SEQ ID NO 84), miR-122-5p (SEQ ID NO 85), miR-122-3p (SEQ ID NO 86).

6. The single stranded circular RNA polynucleotide for use according to any of the preceding claims, comprising a sequence selected from the group consisting of sequences that are capable of base pairing to the complementary sequence of a sequence selected from the group consisting of position 1-20, position 1-19, position 1-18, position 1-17, position 1-16, position 1-15, position 1-14, position 1-13, position 1-12, position 1-11, position 1-10, position 1-9, position 1-8, position 1-7, position 1-6, position 2-20, position 2-19, position 2-18, position 2-17, position 2-16, position 2-15, position 2-14, position 2-13, position 2-12, position 2-11, position 2-10, position 2-9, position 2-8, position 2-7, position 2-6, position 3-20, position 3-19, position 3-18, position 3-17, position 3-16, position 3-15, position 3-14, position 3-13, position 3-12, position 3-11, position 3-10 and position 3-9 of a human microRNA.

7. A pharmaceutical composition comprising
- a single stranded circular RNA polynucleotide, which is at least partly complementary to a human or animal microRNA sequence, wherein the polynucleotide comprises a region of contiguous nucleobase sequence which is 100% complementary to the human or animal microRNA seed region; and
- at least one of a pharmaceutically acceptable diluent, carrier and adjuvant;
for use as a medicament.

8. A single stranded circular RNA polynucleotide according to any of claims 1-6 for use in the treatment, amelioration or prevention of a disease or medical disorder associated with the presence or over-expression of microRNA, wherein the disease or medical disorder is selected from the group consisting of Parkinson's disease, cancer, a viral infection, diabetes, a neurological disease.

9. In vitro use of a single stranded circular RNA polynucleotide as defined in any of the claims 1 - 6 for blocking and inhibition of RNA.

## Patentansprüche

1. Einsträngiges kreisförmiges RNA-Polynukleotid, das zumindest teilweise zu einer menschlichen oder tierischen microRNA-Sequenz komplementär ist, zur Verwendung als ein Medikament;
wobei das Polynukleotid eine Region von zusammenhängender Nukleobasensequenz umfasst, die zu der menschlichen oder tierischen microRNA-Seed-Region 100 % komplementär ist.

2. Einsträngiges kreisförmiges RNA-Polynukleotid zur Verwendung nach Anspruch 1, das eine Akzeptor-Spleißstelle (SA) und eine Spender-Spleißstelle (SD) umfasst.

3. Einsträngiges kreisförmiges RNA-Polynukleotid zur Verwendung nach einem der vorhergehenden Ansprüche, wobei zumindest ein Teil des Polynukleotids zu der reifen menschlichen oder tierischen microRNA-Sequenz komplementär ist.

4. Einsträngiges kreisförmiges RNA-Polynukleotid zur Verwendung nach einem der vorhergehenden Ansprüche, wobei zumindest eine der Nukleobaseneinheiten des Polynukleotids aus einer verbrückten Nukleinsäure (LNA)-Einheit, einer unverbrückten Nukleinsäure (UNA)-Einheit, einer TINA-Einheit, einer 2'-OMe-RNA-Einheit und einer 2'-Fluor-RNA-Einheit ausgewählt wird.

5. Einsträngiges kreisförmiges RNA-Polynukleotid zur Verwendung nach einem der vorhergehenden Ansprüche, wobei die microRNA ausgewählt ist aus der Gruppe bestehend aus miR-7-5p (SEQ ID NO 51), miR-7-1-3p (SEQ ID NO 52), miR-7-2-3p (SEQ ID NO 53), miR-21-5p (SEQ ID NO 54), miR-21-3p (SEQ ID NO 55), miR-96-5p (SEQ ID NO 56), miR-96-3p (SEQ ID NO 57), miR-185-5p (SEQ ID NO 58), miR-185-3p (SEQ ID NO 59), miR-324-5p (SEQ ID NO 60), miR-324a-3p (SEQ ID NO 61), miR-133b (SEQ ID NO 62), miR-205-5p (SEQ ID NO 63), miR-205-3p (SEQ ID NO 64), miR-200a-5p (SEQ ID NO 65), miR-200a-3p (SEQ ID NO 66), miR-200b-5p (SEQ ID NO 67), miR-200b-3p (SEQ ID NO 68), miR-200c-5p (SEQ ID NO 69), miR-200c-3p (SEQ ID NO 70), miR-141-5p (SEQ ID NO 71), miR-141-3p (SEQ ID NO 72), miR-429 (SEQ ID NO 73), miR-132-5p (SEQ ID NO 74), miR-132-3p (SEQ ID NO 75), miR-134 (SEQ ID NO 76), miR-124-5p (SEQ ID NO 77), miR-124-3p (SEQ ID NO 78), miR-155-5p (SEQ ID NO 79), miR-155-3p (SEQ ID NO 80), miR-221-5p (SEQ ID NO 81), miR-221-3p (SEQ ID NO 82), miR-222-5p (SEQ ID NO 83), miR-222-3p (SEQ ID NO 84), miR-122-5p (SEQ ID NO 85), miR-122-3p (SEQ ID NO 86).

6. Einsträngiges kreisförmiges RNA-Polynukleotid zur Verwendung nach einem der vorhergehenden Ansprüche, umfassend eine Sequenz ausgewählt aus der Gruppe bestehend aus Sequenzen, die zum Basen-Pairing mit der komplementären Sequenz einer Sequenz ausgewählt aus der Gruppe bestehend aus Position 1-20, Position 1-19, Position 1-18, Position 1-17, Position 1-16, Position 1-15, Position 1-14, Position 1-13, Position 1-12, Position 1-11, Position 1-10, Position 1-9, Position 1-8, Position 1-7, Position 1-6, Position 2-20, Position 2-19, Position 2-18, Position 2-17, Position 2-16, Position 2-15, Position 2-14, Position 2-13, Position 2-12, Position 2-11, Position 2-10, Position 2-9, Position 2-8, Position 2-7, Position 2-6, Position 3-20, Position 3-19, Position 3-18, Position 3-17, Position 3-16, Position 3-15, Position 3-14, Position 3-13, Position 3-12, Position 3-11, Position 3-10 und Position 3-9 einer menschlichen microRNA in der Lage ist.

7. Pharmazeutische Zusammensetzung, umfassend
- ein einsträngiges kreisförmiges RNA-Polynukleotid, das zumindest teilweise zu einer menschlichen oder tierischen microRNA-Sequenz komplementär ist, wobei das Polynukleotid eine Region von zusammenhängender Nukleobasensequenz umfasst, die zu der menschlichen oder tierischen microRNA-Seed-Region 100 % komplementär ist; und
- zumindest einen von einem pharmazeutisch annehmbaren Verdünnungsmittel, Träger oder Wirkverstärker;
zur Verwendung als ein Medikament.

8. Einsträngiges kreisförmiges RNA-Polynukleotid nach einem der Ansprüche 1-6 zur Verwendung bei der Behandlung, Verbesserung oder Vorbeugung einer Krankheit oder medizinischen Erkrankung in Zusammenhang mit dem Vorhandensein oder der Überexpression von microRNA,
wobei die Krankheit oder medizinische Erkrankung ausgewählt ist aus der Gruppe bestehend aus Parkinson-Krankheit, Krebs, einer Virusinfektion, Diabetes, einer neurologischen Krankheit.

9. In-vitro-Verwendung eines einsträngigen kreisförmigen RNA-Polynukleotids, wie definiert in einem der Ansprüche 1-6 zum Blockieren und Hemmen von RNA.

## Revendications

1. Polynucléotide d'ARN circulaire monocaténaire qui est au moins en partie complémentaire à une séquence de micro-ARN humain ou animal, pour utilisation comme médicament ;
ledit polynucléotide comprenant une région de séquence de nucléobases contiguë qui est 100 % complémentaire à la région germe de micro-ARN animal ou humain.

2. Polynucléotide d'ARN circulaire monocaténaire pour utilisation selon la revendication 1, qui comprend un site accepteur d'épissage (SA) et un site donneur d'épissage (SD).

3. Polynucléotide d'ARN circulaire monocaténaire pour utilisation selon l'une quelconque des revendications précédentes, au moins une partie du polynucléotide étant complémentaire à la séquence de micro-ARN animal ou humain mature.

4. Polynucléotide d'ARN circulaire monocaténaire pour utilisation selon l'une quelconque des revendications précédentes, au moins l'un des motifs de nucléobases du polynucléotide étant choisi parmi un motif d'acide nucléique bloqué (LNA), un motif d'acide nucléique débloqué (UNA), un motif TINA, un motif d'ARN à groupe 2'-OMe et un motif d'ARN à groupe 2'-fluoro.

5. Polynucléotide d'ARN circulaire monocaténaire pour utilisation selon l'une quelconque des revendications précédentes, ledit micro-ARN étant choisi dans le groupe constitué par miR-7-5p (SEQ ID n° 51), miR-7-1-3p (SEQ ID n° 52), miR-7-2-3p (SEQ ID n° 53), miR-21-5p (SEQ ID n° 54), miR-21-3p (SEQ ID n° 55), miR-96-5p (SEQ ID n° 56), miR-96-3p (SEQ ID n° 57), miR-185-5p (SEQ ID n° 58), miR-185-3p (SEQ ID n° 59), miR-324-5p (SEQ ID n° 60), miR-324a-3p (SEQ ID n° 61), miR-133b (SEQ ID n° 62), miR-205-5p (SEQ ID n° 63), miR-205-3p (SEQ ID n° 64), miR-200a-5p (SEQ ID n° 65), miR-200a-3p (SEQ ID n° 66), miR-200b-5p (SEQ ID n° 67), miR-200b-3p (SEQ ID n° 68), miR-200c-5p (SEQ ID n° 69), miR-200c-3p (SEQ ID n° 70), miR-141-5p (SEQ ID n° 71), miR-141-3p (SEQ ID n° 72), miR-429 (SEQ ID n° 73), miR-132-5p (SEQ ID n° 74), miR-132-3p (SEQ ID n° 75), miR-134 (SEQ ID n° 76), miR-124-5p (SEQ ID n° 77), miR-124-3p (SEQ ID n° 78), miR-155-5p (SEQ ID n° 79), miR-155-3p (SEQ ID n° 80), miR-221-5p (SEQ ID n° 81), miR-221-3p (SEQ ID n° 82), miR-222-5p (SEQ ID n° 83), miR-222-3p (SEQ ID n° 84), miR-122-5p (SEQ ID n° 85), miR-122-3p (SEQ ID n° 86).

6. Polynucléotide d'ARN circulaire monocaténaire pour utilisation selon l'une quelconque des revendications précédentes, comprenant une séquence choisie dans le groupe constitué par les séquences qui sont capables d'un appariement de bases à la séquence complémentaire d'une séquence choisie dans le groupe constitué par les positions 1 à 20, les positions 1 à 19, les positions 1 à 18, les positions 1 à 17, les positions 1 à 16, les positions 1 à 15, les positions 1 à 14, les positions 1 à 13, les positions 1 à 12, les positions 1 à 11, les positions 1 à 10, les positions 1 à 9, les positions 1 à 8, les positions 1 à 7, les positions 1 à 6, les positions 2 à 20, les positions 2 à 19, les positions 2 à 18, les positions 2 à 17, les positions 2 à 16, les positions 2 à 15, les positions 2 à 14, les positions 2 à 13, les positions 2 à 12, les positions 2 à 11, les positions 2 à 10, les positions 2 à 9, les positions 2 à 8, les positions 2 à 7, les positions 2 à 6, les positions 3 à 20, les positions 3 à 19, les positions 3 à 18, les positions 3 à 17, les positions 3 à 16, les positions 3 à 15, les positions 3 à 14, les positions 3 à 13, les positions 3 à 12, les positions 3 à 11, les positions 3 à 10 et les positions 3 à 9 d'un micro-ARN humain.

7. Composition pharmaceutique comprenant
- un polynucléotide d'ARN circulaire monocaténaire, qui est au moins en partie complémentaire à une séquence de micro-ARN humain ou animal, ledit polynucléotide comprenant une région de séquence de nucléobases contiguë qui est complémentaire à 100 % à la région germe de micro-ARN animal ou humain ; et
- au moins l'un d'un diluant, vecteur et adjuvant pharmaceutiquement acceptables ;
pour utilisation comme médicament.

8. Polynucléotide d'ARN circulaire monocaténaire selon l'une quelconque des revendications 1 à 6 pour utilisation dans le traitement, l'amélioration ou la prévention d'une maladie ou d'un trouble médical associé à la présence ou la surexpression d'un micro-ARN,
ladite maladie ou ledit trouble médical étant choisi dans le groupe constitué par la maladie de Parkinson, un cancer, une infection virale, le diabète, une maladie neurologique.

9. Utilisation in vitro d'un polynucléotide d'ARN circulaire monocaténaire tel que défini dans l'une quelconque des revendications 1 à 6 pour le blocage et l'inhibition d'un ARN.


### SEQUENCE LISTING

<110> Aarhus University
   Hansen, Thomas
   Kjems, Jørgen
<120> Circular RNA for inhibition of microRNA
<130> 50536US01
<160> 86
<170> PatentIn version 3.5
<210> 1
   <211> 34
   <212> DNA
   <213> Artificial Sequence
<220>
   <223> Primer
<400> 1
   gacccaagct tctgtaagag tagtctcatg atgt 34
<210> 2
   <211> 37
   <212> DNA
   <213> Artificial Sequence
<220>
   <223> Primer
<400> 2
   ttcaggcggc cgccaaactg cagtactgtt ggttcat 37
<210> 3
   <211> 34
   <212> DNA
   <213> Artificial Sequence
<220>
   <223> Primer
<400> 3
   tagaactcga gctgtaagag tagtctcatg atgt 34
<210> 4
   <211> 31
   <212> DNA
   <213> Artificial Sequence
<220>
   <223> Primer
<400> 4
   tagaactcga gtggttactc tggacattga t 31
<210> 5
   <211> 30
   <212> DNA
   <213> Artificial Sequence
<220>
   <223> Primer
<400> 5
   ttcagaagct tagggtttcc gatggcacct 30
<210> 6
   <211> 31
   <212> DNA
   <213> Artificial Sequence
<220>
   <223> Primer
<400> 6
   tagaactcga gctggatatt gcagacactg g 31
<210> 7
   <211> 31
   <212> DNA
   <213> Artificial Sequence
<220>
   <223> Primer
<400> 7
   ttcagggatc ctggactagg gaggtcctga a 31
<210> 8
   <211> 33
   <212> DNA
   <213> Artificial Sequence
<220>
   <223> Primer
<400> 8
   tagaagcggc cgcaacccaa aaggaaggag cag 33
<210> 9
   <211> 34
   <212> DNA
   <213> Artificial Sequence
<220>
   <223> Primer
<400> 9
   atagaagcgg ccgcgccatg gtgtctcaac cttt 34
<210> 10
   <211> 37
   <212> DNA
   <213> Artificial Sequence
<220>
   <223> Primer
<400> 10
   atagaagtcg acgcaatgtt aagtgtcaag aaaaatg 37
<210> 11
   <211> 34
   <212> DNA
   <213> Artificial Sequence
<220>
   <223> Primer
<400> 11
   atagaagcgg ccgcgtcatc ctgtctcccc tcct 34
<210> 12
   <211> 30
   <212> DNA
   <213> Artificial Sequence
<220>
   <223> Primer
<400> 12
   tagaagtcga cggtttcctc acaggcaggt 30
<210> 13
   <211> 45
   <212> DNA
   <213> Artificial Sequence
<220>
   <223> Primer
<400> 13
   tagaactcga gacaacaaaa tcactagtct tccatctaga atgac 45
<210> 14
   <211> 45
   <212> DNA
   <213> Artificial Sequence
<220>
   <223> Primer
<400> 14
   gtcattctag atggaagact agtgattttg ttgtctcgag ttcta 45
<210> 15
   <211> 29
   <212> DNA
   <213> Artificial Sequence
<220>
   <223> Primer
<400> 15
   ttcagctcga gggtttccga tggcacctg 29
<210> 16
   <211> 32
   <212> DNA
   <213> Artificial Sequence
<220>
   <223> Primer
<400> 16
   ttcaggcggc cgcctggata ttgcagacac tg 32
<210> 17
   <211> 36
   <212> DNA
   <213> Artificial Sequence
<220>
   <223> Primer
<400> 17
   tagaactcga gcatttcaga tcttgatttt tagtgt 36
<210> 18
   <211> 35
   <212> DNA
   <213> Artificial Sequence
<220>
   <223> Primer
<400> 18
   gtcatgctag ccagtgatgt cagctgttag taagt 35
<210> 19
   <211> 31
   <212> DNA
   <213> Artificial Sequence
<220>
   <223> Primer
<400> 19
   aattgcggcc gccacggagg atagtatgag c 31
<210> 20
   <211> 34
   <212> DNA
   <213> Artificial Sequence
<220>
   <223> Primer
<400> 20
   aatttctaga ttcattgaga caaaaatcaa atac 34
<210> 21
   <211> 18
   <212> DNA
   <213> Artificial Sequence
<220>
   <223> Primer
<400> 21
   acgtctccag tgtgctga 18
<210> 22
   <211> 18
   <212> DNA
   <213> Artificial Sequence
<220>
   <223> Primer
<400> 22
   cttgacacag gtgccatc 18
<210> 23
   <211> 20
   <212> DNA
   <213> Artificial Sequence
<220>
   <223> Primer
<400> 23
   gtcagccgca tcttcttttg 20
<210> 24
   <211> 20
   <212> DNA
   <213> Artificial Sequence
<220>
   <223> Primer
<400> 24
   gcgcccaata cgaccaaatc 20
<210> 25
   <211> 19
   <212> DNA
   <213> Artificial Sequence
<220>
   <223> Primer
<400> 25
   agttccacga ccagcagct 19
<210> 26
   <211> 22
   <212> DNA
   <213> Artificial Sequence
<220>
   <223> Primer
<400> 26
   cagctgcttg ctgatctctg ta 22
<210> 27
   <211> 18
   <212> DNA
   <213> Artificial Sequence
<220>
   <223> Primer
<400> 27
   ctgctgctga gaaaacca 18
<210> 28
   <211> 18
   <212> DNA
   <213> Artificial Sequence
<220>
   <223> Primer
<400> 28
   ccttggtttt ggagccta 18
<210> 29
   <211> 18
   <212> DNA
   <213> Artificial Sequence
<220>
   <223> Primer
<400> 29
   cgagggcaaa tacagctt 18
<210> 30
   <211> 17
   <212> DNA
   <213> Artificial Sequence
<220>
   <223> Primer
<400> 30
   gccgtcttcc tccatct 17
<210> 31
   <211> 18
   <212> DNA
   <213> Artificial Sequence
<220>
   <223> Primer
<400> 31
   tcttgtccca ccacttga 18
<210> 32
   <211> 18
   <212> DNA
   <213> Artificial Sequence
<220>
   <223> Primer
<400> 32
   cagtgctgag cgtcttct 18
<210> 33
   <211> 23
   <212> DNA
   <213> Artificial Sequence
<220>
   <223> Primer
<400> 33
   acaacaaaat cactagtctt cca 23
<210> 34
   <211> 22
   <212> DNA
   <213> Artificial Sequence
<220>
   <223> Primer
<400> 34
   agctcagaac ccagaggtct ca 22
<210> 35
   <211> 23
   <212> DNA
   <213> Artificial Sequence
<220>
   <223> Primer
<400> 35
   cggcctgatt cacaacacca gct 23
<210> 36
   <211> 22
   <212> DNA
   <213> Artificial Sequence
<220>
   <223> Primer
<400> 36
   tgtaaaccat gatgtgctgc ta 22
<210> 37
   <211> 60
   <212> DNA
   <213> Artificial Sequence
<220>
   <223> Primer
<400> 37
   ttggaagact tgaagtcgct ggaagacccg gagttgttgg aagaccttga cacaggtgcc 60
<210> 38
   <211> 60
   <212> DNA
   <213> Artificial Sequence
<220>
   <223> Primer
<400> 38
   ggctgccaat aaaagctttg ctggtttttg gagtacaggt gtgcagctct actccagtct 60
<210> 39
   <211> 50
   <212> DNA
   <213> Artificial Sequence
<220>
   <223> Primer
<400> 39
   tacatggatt tgttggaaga catggatttt ctggaagaca tggattttct 50
<210> 40
   <211> 28
   <212> DNA
   <213> Artificial Sequence
<220>
   <223> Primer
<400> 40
   tgggaagact cggatttctg ggaagact 28
<210> 41
   <211> 23
   <212> DNA
   <213> Artificial Sequence
<220>
   <223> Primer
<400> 41
   acaacaaaat cactagtctt cca 23
<210> 42
   <211> 26
   <212> DNA
   <213> Artificial Sequence
<220>
   <223> Primer
<400> 42
   cctgcttcac caccttcttg atgtca 26
<210> 43
   <211> 23
   <212> DNA
   <213> Artificial Sequence
<220>
   <223> miRNA
<400> 43
   agctggtgtt gtgaatcagg ccg 23
<210> 44
   <211> 23
   <212> DNA
   <213> Artificial Sequence
<220>
   <223> miRNA
<400> 44
   gcctgattca caacaccagc gcc 23
<210> 45
   <211> 23
   <212> DNA
   <213> Artificial Sequence
<220>
   <223> miRNA
<400> 45
   tggaagacta gtgattttgt tgt 23
<210> 46
   <211> 24
   <212> DNA
   <213> Artificial Sequence
<220>
   <223> miRNA
<400> 46
   caacaaaatc actagtctac ctaa 24
<210> 47
   <211> 9035
   <212> DNA
   <213> Artificial Sequence
<220>
   <223> vector
<400> 47
   gacggatcgg gagatctccc gatcccctat ggtcgactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gcttctgtaa     900
   gagtagtctc atgatgtctt ttaatttaat atctaggccc taatacacaa ctctttgaat     960
   attgctacta tctttcagca tgactttcca ataccattaa gattaagcct ttctgttctt    1020
   cagtttctct aactcaagtc ttctaagctc cacagcttcc aacatcttta attaccagat    1080
   cttcccaata ttataacatc cgtgcattcc agtccctgtc ctgcagcatc tccaatgccc    1140
   ttgttttcta aacttcccag tattcaagtc attaaaaatt taggtcttct aatatctcca    1200
   acaacccatc ttcaaagtcc aggtcttgtc tttcagaatc cagagttcta aattacaagt    1260
   cttccatggt cctaatcctc caatgtctcc tatatacagg tcttctagag gctatgtatt    1320
   tcatagtgtt taagtctaga ttcttcaatg tcttccagca cttacatcat tcagttttta    1380
   catttttaaa tgtctcctac tttctagttt tacagtgtat ttttatcgcc aattcctcca    1440
   atgcccaagt ctttaaatac ctgcaagaac tttcaggtac atcaatatcc tcaatgtctg    1500
   aatgaataga cctcttaata tcctggtctc atattgtcca ggctgccagt attcttgaca    1560
   tccagcatct ttaaaattta attcttccaa actgtctttt tgacataaat atgtcaatat    1620
   ccatgcttac caatgtctcc caatatccag ggcttctcat tttaggattt ataaatgtct    1680
   acatctgctg ttgtttatat ctgccagtat ttaggagttc caaggtacag gacttacaat     1740
   atctctaatt tccagagatt tcagtgtcta tgacttccaa tattcaatat cccaatgttc     1800
   agggcctcca gaaatatcaa tgtccagagt aaccaatgtc ttcaaaatcc aaggcttgca     1860
   atgtctccaa tatcctgtgc ttacaatgtt tccaatgtcc agggtttccg atggcacctg     1920
   tgtcaaggtc ttccaacaac tccgggtctt ccagcgactt caagtcttcc aataatctca     1980
   aggtcttcca gataatcctg agcttccaga aaatccacat cttccagaca atccatgtct     2040
   tccggacaat ccatgtcttc caagaagctc caagtcttcc agtaaatcaa gtcttccagc     2100
   aaatccagtc ttccagcaat tactggtctt ccaccaaatc cagatcttcc aggaaaatcc     2160
   acgtcttcca ggaaatccat gtcttccaat aatttcaagg tcttccatca aatacagatc     2220
   ttccagctaa tccatgtctt ccagaaaaat ctgtgtcttc caccaaatcc aagtcttcca     2280
   gtaaatctag ttcttccaga aaaatctaga tcttccagtc aatcagtgtc ttccagaaag     2340
   aaatccaggt cttccagtca atcagtgtct tccagaaaga aatccaggtc ttccagtcag     2400
   tcagtgtctt ccagaaaaat ctacgtcttc caccaaatcc aggtcttcca gtcaatccac     2460
   atcttccgga aaaaatccag gtcttccagc caatatatgt cttcctgaag atccacgtct     2520
   tccagaaaat ccatgtcttc cagaaaatcc atgtcttcca gtaacctccc agtcttccag     2580
   aaaatccacg tcttcccaac aatccaagtc ttccggataa tttgggtctt cctgaaaatc     2640
   tacgtcttcc aaaaaagcca tgtcttccag aaaatccaca tcttccaatg gcctccaggt     2700
   cttccagact atccatgtct tccagaaaat ccttgtcttc ccttaaatct atagcttcca     2760
   aaaaatccgg gtcttccagg aaatccgtgt cttccagcaa gtccacgtct tccaacaaag     2820
   ccatgtcttc cagactatcc atgtcttcca gaaaatcctt gtcttccctc aaatccatag     2880
   cttccgaaaa atccaggtct tccaggaaat ccgtgtcttc cagcaaatcc acgtcttcca     2940
   acaaagccat gtcttccatc aaattaatgt cttccagcct acttgtgtct tccaacaaag     3000
   gtacgtcttc caacaaaggt acgtcttcca acaaaggtat gtcttccaac aaaggtacgt     3060
   cttccagaaa atccacgtct tccaaccaag ccatgtcttc cagaaaatcc acgtcttcca     3120
   gaaaatatat gtcttccaac taagctacgt cttccaacaa atccatgtct tcctatatct     3180
   ccaggtcttc cagcatctcc agggcttcca gcatctgctc gtcttccaac atctccacgt     3240
   cttccagcat ctctgtgtct tccagcatct tcatgtcttc caacaactac ccagtcttcc     3300
   atcaactggc tcaatatcca tgtcttccaa cgtctccagt gtgctgatct tctgacattc     3360
   aggtcttcca gtgtctgcaa tatccaggta ttctaacatg ttctatagtc cagatcttcc     3420
   agcctttccc caggtccagg ccttttcaaa tctaggcttc ccctgtctga gccttcccat     3480
   gttaaaatca ctctacagaa aaatcttcca actcaactat ttctgcttct agttgaatta     3540
   gcttcaaaaa catttaatgg gacattcctc cttatagacc agatcttatg aaccaacagt 3600
   actgcagttt ggcggccgct tggttactct ggacattgat atttctggag gccctgaaca 3660
   ttgggatatt gaatattgga agtcatagac actgaaatct ctggaaatta gagatattgt 3720
   aagtcctgta ccttggaact cctaaatact ggcagatata aacaacagca gatgtagaca 3780
   tttataaatc ctaaaatgag aagccctgga tattgggaga cattggtaag catggatatt 3840
   gacatattta tgtcaaaaag acagtttgga agaattaaat tttaaagatg ctggatgtca 3900
   agaatactgg cagcctggac aatatgagac caggatatta agaggtctat tcattcagac 3960
   attgaggata ttgatgtacc tgaaagttct tgcaggtatt taaagacttg ggcattggag 4020
   gaattggcga taaaaataca ctgtaaaact agaaagtagg agacatttaa aaatgtaaaa 4080
   actgaatgat gtaagtgctg gaagacattg aagaatctag acttaaacac tatgaaatac 4140
   atagcctcta gaagacctgt atataggaga cattggagga ttaggaccat ggaagacttg 4200
   taatttagaa ctctggattc tgaaagacaa gacctggact ttgaagatgg gttgttggag 4260
   atattagaag acctaaattt ttaatgactt gaatactggg aagtttagaa aacaagggca 4320
   ttggagatgc tgcaggacag ggactggaat gcacggatgt tataatattg ggaagatctg 4380
   gtaattaaag atgttggaag ctgtggagct tagaagactt gagttagaga aactgaagaa 4440
   cagaaaggct taatcttaat ggtattggaa agtcatgctg aaagatagta gcaatattca 4500
   aagagttgtg tattagggcc tagatattaa attaaaagac atcatgagac tactcttaca 4560
   gcgagcatgc atctagaggg ccctattcta tagtgtcacc taaatgctag agctcgctga 4620
   tcagcctcga ctgtgccttc tagttgccag ccatctgttg tttgcccctc ccccgtgcct 4680
   tccttgaccc tggaaggtgc cactcccact gtcctttcct aataaaatga ggaaattgca 4740
   tcgcattgtc tgagtaggtg tcattctatt ctggggggtg gggtggggca ggacagcaag 4800
   ggggaggatt gggaagacaa tagcaggcat gctggggatg cggtgggctc tatggcttct 4860
   gaggcggaaa gaaccagctg gggctctagg gggtatcccc acgcgccctg tagcggcgca 4920
   ttaagcgcgg cgggtgtggt ggttacgcgc agcgtgaccg ctacacttgc cagcgcccta 4980
   gcgcccgctc ctttcgcttt cttcccttcc tttctcgcca cgttcgccgg ctttccccgt 5040
   caagctctaa atcggggcat ccctttaggg ttccgattta gtgctttacg gcacctcgac 5100
   cccaaaaaac ttgattaggg tgatggttca cgtagtgggc catcgccctg atagacggtt 5160
   tttcgccctt tgacgttgga gtccacgttc tttaatagtg gactcttgtt ccaaactgga 5220
   acaacactca accctatctc ggtctattct tttgatttat aagggatttt ggggatttcg 5280
   gcctattggt taaaaaatga gctgatttaa caaaaattta acgcgaatta attctgtgga 5340
   atgtgtgtca gttagggtgt ggaaagtccc caggctcccc aggcaggcag aagtatgcaa 5400
   agcatgcatc tcaattagtc agcaaccagg tgtggaaagt ccccaggctc cccagcaggc 5460
   agaagtatgc aaagcatgca tctcaattag tcagcaacca tagtcccgcc cctaactccg 5520
   cccatcccgc ccctaactcc gcccagttcc gcccattctc cgccccatgg ctgactaatt 5580
   ttttttattt atgcagaggc cgaggccgcc tctgcctctg agctattcca gaagtagtga 5640
   ggaggctttt ttggaggcct aggcttttgc aaaaagctcc cgggagcttg tatatccatt 5700
   ttcggatctg atcaagagac aggatgagga tcgtttcgca tgattgaaca agatggattg 5760
   cacgcaggtt ctccggccgc ttgggtggag aggctattcg gctatgactg ggcacaacag 5820
   acaatcggct gctctgatgc cgccgtgttc cggctgtcag cgcaggggcg cccggttctt 5880
   tttgtcaaga ccgacctgtc cggtgccctg aatgaactgc aggacgaggc agcgcggcta 5940
   tcgtggctgg ccacgacggg cgttccttgc gcagctgtgc tcgacgttgt cactgaagcg 6000
   ggaagggact ggctgctatt gggcgaagtg ccggggcagg atctcctgtc atctcacctt 6060
   gctcctgccg agaaagtatc catcatggct gatgcaatgc ggcggctgca tacgcttgat 6120
   ccggctacct gcccattcga ccaccaagcg aaacatcgca tcgagcgagc acgtactcgg 6180
   atggaagccg gtcttgtcga tcaggatgat ctggacgaag agcatcaggg gctcgcgcca 6240
   gccgaactgt tcgccaggct caaggcgcgc atgcccgacg gcgaggatct cgtcgtgacc 6300
   catggcgatg cctgcttgcc gaatatcatg gtggaaaatg gccgcttttc tggattcatc 6360
   gactgtggcc ggctgggtgt ggcggaccgc tatcaggaca tagcgttggc tacccgtgat 6420
   attgctgaag agcttggcgg cgaatgggct gaccgcttcc tcgtgcttta cggtatcgcc 6480
   gctcccgatt cgcagcgcat cgccttctat cgccttcttg acgagttctt ctgagcggga 6540
   ctctggggtt cgaaatgacc gaccaagcga cgcccaacct gccatcacga gatttcgatt 6600
   ccaccgccgc cttctatgaa aggttgggct tcggaatcgt tttccgggac gccggctgga 6660
   tgatcctcca gcgcggggat ctcatgctgg agttcttcgc ccaccccaac ttgtttattg 6720
   cagcttataa tggttacaaa taaagcaata gcatcacaaa tttcacaaat aaagcatttt 6780
   tttcactgca ttctagttgt ggtttgtcca aactcatcaa tgtatcttat catgtctgta 6840
   taccgtcgac ctctagctag agcttggcgt aatcatggtc atagctgttt cctgtgtgaa 6900
   attgttatcc gctcacaatt ccacacaaca tacgagccgg aagcataaag tgtaaagcct 6960
   ggggtgccta atgagtgagc taactcacat taattgcgtt gcgctcactg cccgctttcc 7020
   agtcgggaaa cctgtcgtgc cagctgcatt aatgaatcgg ccaacgcgcg gggagaggcg 7080
   gtttgcgtat tgggcgctct tccgcttcct cgctcactga ctcgctgcgc tcggtcgttc 7140
   ggctgcggcg agcggtatca gctcactcaa aggcggtaat acggttatcc acagaatcag 7200
   gggataacgc aggaaagaac atgtgagcaa aaggccagca aaaggccagg aaccgtaaaa 7260
   aggccgcgtt gctggcgttt ttccataggc tccgcccccc tgacgagcat cacaaaaatc 7320
   gacgctcaag tcagaggtgg cgaaacccga caggactata aagataccag gcgtttcccc 7380
   ctggaagctc cctcgtgcgc tctcctgttc cgaccctgcc gcttaccgga tacctgtccg 7440
   cctttctccc ttcgggaagc gtggcgcttt ctcaatgctc acgctgtagg tatctcagtt 7500
   cggtgtaggt cgttcgctcc aagctgggct gtgtgcacga accccccgtt cagcccgacc 7560
   gctgcgcctt atccggtaac tatcgtcttg agtccaaccc ggtaagacac gacttatcgc 7620
   cactggcagc agccactggt aacaggatta gcagagcgag gtatgtaggc ggtgctacag 7680
   agttcttgaa gtggtggcct aactacggct acactagaag gacagtattt ggtatctgcg 7740
   ctctgctgaa gccagttacc ttcggaaaaa gagttggtag ctcttgatcc ggcaaacaaa 7800
   ccaccgctgg tagcggtggt ttttttgttt gcaagcagca gattacgcgc agaaaaaaag 7860
   gatctcaaga agatcctttg atcttttcta cggggtctga cgctcagtgg aacgaaaact 7920
   cacgttaagg gattttggtc atgagattat caaaaaggat cttcacctag atccttttaa 7980
   attaaaaatg aagttttaaa tcaatctaaa gtatatatga gtaaacttgg tctgacagtt 8040
   accaatgctt aatcagtgag gcacctatct cagcgatctg tctatttcgt tcatccatag 8100
   ttgcctgact ccccgtcgtg tagataacta cgatacggga gggcttacca tctggcccca 8160
   gtgctgcaat gataccgcga gacccacgct caccggctcc agatttatca gcaataaacc 8220
   agccagccgg aagggccgag cgcagaagtg gtcctgcaac tttatccgcc tccatccagt 8280
   ctattaattg ttgccgggaa gctagagtaa gtagttcgcc agttaatagt ttgcgcaacg 8340
   ttgttgccat tgctacaggc atcgtggtgt cacgctcgtc gtttggtatg gcttcattca 8400
   gctccggttc ccaacgatca aggcgagtta catgatcccc catgttgtgc aaaaaagcgg 8460
   ttagctcctt cggtcctccg atcgttgtca gaagtaagtt ggccgcagtg ttatcactca 8520
   tggttatggc agcactgcat aattctctta ctgtcatgcc atccgtaaga tgcttttctg 8580
   tgactggtga gtactcaacc aagtcattct gagaatagtg tatgcggcga ccgagttgct 8640
   cttgcccggc gtcaatacgg gataataccg cgccacatag cagaacttta aaagtgctca 8700
   tcattggaaa acgttcttcg gggcgaaaac tctcaaggat cttaccgctg ttgagatcca 8760
   gttcgatgta acccactcgt gcacccaact gatcttcagc atcttttact ttcaccagcg 8820
   tttctgggtg agcaaaaaca ggaaggcaaa atgccgcaaa aaagggaata agggcgacac 8880
   ggaaatgttg aatactcata ctcttccttt ttcaatatta ttgaagcatt tatcagggtt 8940
   attgtctcat gagcggatac atatttgaat gtatttagaa aaataaacaa ataggggttc 9000
   cgcgcacatt tccccgaaaa gtgccacctg acgtc 9035
<210> 48
   <211> 6857
   <212> DNA
   <213> Artificial Sequence
<220>
   <223> vector
<400> 48
   gacggatcgg gagatctccc gatcccctat ggtcgactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gcttagggtt     900
   tccgatggca cctgtgtcaa ggtcttccaa caactccggg tcttccagcg acttcaagtc     960
   ttccaataat ctcaaggtct tccagataat cctgagcttc cagaaaatcc acatcttcca    1020
   gacaatccat gtcttccgga caatccatgt cttccaagaa gctccaagtc ttccagtaaa    1080
   tcaagtcttc cagcaaatcc agtcttccag caattactgg tcttccacca aatccagatc    1140
   ttccaggaaa atccacgtct tccaggaaat ccatgtcttc caataatttc aaggtcttcc    1200
   atcaaataca gatcttccag ctaatccatg tcttccagaa aaatctgtgt cttccaccaa    1260
   atccaagtct tccagtaaat ctagttcttc cagaaaaatc tagatcttcc agtcaatcag    1320
   tgtcttccag aaagaaatcc aggtcttcca gtcaatcagt gtcttccaga aagaaatcca    1380
   ggtcttccag tcagtcagtg tcttccagaa aaatctacgt cttccaccaa atccaggtct    1440
   tccagtcaat ccacatcttc cggaaaaaat ccaggtcttc cagccaatat atgtcttcct    1500
   gaagatccac gtcttccaga aaatccatgt cttccagaaa atccatgtct tccagtaacc    1560
   tcccagtctt ccagaaaatc cacgtcttcc caacaatcca agtcttccgg ataatttggg    1620
   tcttcctgaa aatctacgtc ttccaaaaaa gccatgtctt ccagaaaatc cacatcttcc    1680
   aatggcctcc aggtcttcca gactatccat gtcttccaga aaatccttgt cttcccttaa    1740
   atctatagct tccaaaaaat ccgggtcttc caggaaatcc gtgtcttcca gcaagtccac 1800
   gtcttccaac aaagccatgt cttccagact atccatgtct tccagaaaat ccttgtcttc 1860
   cctcaaatcc atagcttccg aaaaatccag gtcttccagg aaatccgtgt cttccagcaa 1920
   atccacgtct tccaacaaag ccatgtcttc catcaaatta atgtcttcca gcctacttgt 1980
   gtcttccaac aaaggtacgt cttccaacaa aggtacgtct tccaacaaag gtatgtcttc 2040
   caacaaaggt acgtcttcca gaaaatccac gtcttccaac caagccatgt cttccagaaa 2100
   atccacgtct tccagaaaat atatgtcttc caactaagct acgtcttcca acaaatccat 2160
   gtcttcctat atctccaggt cttccagcat ctccagggct tccagcatct gctcgtcttc 2220
   caacatctcc acgtcttcca gcatctctgt gtcttccagc atcttcatgt cttccaacaa 2280
   ctacccagtc ttccatcaac tggctcaata tccatgtctt ccaacgtctc cagtgtgctg 2340
   atcttctgac attcaggtct tccagtgtct gcaatatcca gctcgagcat gcatctagag 2400
   ggccctattc tatagtgtca cctaaatgct agagctcgct gatcagcctc gactgtgcct 2460
   tctagttgcc agccatctgt tgtttgcccc tcccccgtgc cttccttgac cctggaaggt 2520
   gccactccca ctgtcctttc ctaataaaat gaggaaattg catcgcattg tctgagtagg 2580
   tgtcattcta ttctgggggg tggggtgggg caggacagca agggggagga ttgggaagac 2640
   aatagcaggc atgctgggga tgcggtgggc tctatggctt ctgaggcgga aagaaccagc 2700
   tggggctcta gggggtatcc ccacgcgccc tgtagcggcg cattaagcgc ggcgggtgtg 2760
   gtggttacgc gcagcgtgac cgctacactt gccagcgccc tagcgcccgc tcctttcgct 2820
   ttcttccctt cctttctcgc cacgttcgcc ggctttcccc gtcaagctct aaatcggggc 2880
   atccctttag ggttccgatt tagtgcttta cggcacctcg accccaaaaa acttgattag 2940
   ggtgatggtt cacgtagtgg gccatcgccc tgatagacgg tttttcgccc tttgacgttg 3000
   gagtccacgt tctttaatag tggactcttg ttccaaactg gaacaacact caaccctatc 3060
   tcggtctatt cttttgattt ataagggatt ttggggattt cggcctattg gttaaaaaat 3120
   gagctgattt aacaaaaatt taacgcgaat taattctgtg gaatgtgtgt cagttagggt 3180
   gtggaaagtc cccaggctcc ccaggcaggc agaagtatgc aaagcatgca tctcaattag 3240
   tcagcaacca ggtgtggaaa gtccccaggc tccccagcag gcagaagtat gcaaagcatg 3300
   catctcaatt agtcagcaac catagtcccg cccctaactc cgcccatccc gcccctaact 3360
   ccgcccagtt ccgcccattc tccgccccat ggctgactaa ttttttttat ttatgcagag 3420
   gccgaggccg cctctgcctc tgagctattc cagaagtagt gaggaggctt ttttggaggc 3480
   ctaggctttt gcaaaaagct cccgggagct tgtatatcca ttttcggatc tgatcaagag 3540
   acaggatgag gatcgtttcg catgattgaa caagatggat tgcacgcagg ttctccggcc 3600
   gcttgggtgg agaggctatt cggctatgac tgggcacaac agacaatcgg ctgctctgat 3660
   gccgccgtgt tccggctgtc agcgcagggg cgcccggttc tttttgtcaa gaccgacctg 3720
   tccggtgccc tgaatgaact gcaggacgag gcagcgcggc tatcgtggct ggccacgacg 3780
   ggcgttcctt gcgcagctgt gctcgacgtt gtcactgaag cgggaaggga ctggctgcta 3840
   ttgggcgaag tgccggggca ggatctcctg tcatctcacc ttgctcctgc cgagaaagta 3900
   tccatcatgg ctgatgcaat gcggcggctg catacgcttg atccggctac ctgcccattc 3960
   gaccaccaag cgaaacatcg catcgagcga gcacgtactc ggatggaagc cggtcttgtc 4020
   gatcaggatg atctggacga agagcatcag gggctcgcgc cagccgaact gttcgccagg 4080
   ctcaaggcgc gcatgcccga cggcgaggat ctcgtcgtga cccatggcga tgcctgcttg 4140
   ccgaatatca tggtggaaaa tggccgcttt tctggattca tcgactgtgg ccggctgggt 4200
   gtggcggacc gctatcagga catagcgttg gctacccgtg atattgctga agagcttggc 4260
   ggcgaatggg ctgaccgctt cctcgtgctt tacggtatcg ccgctcccga ttcgcagcgc 4320
   atcgccttct atcgccttct tgacgagttc ttctgagcgg gactctgggg ttcgaaatga 4380
   ccgaccaagc gacgcccaac ctgccatcac gagatttcga ttccaccgcc gccttctatg 4440
   aaaggttggg cttcggaatc gttttccggg acgccggctg gatgatcctc cagcgcgggg 4500
   atctcatgct ggagttcttc gcccacccca acttgtttat tgcagcttat aatggttaca 4560
   aataaagcaa tagcatcaca aatttcacaa ataaagcatt tttttcactg cattctagtt 4620
   gtggtttgtc caaactcatc aatgtatctt atcatgtctg tataccgtcg acctctagct 4680
   agagcttggc gtaatcatgg tcatagctgt ttcctgtgtg aaattgttat ccgctcacaa 4740
   ttccacacaa catacgagcc ggaagcataa agtgtaaagc ctggggtgcc taatgagtga 4800
   gctaactcac attaattgcg ttgcgctcac tgcccgcttt ccagtcggga aacctgtcgt 4860
   gccagctgca ttaatgaatc ggccaacgcg cggggagagg cggtttgcgt attgggcgct 4920
   cttccgcttc ctcgctcact gactcgctgc gctcggtcgt tcggctgcgg cgagcggtat 4980
   cagctcactc aaaggcggta atacggttat ccacagaatc aggggataac gcaggaaaga 5040
   acatgtgagc aaaaggccag caaaaggcca ggaaccgtaa aaaggccgcg ttgctggcgt 5100
   ttttccatag gctccgcccc cctgacgagc atcacaaaaa tcgacgctca agtcagaggt 5160
   ggcgaaaccc gacaggacta taaagatacc aggcgtttcc ccctggaagc tccctcgtgc 5220
   gctctcctgt tccgaccctg ccgcttaccg gatacctgtc cgcctttctc ccttcgggaa 5280
   gcgtggcgct ttctcaatgc tcacgctgta ggtatctcag ttcggtgtag gtcgttcgct 5340
   ccaagctggg ctgtgtgcac gaaccccccg ttcagcccga ccgctgcgcc ttatccggta 5400
   actatcgtct tgagtccaac ccggtaagac acgacttatc gccactggca gcagccactg 5460
   gtaacaggat tagcagagcg aggtatgtag gcggtgctac agagttcttg aagtggtggc 5520
   ctaactacgg ctacactaga aggacagtat ttggtatctg cgctctgctg aagccagtta      5580
   ccttcggaaa aagagttggt agctcttgat ccggcaaaca aaccaccgct ggtagcggtg      5640
   gtttttttgt ttgcaagcag cagattacgc gcagaaaaaa aggatctcaa gaagatcctt      5700
   tgatcttttc tacggggtct gacgctcagt ggaacgaaaa ctcacgttaa gggattttgg      5760
   tcatgagatt atcaaaaagg atcttcacct agatcctttt aaattaaaaa tgaagtttta      5820
   aatcaatcta aagtatatat gagtaaactt ggtctgacag ttaccaatgc ttaatcagtg      5880
   aggcacctat ctcagcgatc tgtctatttc gttcatccat agttgcctga ctccccgtcg      5940
   tgtagataac tacgatacgg gagggcttac catctggccc cagtgctgca atgataccgc      6000
   gagacccacg ctcaccggct ccagatttat cagcaataaa ccagccagcc ggaagggccg      6060
   agcgcagaag tggtcctgca actttatccg cctccatcca gtctattaat tgttgccggg      6120
   aagctagagt aagtagttcg ccagttaata gtttgcgcaa cgttgttgcc attgctacag      6180
   gcatcgtggt gtcacgctcg tcgtttggta tggcttcatt cagctccggt tcccaacgat      6240
   caaggcgagt tacatgatcc cccatgttgt gcaaaaaagc ggttagctcc ttcggtcctc      6300
   cgatcgttgt cagaagtaag ttggccgcag tgttatcact catggttatg gcagcactgc      6360
   ataattctct tactgtcatg ccatccgtaa gatgcttttc tgtgactggt gagtactcaa      6420
   ccaagtcatt ctgagaatag tgtatgcggc gaccgagttg ctcttgcccg gcgtcaatac      6480
   gggataatac cgcgccacat agcagaactt taaaagtgct catcattgga aaacgttctt      6540
   cggggcgaaa actctcaagg atcttaccgc tgttgagatc cagttcgatg taacccactc      6600
   gtgcacccaa ctgatcttca gcatctttta ctttcaccag cgtttctggg tgagcaaaaa      6660
   caggaaggca aaatgccgca aaaaagggaa taagggcgac acggaaatgt tgaatactca      6720
   tactcttcct ttttcaatat tattgaagca tttatcaggg ttattgtctc atgagcggat      6780
   acatatttga atgtatttag aaaaataaac aaataggggt tccgcgcaca tttccccgaa      6840
   aagtgccacc tgacgtc                                                     6857
<210> 49
   <211> 8094
   <212> DNA
   <213> Artificial Sequence
<220>
   <223> vector
<400> 49
   gacggatcgg gagatctccc gatcccctat ggtcgactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gcttctgtaa 900
   gagtagtctc atgatgtctt ttaatttaat atctaggccc taatacacaa ctctttgaat 960
   attgctacta tctttcagca tgactttcca ataccattaa gattaagcct ttctgttctt 1020
   cagtttctct aactcaagtc ttctaagctc cacagcttcc aacatcttta attaccagat 1080
   cttcccaata ttataacatc cgtgcattcc agtccctgtc ctgcagcatc tccaatgccc 1140
   ttgttttcta aacttcccag tattcaagtc attaaaaatt taggtcttct aatatctcca 1200
   acaacccatc ttcaaagtcc aggtcttgtc tttcagaatc cagagttcta aattacaagt 1260
   cttccatggt cctaatcctc caatgtctcc tatatacagg tcttctagag gctatgtatt 1320
   tcatagtgtt taagtctaga ttcttcaatg tcttccagca cttacatcat tcagttttta 1380
   catttttaaa tgtctcctac tttctagttt tacagtgtat ttttatcgcc aattcctcca 1440
   atgcccaagt ctttaaatac ctgcaagaac tttcaggtac atcaatatcc tcaatgtctg 1500
   aatgaataga cctcttaata tcctggtctc atattgtcca ggctgccagt attcttgaca 1560
   tccagcatct ttaaaattta attcttccaa actgtctttt tgacataaat atgtcaatat 1620
   ccatgcttac caatgtctcc caatatccag ggcttctcat tttaggattt ataaatgtct 1680
   acatctgctg ttgtttatat ctgccagtat ttaggagttc caaggtacag gacttacaat 1740
   atctctaatt tccagagatt tcagtgtcta tgacttccaa tattcaatat cccaatgttc 1800
   agggcctcca gaaatatcaa tgtccagagt aaccaatgtc ttcaaaatcc aaggcttgca 1860
   atgtctccaa tatcctgtgc ttacaatgtt tccaatgtcc agggtttccg atggcacctg 1920
   tgtcaaggtc ttccaacaac tccgggtctt ccagcgactt caagtcttcc aataatctca 1980
   aggtcttcca gataatcctg agcttccaga aaatccacat cttccagaca atccatgtct 2040
   tccggacaat ccatgtcttc caagaagctc caagtcttcc agtaaatcaa gtcttccagc 2100
   aaatccagtc ttccagcaat tactggtctt ccaccaaatc cagatcttcc aggaaaatcc 2160
   acgtcttcca ggaaatccat gtcttccaat aatttcaagg tcttccatca aatacagatc 2220
   ttccagctaa tccatgtctt ccagaaaaat ctgtgtcttc caccaaatcc aagtcttcca 2280
   gtaaatctag ttcttccaga aaaatctaga tcttccagtc aatcagtgtc ttccagaaag 2340
   aaatccaggt cttccagtca atcagtgtct tccagaaaga aatccaggtc ttccagtcag 2400
   tcagtgtctt ccagaaaaat ctacgtcttc caccaaatcc aggtcttcca gtcaatccac 2460
   atcttccgga aaaaatccag gtcttccagc caatatatgt cttcctgaag atccacgtct 2520
   tccagaaaat ccatgtcttc cagaaaatcc atgtcttcca gtaacctccc agtcttccag 2580
   aaaatccacg tcttcccaac aatccaagtc ttccggataa tttgggtctt cctgaaaatc 2640
   tacgtcttcc aaaaaagcca tgtcttccag aaaatccaca tcttccaatg gcctccaggt 2700
   cttccagact atccatgtct tccagaaaat ccttgtcttc ccttaaatct atagcttcca 2760
   aaaaatccgg gtcttccagg aaatccgtgt cttccagcaa gtccacgtct tccaacaaag 2820
   ccatgtcttc cagactatcc atgtcttcca gaaaatcctt gtcttccctc aaatccatag 2880
   cttccgaaaa atccaggtct tccaggaaat ccgtgtcttc cagcaaatcc acgtcttcca 2940
   acaaagccat gtcttccatc aaattaatgt cttccagcct acttgtgtct tccaacaaag 3000
   gtacgtcttc caacaaaggt acgtcttcca acaaaggtat gtcttccaac aaaggtacgt 3060
   cttccagaaa atccacgtct tccaaccaag ccatgtcttc cagaaaatcc acgtcttcca 3120
   gaaaatatat gtcttccaac taagctacgt cttccaacaa atccatgtct tcctatatct 3180
   ccaggtcttc cagcatctcc agggcttcca gcatctgctc gtcttccaac atctccacgt 3240
   cttccagcat ctctgtgtct tccagcatct tcatgtcttc caacaactac ccagtcttcc 3300
   atcaactggc tcaatatcca tgtcttccaa cgtctccagt gtgctgatct tctgacattc 3360
   aggtcttcca gtgtctgcaa tatccaggta ttctaacatg ttctatagtc cagatcttcc 3420
   agcctttccc caggtccagg ccttttcaaa tctaggcttc ccctgtctga gccttcccat 3480
   gttaaaatca ctctacagaa aaatcttcca actcaactat ttctgcttct agttgaatta 3540
   gcttcaaaaa catttaatgg gacattcctc cttatagacc agatcttatg aaccaacagt 3600
   actgcagttt ggcggccgct cgagcatgca tctagagggc cctattctat agtgtcacct 3660
   aaatgctaga gctcgctgat cagcctcgac tgtgccttct agttgccagc catctgttgt 3720
   ttgcccctcc cccgtgcctt ccttgaccct ggaaggtgcc actcccactg tcctttccta 3780
   ataaaatgag gaaattgcat cgcattgtct gagtaggtgt cattctattc tggggggtgg 3840
   ggtggggcag gacagcaagg gggaggattg ggaagacaat agcaggcatg ctggggatgc 3900
   ggtgggctct atggcttctg aggcggaaag aaccagctgg ggctctaggg ggtatcccca 3960
   cgcgccctgt agcggcgcat taagcgcggc gggtgtggtg gttacgcgca gcgtgaccgc 4020
   tacacttgcc agcgccctag cgcccgctcc tttcgctttc ttcccttcct ttctcgccac 4080
   gttcgccggc tttccccgtc aagctctaaa tcggggcatc cctttagggt tccgatttag 4140
   tgctttacgg cacctcgacc ccaaaaaact tgattagggt gatggttcac gtagtgggcc 4200
   atcgccctga tagacggttt ttcgcccttt gacgttggag tccacgttct ttaatagtgg 4260
   actcttgttc caaactggaa caacactcaa ccctatctcg gtctattctt ttgatttata 4320
   agggattttg gggatttcgg cctattggtt aaaaaatgag ctgatttaac aaaaatttaa 4380
   cgcgaattaa ttctgtggaa tgtgtgtcag ttagggtgtg gaaagtcccc aggctcccca 4440
   ggcaggcaga agtatgcaaa gcatgcatct caattagtca gcaaccaggt gtggaaagtc 4500
   cccaggctcc ccagcaggca gaagtatgca aagcatgcat ctcaattagt cagcaaccat 4560
   agtcccgccc ctaactccgc ccatcccgcc cctaactccg cccagttccg cccattctcc 4620
   gccccatggc tgactaattt tttttattta tgcagaggcc gaggccgcct ctgcctctga 4680
   gctattccag aagtagtgag gaggcttttt tggaggccta ggcttttgca aaaagctccc 4740
   gggagcttgt atatccattt tcggatctga tcaagagaca ggatgaggat cgtttcgcat 4800
   gattgaacaa gatggattgc acgcaggttc tccggccgct tgggtggaga ggctattcgg 4860
   ctatgactgg gcacaacaga caatcggctg ctctgatgcc gccgtgttcc ggctgtcagc 4920
   gcaggggcgc ccggttcttt ttgtcaagac cgacctgtcc ggtgccctga atgaactgca 4980
   ggacgaggca gcgcggctat cgtggctggc cacgacgggc gttccttgcg cagctgtgct 5040
   cgacgttgtc actgaagcgg gaagggactg gctgctattg ggcgaagtgc cggggcagga 5100
   tctcctgtca tctcaccttg ctcctgccga gaaagtatcc atcatggctg atgcaatgcg 5160
   gcggctgcat acgcttgatc cggctacctg cccattcgac caccaagcga aacatcgcat 5220
   cgagcgagca cgtactcgga tggaagccgg tcttgtcgat caggatgatc tggacgaaga 5280
   gcatcagggg ctcgcgccag ccgaactgtt cgccaggctc aaggcgcgca tgcccgacgg 5340
   cgaggatctc gtcgtgaccc atggcgatgc ctgcttgccg aatatcatgg tggaaaatgg 5400
   ccgcttttct ggattcatcg actgtggccg gctgggtgtg gcggaccgct atcaggacat 5460
   agcgttggct acccgtgata ttgctgaaga gcttggcggc gaatgggctg accgcttcct 5520
   cgtgctttac ggtatcgccg ctcccgattc gcagcgcatc gccttctatc gccttcttga 5580
   cgagttcttc tgagcgggac tctggggttc gaaatgaccg accaagcgac gcccaacctg 5640
   ccatcacgag atttcgattc caccgccgcc ttctatgaaa ggttgggctt cggaatcgtt 5700
   ttccgggacg ccggctggat gatcctccag cgcggggatc tcatgctgga gttcttcgcc 5760
   caccccaact tgtttattgc agcttataat ggttacaaat aaagcaatag catcacaaat 5820
   ttcacaaata aagcattttt ttcactgcat tctagttgtg gtttgtccaa actcatcaat 5880
   gtatcttatc atgtctgtat accgtcgacc tctagctaga gcttggcgta atcatggtca 5940
   tagctgtttc ctgtgtgaaa ttgttatccg ctcacaattc cacacaacat acgagccgga 6000
   agcataaagt gtaaagcctg gggtgcctaa tgagtgagct aactcacatt aattgcgttg 6060
   cgctcactgc ccgctttcca gtcgggaaac ctgtcgtgcc agctgcatta atgaatcggc 6120
   caacgcgcgg ggagaggcgg tttgcgtatt gggcgctctt ccgcttcctc gctcactgac 6180
   tcgctgcgct cggtcgttcg gctgcggcga gcggtatcag ctcactcaaa ggcggtaata 6240
   cggttatcca cagaatcagg ggataacgca ggaaagaaca tgtgagcaaa aggccagcaa 6300
   aaggccagga accgtaaaaa ggccgcgttg ctggcgtttt tccataggct ccgcccccct 6360
   gacgagcatc acaaaaatcg acgctcaagt cagaggtggc gaaacccgac aggactataa 6420
   agataccagg cgtttccccc tggaagctcc ctcgtgcgct ctcctgttcc gaccctgccg 6480
   cttaccggat acctgtccgc ctttctccct tcgggaagcg tggcgctttc tcaatgctca 6540
   cgctgtaggt atctcagttc ggtgtaggtc gttcgctcca agctgggctg tgtgcacgaa 6600
   ccccccgttc agcccgaccg ctgcgcctta tccggtaact atcgtcttga gtccaacccg 6660
   gtaagacacg acttatcgcc actggcagca gccactggta acaggattag cagagcgagg 6720
   tatgtaggcg gtgctacaga gttcttgaag tggtggccta actacggcta cactagaagg 6780
   acagtatttg gtatctgcgc tctgctgaag ccagttacct tcggaaaaag agttggtagc 6840
   tcttgatccg gcaaacaaac caccgctggt agcggtggtt tttttgtttg caagcagcag 6900
   attacgcgca gaaaaaaagg atctcaagaa gatcctttga tcttttctac ggggtctgac 6960
   gctcagtgga acgaaaactc acgttaaggg attttggtca tgagattatc aaaaaggatc 7020
   ttcacctaga tccttttaaa ttaaaaatga agttttaaat caatctaaag tatatatgag 7080
   taaacttggt ctgacagtta ccaatgctta atcagtgagg cacctatctc agcgatctgt 7140
   ctatttcgtt catccatagt tgcctgactc cccgtcgtgt agataactac gatacgggag 7200
   ggcttaccat ctggccccag tgctgcaatg ataccgcgag acccacgctc accggctcca 7260
   gatttatcag caataaacca gccagccgga agggccgagc gcagaagtgg tcctgcaact 7320
   ttatccgcct ccatccagtc tattaattgt tgccgggaag ctagagtaag tagttcgcca 7380
   gttaatagtt tgcgcaacgt tgttgccatt gctacaggca tcgtggtgtc acgctcgtcg 7440
   tttggtatgg cttcattcag ctccggttcc caacgatcaa ggcgagttac atgatccccc 7500
   atgttgtgca aaaaagcggt tagctccttc ggtcctccga tcgttgtcag aagtaagttg 7560
   gccgcagtgt tatcactcat ggttatggca gcactgcata attctcttac tgtcatgcca 7620
   tccgtaagat gcttttctgt gactggtgag tactcaacca agtcattctg agaatagtgt 7680
   atgcggcgac cgagttgctc ttgcccggcg tcaatacggg ataataccgc gccacatagc 7740
   agaactttaa aagtgctcat cattggaaaa cgttcttcgg ggcgaaaact ctcaaggatc 7800
   ttaccgctgt tgagatccag ttcgatgtaa cccactcgtg cacccaactg atcttcagca 7860
   tcttttactt tcaccagcgt ttctgggtga gcaaaaacag gaaggcaaaa tgccgcaaaa 7920
   aagggaataa gggcgacacg gaaatgttga atactcatac tcttcctttt tcaatattat 7980
   tgaagcattt atcagggtta ttgtctcatg agcggataca tatttgaatg tatttagaaa 8040
   aataaacaaa taggggttcc gcgcacattt ccccgaaaag tgccacctga cgtc 8094
<210> 50
   <211> 7924
   <212> DNA
   <213> Artificial Sequence
<220>
   <223> vector
<400> 50
   gacggatcgg gagatctccc gatcccctat ggtcgactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gcttggtacc     900
   gagctcggat cctggactag ggaggtcctg aaggaggagg gataaatatt ttcttacaca     960
   cgttaaatat taaaatcttt gttacaagca cattttggtc agtggctttt agctcttaca    1020
   ctttaagttt tgacttctgt atctgtctgt ctctgtctgt ctctgtctgt ctgtctgtct    1080
   ttgtctgtct gtctgtttct ctttgcatca catctctgtc tttttggttg caatcataat    1140
   tcttccatta tgttcagcat ttcagatctt gatttttagt gttcagccct acagccacat    1200
   gatatcttaa actctgaaga agagacaagt tttgggactg gtgacaattg tctagagagc    1260
   atggagggcc atgtcaagcg ccccatgaat gcatttatgg tgtggtcccg tggtgagagg 1320
   cacaagttgg cccagcagaa tcccagcatg caaaatacag agatcagcaa gcagctggga 1380
   tgcaggtgga aaagccttac agaagccgaa aaaaggccct ttttccagga ggcacagaga 1440
   ttgaagatcc tacacagaga gaaataccca aactataaat atcagcctca tcggagggct 1500
   aaagtgtcac agaggagtgg cattttacag cctgcagttg cctcaacaaa actgtacaac 1560
   cttctgcagt gggacaggaa cccacatgcc atcacataca ggcaagactg gagtagagct 1620
   gcacacctgt actccaaaaa ccagcaaagc ttttattggc agcctgttga tatccccact 1680
   gggcacctgc agcagcagca gcagcagcag cagcagcagc agttccataa ccaccaccag 1740
   cagcaacagc agttctatga ccaccaccag cagcagcagc agcagcagca gcagcagcag 1800
   cagttccatg accaccacca gcagaagcag cagtttcatg accaccacca gcagcaacag 1860
   cagttccatg accaccacca ccaccaccag gagcagcagt tccatgacca ccaccagcag 1920
   caacagcagt tccatgacca ccagcagcag cagcagcagc agcagcagca gcagttccat 1980
   gaccaccacc agcagaagca gcagttccat gaccaccacc accaccaaca gcagcagcag 2040
   ttccatgacc accagcagca gcagcagcag ttccatgacc accagcagca gcagcatcag 2100
   ttccatgacc acccccagca gaagcagcag ttccatgacc acccccagca gcaacagcag 2160
   ttccatgacc accaccacca gcagcagcag aagcagcagt tccatgacca ccaccagcag 2220
   aagcagcagt tccatgacca ccaccagcag aagcagcagt tccatgacca ccaccagcag 2280
   caacagcagt tccatgacca ccaccagcag cagcagcagc agcagcagca gcagcagcag 2340
   cagttccacg accagcagct tacctactta ctaacagctg acatcactgg tgagcataca 2400
   ccataccagg agcacctcag cacagccctg tggttggcag tctcatgaca ctggcctttt 2460
   ctcctaccta tgccaacact cccccttgct atgattttta agtctgagat tcctagttgg 2520
   tttagatact gacttttctc ctgtcatgaa aaaggtcctg ctccttcctt ttgggttgcg 2580
   gccgctcgac gatctctatc actgataggg agatctctat cactgatagg gagagctctg 2640
   cttatataga cctcccaccg tacacgccta ccgcccattt gcgtcaatgg ggcggagttg 2700
   ttacgacatt ttggaaagtc ccgttgattt tggtgccaaa acaaactccc attgacgtca 2760
   atggggtgga gacttggaaa tccccgtgag tcaaaccgct atccacgccc attgatgtac 2820
   tgccaaaacc gcatcaccat ggtaatagcg atgactaata cgtagatgta ctgccaagta 2880
   ggaaagtccc ataaggtcat gtactgggca taatgccagg cgggccattt accgtcattg 2940
   acgtcaatag ggggcgtact tggcatatga tacacttgat gtactgccaa gtgggcagtt 3000
   taccgtaaat actccaccca ttgacgtcaa tggaaagtcc ctattggcgt tactatggga 3060
   acatacgtca ttattgacgt caatgggcgg gggtcgttgg gcggtcagcc aggcgggcca 3120
   tttaccgtaa gttatgtaac gcggaactcc atatatgggc tatgaactaa tgaccccgta 3180
   attgattact attaataact agtcaataat caatgtcaac gcgtatatct ggcccgtaca 3240
   tcgcgaagca gcgcaaaacg cctaacccta agcagattct tcatgcaatt gtcggtcaag 3300
   ccttgccttg ttgtagctta aattttgctc gcgcactact cagcgacctc caacacacaa 3360
   gcagggagca gatactggct taactatgcg gcatcagagc agattgtact gagagtgcac 3420
   cataggggat cgggagatct cccgatccgt cgagcatgca tctagagggc cctattctat 3480
   agtgtcacct aaatgctaga gctcgctgat cagcctcgac tgtgccttct agttgccagc 3540
   catctgttgt ttgcccctcc cccgtgcctt ccttgaccct ggaaggtgcc actcccactg 3600
   tcctttccta ataaaatgag gaaattgcat cgcattgtct gagtaggtgt cattctattc 3660
   tggggggtgg ggtggggcag gacagcaagg gggaggattg ggaagacaat agcaggcatg 3720
   ctggggatgc ggtgggctct atggcttctg aggcggaaag aaccagctgg ggctctaggg 3780
   ggtatcccca cgcgccctgt agcggcgcat taagcgcggc gggtgtggtg gttacgcgca 3840
   gcgtgaccgc tacacttgcc agcgccctag cgcccgctcc tttcgctttc ttcccttcct 3900
   ttctcgccac gttcgccggc tttccccgtc aagctctaaa tcggggcatc cctttagggt 3960
   tccgatttag tgctttacgg cacctcgacc ccaaaaaact tgattagggt gatggttcac 4020
   gtagtgggcc atcgccctga tagacggttt ttcgcccttt gacgttggag tccacgttct 4080
   ttaatagtgg actcttgttc caaactggaa caacactcaa ccctatctcg gtctattctt 4140
   ttgatttata agggattttg gggatttcgg cctattggtt aaaaaatgag ctgatttaac 4200
   aaaaatttaa cgcgaattaa ttctgtggaa tgtgtgtcag ttagggtgtg gaaagtcccc 4260
   aggctcccca ggcaggcaga agtatgcaaa gcatgcatct caattagtca gcaaccaggt 4320
   gtggaaagtc cccaggctcc ccagcaggca gaagtatgca aagcatgcat ctcaattagt 4380
   cagcaaccat agtcccgccc ctaactccgc ccatcccgcc cctaactccg cccagttccg 4440
   cccattctcc gccccatggc tgactaattt tttttattta tgcagaggcc gaggccgcct 4500
   ctgcctctga gctattccag aagtagtgag gaggcttttt tggaggccta ggcttttgca 4560
   aaaagctccc gggagcttgt atatccattt tcggatctga tcaagagaca ggatgaggat 4620
   cgtttcgcat gattgaacaa gatggattgc acgcaggttc tccggccgct tgggtggaga 4680
   ggctattcgg ctatgactgg gcacaacaga caatcggctg ctctgatgcc gccgtgttcc 4740
   ggctgtcagc gcaggggcgc ccggttcttt ttgtcaagac cgacctgtcc ggtgccctga 4800
   atgaactgca ggacgaggca gcgcggctat cgtggctggc cacgacgggc gttccttgcg 4860
   cagctgtgct cgacgttgtc actgaagcgg gaagggactg gctgctattg ggcgaagtgc 4920
   cggggcagga tctcctgtca tctcaccttg ctcctgccga gaaagtatcc atcatggctg 4980
   atgcaatgcg gcggctgcat acgcttgatc cggctacctg cccattcgac caccaagcga 5040
   aacatcgcat cgagcgagca cgtactcgga tggaagccgg tcttgtcgat caggatgatc 5100
   tggacgaaga gcatcagggg ctcgcgccag ccgaactgtt cgccaggctc aaggcgcgca 5160
   tgcccgacgg cgaggatctc gtcgtgaccc atggcgatgc ctgcttgccg aatatcatgg 5220
   tggaaaatgg ccgcttttct ggattcatcg actgtggccg gctgggtgtg gcggaccgct 5280
   atcaggacat agcgttggct acccgtgata ttgctgaaga gcttggcggc gaatgggctg 5340
   accgcttcct cgtgctttac ggtatcgccg ctcccgattc gcagcgcatc gccttctatc 5400
   gccttcttga cgagttcttc tgagcgggac tctggggttc gaaatgaccg accaagcgac 5460
   gcccaacctg ccatcacgag atttcgattc caccgccgcc ttctatgaaa ggttgggctt 5520
   cggaatcgtt ttccgggacg ccggctggat gatcctccag cgcggggatc tcatgctgga 5580
   gttcttcgcc caccccaact tgtttattgc agcttataat ggttacaaat aaagcaatag 5640
   catcacaaat ttcacaaata aagcattttt ttcactgcat tctagttgtg gtttgtccaa 5700
   actcatcaat gtatcttatc atgtctgtat accgtcgacc tctagctaga gcttggcgta 5760
   atcatggtca tagctgtttc ctgtgtgaaa ttgttatccg ctcacaattc cacacaacat 5820
   acgagccgga agcataaagt gtaaagcctg gggtgcctaa tgagtgagct aactcacatt 5880
   aattgcgttg cgctcactgc ccgctttcca gtcgggaaac ctgtcgtgcc agctgcatta 5940
   atgaatcggc caacgcgcgg ggagaggcgg tttgcgtatt gggcgctctt ccgcttcctc 6000
   gctcactgac tcgctgcgct cggtcgttcg gctgcggcga gcggtatcag ctcactcaaa 6060
   ggcggtaata cggttatcca cagaatcagg ggataacgca ggaaagaaca tgtgagcaaa 6120
   aggccagcaa aaggccagga accgtaaaaa ggccgcgttg ctggcgtttt tccataggct 6180
   ccgcccccct gacgagcatc acaaaaatcg acgctcaagt cagaggtggc gaaacccgac 6240
   aggactataa agataccagg cgtttccccc tggaagctcc ctcgtgcgct ctcctgttcc 6300
   gaccctgccg cttaccggat acctgtccgc ctttctccct tcgggaagcg tggcgctttc 6360
   tcaatgctca cgctgtaggt atctcagttc ggtgtaggtc gttcgctcca agctgggctg 6420
   tgtgcacgaa ccccccgttc agcccgaccg ctgcgcctta tccggtaact atcgtcttga 6480
   gtccaacccg gtaagacacg acttatcgcc actggcagca gccactggta acaggattag 6540
   cagagcgagg tatgtaggcg gtgctacaga gttcttgaag tggtggccta actacggcta 6600
   cactagaagg acagtatttg gtatctgcgc tctgctgaag ccagttacct tcggaaaaag 6660
   agttggtagc tcttgatccg gcaaacaaac caccgctggt agcggtggtt tttttgtttg 6720
   caagcagcag attacgcgca gaaaaaaagg atctcaagaa gatcctttga tcttttctac 6780
   ggggtctgac gctcagtgga acgaaaactc acgttaaggg attttggtca tgagattatc 6840
   aaaaaggatc ttcacctaga tccttttaaa ttaaaaatga agttttaaat caatctaaag 6900
   tatatatgag taaacttggt ctgacagtta ccaatgctta atcagtgagg cacctatctc 6960
   agcgatctgt ctatttcgtt catccatagt tgcctgactc cccgtcgtgt agataactac 7020
   gatacgggag ggcttaccat ctggccccag tgctgcaatg ataccgcgag acccacgctc 7080
   accggctcca gatttatcag caataaacca gccagccgga agggccgagc gcagaagtgg 7140
   tcctgcaact ttatccgcct ccatccagtc tattaattgt tgccgggaag ctagagtaag 7200
   tagttcgcca gttaatagtt tgcgcaacgt tgttgccatt gctacaggca tcgtggtgtc 7260
   acgctcgtcg tttggtatgg cttcattcag ctccggttcc caacgatcaa ggcgagttac 7320
   atgatccccc atgttgtgca aaaaagcggt tagctccttc ggtcctccga tcgttgtcag 7380
   aagtaagttg gccgcagtgt tatcactcat ggttatggca gcactgcata attctcttac 7440
   tgtcatgcca tccgtaagat gcttttctgt gactggtgag tactcaacca agtcattctg 7500
   agaatagtgt atgcggcgac cgagttgctc ttgcccggcg tcaatacggg ataataccgc 7560
   gccacatagc agaactttaa aagtgctcat cattggaaaa cgttcttcgg ggcgaaaact 7620
   ctcaaggatc ttaccgctgt tgagatccag ttcgatgtaa cccactcgtg cacccaactg 7680
   atcttcagca tcttttactt tcaccagcgt ttctgggtga gcaaaaacag gaaggcaaaa 7740
   tgccgcaaaa aagggaataa gggcgacacg gaaatgttga atactcatac tcttcctttt 7800
   tcaatattat tgaagcattt atcagggtta ttgtctcatg agcggataca tatttgaatg 7860
   tatttagaaa aataaacaaa taggggttcc gcgcacattt ccccgaaaag tgccacctga 7920
   cgtc 7924
<210> 51
   <211> 22
   <212> RNA
   <213> Homo sapiens
<400> 51
   uggaagacua gugauuuugu ug 22
<210> 52
   <211> 22
   <212> RNA
   <213> Homo sapiens
<400> 52
   caacaaauca cagucugcca ua 22
<210> 53
   <211> 22
   <212> RNA
   <213> Homo sapiens
<400> 53
   caacaaaucc cagucuaccu aa 22
<210> 54
   <211> 22
   <212> RNA
   <213> Homo sapiens
<400> 54
   uagcuuauca gacugauguu ga 22
<210> 55
   <211> 21
   <212> RNA
   <213> Homo sapiens
<400> 55
   caacaccagu cgaugggcug u 21
<210> 56
   <211> 23
   <212> RNA
   <213> Homo sapiens
<400> 56
   uuuggcacua gcacauuuuu gcu 23
<210> 57
   <211> 22
   <212> RNA
   <213> Homo sapiens
<400> 57
   aaucaugugc agugccaaua ug 22
<210> 58
   <211> 22
   <212> RNA
   <213> Homo sapiens
<400> 58
   uggagagaaa ggcaguuccu ga 22
<210> 59
   <211> 22
   <212> RNA
   <213> Homo sapiens
<400> 59
   aggggcuggc uuuccucugg uc 22
<210> 60
   <211> 23
   <212> RNA
   <213> Homo sapiens
<400> 60
   cgcauccccu agggcauugg ugu 23
<210> 61
   <211> 20
   <212> RNA
   <213> Homo sapiens
<400> 61
   acugccccag gugcugcugg 20
<210> 62
   <211> 22
   <212> RNA
   <213> Homo sapiens
<400> 62
   uuuggucccc uucaaccagc ua 22
<210> 63
   <211> 22
   <212> RNA
   <213> Homo sapiens
<400> 63
   uccuucauuc caccggaguc ug 22
<210> 64
   <211> 21
   <212> RNA
   <213> Homo sapiens
<400> 64
   gauuucagug gagugaaguu c 21
<210> 65
   <211> 22
   <212> RNA
   <213> Homo sapiens
<400> 65
   caucuuaccg gacagugcug ga 22
<210> 66
   <211> 22
   <212> RNA
   <213> Homo sapiens
<400> 66
   uaacacuguc ugguaacgau gu 22
<210> 67
   <211> 22
   <212> RNA
   <213> Homo sapiens
<400> 67
   caucuuacug ggcagcauug ga 22
<210> 68
   <211> 22
   <212> RNA
   <213> Homo sapiens
<400> 68
   uaauacugcc ugguaaugau ga 22
<210> 69
   <211> 22
   <212> RNA
   <213> Homo sapiens
<400> 69
   cgucuuaccc agcaguguuu gg 22
<210> 70
   <211> 23
   <212> RNA
   <213> Homo sapiens
<400> 70
   uaauacugcc ggguaaugau gga 23
<210> 71
   <211> 22
   <212> RNA
   <213> Homo sapiens
<400> 71
   caucuuccag uacaguguug ga 22
<210> 72
   <211> 22
   <212> RNA
   <213> Homo sapiens
<400> 72
   uaacacuguc ugguaaagau gg 22
<210> 73
   <211> 22
   <212> RNA
   <213> Homo sapiens
<400> 73
   uaauacuguc ugguaaaacc gu 22
<210> 74
   <211> 22
   <212> RNA
   <213> Homo sapiens
<400> 74
   accguggcuu ucgauuguua cu 22
<210> 75
   <211> 22
   <212> RNA
   <213> Homo sapiens
<400> 75
   uaacagucua cagccauggu cg 22
<210> 76
   <211> 22
   <212> RNA
   <213> Homo sapiens
<400> 76
   ugugacuggu ugaccagagg gg 22
<210> 77
   <211> 22
   <212> RNA
   <213> Homo sapiens
<400> 77
   cguguucaca gcggaccuug au 22
<210> 78
   <211> 20
   <212> RNA
   <213> Homo sapiens
<400> 78
   uaaggcacgc ggugaaugcc 20
<210> 79
   <211> 23
   <212> RNA
   <213> Homo sapiens
<400> 79
   uuaaugcuaa ucgugauagg ggu 23
<210> 80
   <211> 22
   <212> RNA
   <213> Homo sapiens
<400> 80
   cuccuacaua uuagcauuaa ca 22
<210> 81
   <211> 22
   <212> RNA
   <213> Homo sapiens
<400> 81
   accuggcaua caauguagau uu 22
<210> 82
   <211> 23
   <212> RNA
   <213> Homo sapiens
<400> 82
   agcuacauug ucugcugggu uuc 23
<210> 83
   <211> 22
   <212> RNA
   <213> Homo sapiens
<400> 83
   cucaguagcc aguguagauc cu 22
<210> 84
   <211> 21
   <212> RNA
   <213> Homo sapiens
<400> 84
   agcuacaucu ggcuacuggg u 21
<210> 85
   <211> 22
   <212> RNA
   <213> Homo sapiens
<400> 85
   uggaguguga caaugguguu ug 22
<210> 86
   <211> 22
   <212> RNA
   <213> Homo sapiens
<400> 86
   aacgccauua ucacacuaaa ua 22